# EUROPEAN PATENT SPECIFICATION

(11) **EP 1 100 818 B1**
(45) Date of publication and mention of the grant of the patent: **02.01.2013**
(21) Application number: 99937691.6
(22) Date of filing: 30.07.1999
(51) Int. Cl.: C07K 14/16, A61K 38/10, A61K 38/12

(54) **INHIBITORS OF HIV MEMBRANE FUSION**
INHIBITOREN DER MEMBRANFUSION VON HIV
INHIBITEURS DE LA FUSION DE LA MEMBRANE DU VIH

(30) Priority: 30.07.1998 US 94676 P; 14.09.1998 US 100265 P; 18.09.1998 US 101058 P; 03.05.1999 US 132295 P
(43) Date of publication of application: 23.05.2001
(73) Proprietor: WHITEHEAD INSTITUTE FOR BIOMEDICAL RESEARCH, Cambridge, MA 02142 (US)
(72) Inventor: ECKERT, Debra, M., Cambridge, MA 02138 (US); CHAN, David C., Brookline, MA 02146 (US); MALASHKEVICH, Vladimir, Somerville, MA 02144 (US); CARR, Peter A., Cambridge, MA 02139 (US); KIM, Peter, S., Lexington, MA 02420 (US)
(74) Representative: Price, Vincent Andrew
(86) International application number: PCT/US1999/017351
(87) International publication number: WO 2000/006599

(56) References cited:
- WO-A-94/02505
- J.K. JUDICE ET AL.: "Inhibition of HIV type 1 infectivity by constrained alpha-helical peptides: Implications for the viral fusion mechanism" PROCEEDINGS OF THE NATIONAL ACADEMY OF SCIENCES OF USA, vol. 94, December 1997 (1997-12), pages 13426-13430, XP002126803 WASHINGTON US
- W. WEISSENHORN ET AL.: "Assembly of a rod-shaped chimera of a trimeric GCN4 zipper and the HIV-1 gp41 ectodomain expressed in Escherichia coli" PROCEEDINGS OF THE NATIONAL ACADEMY OF SCIENCES OF USA, vol. 94, June 1997 (1997-06), pages 6065-6069, XP002126804 WASHINGTON US
- D.C. CHAN ET AL.: "Evidence that a prominent cavity in the coiled coil of HIV type 1 gp41 is an attractive drug target" PROCEEDINGS OF THE NATIONAL ACADEMY OF SCIENCES OF USA, vol. 95, December 1998 (1998-12), pages 1513-15617, XP002126805 WASHINGTON US
- D.M. ECKERT ET AL.: "Inhibiting hIV-1 Entry: Discovery of D-Peptide Inhibitors that Target the gp41 Coiled-Coil Pocket" CELL, vol. 99, 1 October 1999 (1999-10-01), pages 103-115, XP002126806 NA US

## Description

### RELATED APPLICATIONS

This application is related to U.S. Provisional Application 60/043,280, entitled Core Structure of gp41 from the HIV Envelope Glycoprotein, by David C. Chan, Deborah Fass, Min Lu, James M. Berger and Peter S. Kim, filed April 17, 1997 and U.S. Application 09/062,241, entitled Core Structure of gp41 from the HIV Envelope Glycoprotein, by David C. Chan, Deborah Fass, Min Lu, James M. Berger and Peter S. Kim, filed April 17, 1998. The present application claims the benefit of U.S. Provisional Application 60/094,676, entitled Inhibitors of HIV Membrane Fusion by David C. Chan, Debra M. Ehrgott and Peter S. Kim, filed July 30, 1998; U.S. Provisional Application 60/100,265, entitled Inhibitors of HIV Membrane Fusion, by David C. Chan, Debra M. Ehrgott and Peter S. Kim, filed September 14, 1998 and U.S. Provisional Application 60/101,058, entitled Inhibitors of HIV Membrane Fusion, by David C. Chan, Debra M. Ehrgott and Peter S. Kim, filed September 18, 1998; and U.S. Provisional Application 60/132,295, entitled Inhibitors of HIV Membrane Fusion, by Debra M. Ehrgott, David C. Chan, Vladimir Malashkevich and Peter S. Kim, filed May 3, 1999. The entire teachings of these referenced applications are incorporated herein by reference.

### GOVERNMENT SUPPORT

The invention was supported, in whole or in part, by National Institutes of Health Grant Number P01 GM56552. The United States Government has certain rights in the invention.

### BACKGROUND OF THE INVENTION

Structural studies of proteins from human immunodeficiency virus type 1 (HIV-1) have been essential in the development of anti-retroviral drugs. Structure-based drug development has been most intense for reverse transcriptase inhibitors and protease inhibitors, the two classes of HIV-1 drugs in clinical use. It would also be useful to be able to carry out structure-based drug development against HIV entry.

### SUMMARY OF THE INVENTION

As described herein, the cavities on the surface of the N-helix coiled-coil of HIV envelope protein gp41 subunit (e.g., HIV-1 envelope protein gp41-subunit) are targets for drugs or other agents which, by binding the coiled-coil surface, particularly the cavities, inhibit HIV entry into cells. This is useful as the basis for identifying and designing drugs or agents which inhibit entry of HIV (e.g., HIV-1, HIV-2) into cells.

Results described herein show that the coiled-coil cavity (also referred to as the hydrophobic pocket) in the gp41 core is an attractive drug target and that molecules which bind the cavity interfere with (inhibit) HIV infectivity (HIV entry into cells). Applicants have shown, for the first time, that conserved residues projecting into the hydrophobic pocket clearly play a major role in the ability of C34 to inhibit HIV-1 infection. The importance of cavity contacts (between the N-helix coiled-coil cavity and residues of the C peptide region of gp41) to gp41 function is clear. Conversely, the importance of preventing such cavity contacts in inhibiting gp41 function and, thus, inhibiting HIV-1 entry into cells, is also clear. In addition, directing drugs against the hydrophobic pocket of the central-coiled coil of gp41 targets one of the most highly conserved regions of the HIV-1 envelope proteins, which means that drugs which target the coiled-coil surface, and particularly its hydrophobic pocket, will have broad activity against diverse HIV isolates and that it will be difficult for drug-escape mutants to emerge.

A variety of methods, such as mirror-image phage display techniques (T. N. Schumacher, et al., Science, 271:1854 (1996)), combinatorial chemistry (A. Borchardt, S. D. Liberles, S. R. Biggar, G.R. Crabtree, S.L. Schreiber, Chem.Biol., 4:961 (1997); J.C. Chabala, Curr. Opin. Biotechnol., 6:632 (1995)), rational drug design and other drug screening and medicinal chemistry methods can be used to identify D-peptides, peptidomimetics and small molecules that bind the coiled-coil cavity with sufficient affinity to inhibit HIV-1 infection. The close correlation between N36/C34 stability and C34 potency, described herein, suggests that the effectiveness of such compounds will depend critically on the strength of their cavity-contacts. As described herein, candidate compounds can be tested for their ability to interfere with formation of a stable complex between C34 and N36 or their ability to disrupt binding of the two (disrupt the complex), thereby providing rapid, quantitative screens to identify and evaluate potential inhibitors of HIV-1 entry.

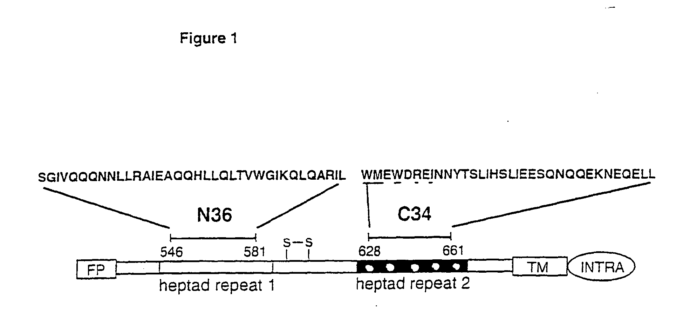

Alternatively, screening can be carried out to identify molecules or compounds which interfere with or disrupt binding of the N-helix coiled-coil cavity and a peptide which binds the cavity, thus providing methods of identifying molecules which are "pocket specific" binding agents or drugs. Molecules and compounds described herein (also referred to as drugs or agents) are useful to inactivate gp41 and, thus, prevent or reduce (inhibit) HIV-1 entry into cells. Without wishing to be bound by theory, it is reasonable to propose that these inhibitors bind to the pre-hairpin intermediate of gp41 and prevent its conversion to the trimeric hairpin structure of the gp41 core which corresponds to the fusion-active state of gp41. (Chan, D.C. and P.S. Kim, Cell, 93:681 (1998), See Figure 1). Thus, the present methods are useful to identify drugs or agents which inhibit (totally or partially) formation of the fusion-active state of HIV-1 gp41 envelope protein. In the method, the ability of a candidate inhibitor (also referred to as a candidate drug), which can be any type of compound or molecule, such as a small molecule (e.g., a small organic molecule), a peptide (a D-peptide or an L-peptide), a peptidomimetic, a protein or an antibody, to bind the N-helix coiled-coil of gp41 and form a stable complex is assessed. Compounds or molecules which bind to the N-helix coiled-coil are further assessed for their ability to inhibit gp41 function (inhibit membrane fusion), such as through HIV-1 infection (viral entry) and syncytium assays, representative models of which are described and referenced herein. Those agents shown to inhibit gp41 function through such assays can be further assessed for their activity in additional *in vitro* assays and in appropriate animal models (e.g., Letvin, N.L., Science, 280, (5371): 1875 - 1880 (1998), Hirsch, V.M. and P.R. Johnson, Virus Research, 32 (2): 183-203 (1994); Reimann, K.A. et al., J. Vivol., 70 (10): 6922-6928 (1996)). Any suitable approach can be used to assess binding of candidate inhibitors to the N-helix coiled-coil and, as a result of the work described herein, to the N-helix coiled coil cavity. In one embodiment, the ability of a candidate inhibitor to bind the synthetic peptide N36 (described in Lu, M. et al., J. Biomol. Struct. Dyn. 15: 465 (1997), Chan, D.C. et al., Cell, 89, 263 (1997) and U.S. Provisional Application 60/043,280, entitled Core Structure of gp41 From the HIV Envelope Glycoprotein, by David C. Chan, Deborah Fass, Min Lu, James M. Berger and Peter S. Kim, filed April 17, 1997) is assessed. The stability of the resulting complexes is assessed using methods described herein.

In a particular embodiment of the method of identifying compounds or molecules (drugs or agents) which bind the N-helix coiled-coil cavity, a soluble model that presents the gp41 coiled-coil cavity is used. The six helix bundle of HIV gp41 consists of an internal trimeric coiled-coil, composed of three identical N-peptides, surrounded by three C-peptides which fit into a conserved hydrophobic groove on the outside of the trimeric coiled-coil. The C-terminal end of the trimeric coiled-coil contains a large cavity into which bulky hydrophobic groups from the C-peptide pack. This hydrophobic pocket is used as the target for anti-HIV drug discovery and/or design. Unfortunately, in the absence of the C-peptide, the N-peptide is aggregated and not 100% helical. Thus, simply using an N peptide from HIV-1 gp41, such as N36, N51 (Lu, M. et al.. Nature Struct. Biology, 1995) or DP-107 (Wild et al., PNAS 89:10537-10541 (1992) is unlikely to provide an effective model for the N-helix coiled-coil.

As described herein, Applicants have succeeded in producing a soluble, non-aggregating trimeric peptide model of the hydrophobic pocket of HIV gp41 and, thus, for the first time, have provided a model that properly presents this hydrophobic pocket or cavity (in a manner or configuration which forms a similar structure to the corresponding residues in the HIV gp41 structure). (The terms "pocket" and "cavity" are used interchangeably.) As described, a peptide (also referred to as a fusion protein) which includes a soluble, trimeric coiled coil portion and a portion from the N-peptide region of HIV gp41 that includes the amino acid residues which form the pocket or cavity of the N-helix coiled-coil of HIVgp41 (the pocket-comprising residues of the N-peptide) has been produced and shown to be such a soluble model, useful to identify molecules or compounds which inhibit HIV gp41 function and, thus, HIV entry into cells. The trimeric version of the coiled-coil in the peptide (also referred to as a fusion protein) can be the coiled-coil region of a protein which is not a protein of HIV (a non HIV protein, such as GCN4-pI_{Q}I) or a protein of HIV origin (a protein derived from HIV or having the same or a similar amino acid sequence as an HIV protein). In a specific embodiment, the soluble, non-aggregating trimeric peptide model of the large cavity, referred to as IQN17, comprises a trimeric version of the coiled-coil region of GCN4, the yeast transcription activator, and a portion of the C-terminal end of the N peptide of gp41. IQN17 contains 29 residues of GCN4-pI_{Q}I (formerly referred to as GCN4-pIQ in U.S. Provisional Application 60/101,058) (Eckert, D.M. et al. J. Mol. Biol., 284:859-865 (1998)), including three mutations for increased solubility, and 17 residues of HIV; there is a one residue overlap between the two proteins, making the total length of the fusion protein 45 residues. The sequence of GCN4-pI_{Q}I is: ac-RMKQIEDKIEEI LSKQYHIENEIAR IKKLIGER (SEQ ID NO:1). The HIV Sequence is: LLQLTVWG IKQLQARIL (SEQ ID NO:20). The sequence of IQN17 is: ac-RMKQIEDKIEEIESKQKKIENEIARIKK LLQLTVWGIKQLQARIL-am (SEQ ID No:2). The HIV portion is underlined in SEQ ID No: 2; ac- represents an N-terminal acetyl group and -am represents a C-terminal amide. The sequence of the soluble, trimeric version of the coiled-coil region of GCN4 (referred to as a soluble, trimeric coiled coil of GCN4) in IQN17 is:
RMKQIEDKIEEIESKQKKIENEIARIKK (SEQ ID No: 25). The superhelix parameters such as rise and pitch (Harbury, P.B. et al., Nature 371:80-83 (1994); Harbury et al., PNAS 92:8408-8412 (1995)) of the GCN4-pI_{Q}I coiled coil are nearly identical to the HIV gp41 N-helix coiled coil. Therefore, the resulting fusion protein molecule (IQN17) is predicted to form a long trimeric coiled coil, which presents the N-peptide hydrophobic cavity at the C terminus. IQN17 is fully helical, as determined by circular dichroism, with a molar ellipicity at 222nm of -36,000 deg cm² dmol-¹. As determined by sedimentation equilibrium, IQN17 is close to a discrete trimeric species with a ratio of observed molecular weight to calculated molecular weight ranging from 3.00 to 3.16 times the monomer molecular weight at a concentration of 20 µM. As determined by X-ray crystallography, IQN17 presents the N-peptide hydrophobic pocket in a manner that is nearly identical to the pocket in the HIV gp41 N-helix coiled coil.

The IQN17 molecule (in the natural L-handedness or enantiomeric D-handedness) can be used in screens, including high-throughput drug screens, to identify molecules that bind to the coiled-coil pocket. The IQN17 molecule, in the D-handedness, has been used as a target in mirror image phage display (Schumacher et al., Science, 271: 1854, 1996) to identify small molecules (D-peptides) which bind to the hydrophobic pocket of gp41 (in the natural L-handedness) and inhibit HIV-membrane fusion. The desired target (the N-helix of HIV gp41 which includes the hydrophobic pocket) is the exact mirror image of the naturally-occurring target. It is used to screen a library or collection of compounds or molecules which are to be assessed for their ability to bind the mirror image of the naturally-ocurring coiled-coil pocket. The mirror image of a compound or molecule found to bind the mirror image of the naturally-occurring gp41 pocket, will bind the gp41 pocket in the natural handedness. The library or collection screened can be of any type, such as a phage display library, peptide library, DNA library, RNA library, combinatorial library, collection of chemical agents or drugs, cell lysate, cell culture medium or supernatant containing products produced by cells. In the case of a phage display library, the D-target is used to screen phage coat proteins. Specific phage clones that bind to the target are identified and the mirror images of the expressed proteins are chemically synthesized with D-amino acids. By using IQN17 in mirror-image phage display, D-peptides that bind to the gp41 hydrophobic pocket have been identified. Further assessment has been carried out, as described, to demonstrate the ability of D-peptides to inhibit HIV gp41 function. D-peptides which bind the gp41 hydrophobic pocket and inhibit HIV infectivity have been identified. D-peptides which bind the hydrophobic pocket also will serve as lead molecules for drug development and/or reagents for drug discovery (where the drugs bind to the coiled-coil pocket and inhibit HIV infectivity). The IQN17 molecule, in the natural L-handedness, can be used in screens, including high-throughput screens, to identify molecules that bind to the coiled-coil pocket. IQN17 can be used to screen a collection or library of compounds or molecules which are to be assessed for their ability to bind the hydrophobic pocket. The library or collection screened can be of any type, such as a phage display library, RNA library, DNA library, peptide library, combinatorial library, collection of chemical agents or drugs, cell lysate, cell culture medium or supernatant containing products produced by cells. Compounds or molecules which bind the hydrophobic pocket also will serve as lead molecules for drug development and/or reagents for drug discovery.

Fusion proteins which are variants of IQN17 can be produced and used to screen for drugs which bind the gp41 N-helix coiled-coil pocket. Any of a wide variety of variations can be made in the GCN4-pI_{Q}I component of IQN17 and used in the method, provided that these changes do not alter the trimeric state of the coiled-coil. For example, the amino acid composition of the GCN4 component can be changed by the addition, substitution, modification and/or deletion of one or more amino acid residues, provided that the trimeric state of the coiled-coil is maintained. For example, the Asp residue in IQN17 (at a "f-position" of the coiled coil) can be replaced by any of the naturally-occurring amino acids. (O'Neil and DeGrado, Science 250:646 (1990)). Alternatively, this component of the fusion protein can be a trimeric version of the coiled-coil region of another protein, such as that from Moloney Murine Leukemia Virus (Fass, D. et al. Nature Struct. Biology, 3:465 (1996)), GCN4-pII (Harbury et al., Nature, 317:80, 1994) or the ABC heterotrimer (Nautiyal and Alber, Protein Science 8:84 (1999)).

Changes can also be made in the amino acid composition of the fusion protein component which is the C-terminal portion of the HIV gp41 N peptide to produce IQN17 variants. The C-terminal portion can be changed by the addition, substitution, modification and/or deletion of one or more amino acid residues. The amino acid composition of either or both components of the fusion protein can be altered, and there is no limit to the number or types of amino acid residue changes possible, provided that the trimeric state of the coiled-coil and the hydrophobic pocket of the N peptide of HIV gp41 are maintained. IQN17, IQN17 variants or any soluble model of the large cavity can be used to screen for drugs which bind the N-helix coiled-coil, especially the pocket, or for lead drug candidates or candidates for use in vaccine preparations, to be further screened using methods known to those of skill in the art, such as in a high throughput format.

Results described herein are useful to screen for inhibitors of HIV gp41 which are variants of C34 as described below. Once a variant of C34, such as a C34 variant which stably binds N36, has been identified, it can be used and further assessed as obtained or it can be modified (e.g., by altering, adding, deleting or substituting at least one amino acid residue or adding a non-amino acid substituent), if desired or needed (e.g., to enhance stability, solubility, bioavailability). Alternatively, a C34 variant can be assessed to determine if a shorter component (region of fewer amino acid residues) also is active as an inhibitor. As discussed herein, the three C34 residues Trp⁶²⁸, Trp⁶³¹ and Ile⁶³⁵ that pack into the deep, conserved pocket in the N36 trimer are critical for inhibitory activity. The observation that C34 variants that have a higher affinity for the N36 coiled-coil have more potent inhibitory activity against HIV infection forms the basis for screens to identify and evaluate potential inhibitors. For example, using the "split-synthesis" technique (Chen, C.L., et al. Methods Enzymol. 267:211-219 (1996*)*; Lam, K.S. et al., Nature, 354: 82-84, (1991)) of combinatorial peptide chemistry, a library of C34 variants is synthesized in which the three critical hydrophobic residues are randomly replaced by chemical substitutions of varying hydrophobic character. This synthesis technique results in the generation of a vast library of beads, each containing many copies of a single variant C34 peptide (i.e., a "one-bead, one-compound" type of library). To identify C34 variants which stably bind the N-helix coiled-coil, a labeled version of N36 (or a modified N-peptide) is mixed with the peptide beads under conditions (e.g., elevated temperature) that restrict binding to only those C34 variants with the highest affinity. Binding is measured by detection of the label on the N-helix peptide, using known methods. Simple modifications of the split-synthesis technique allow ready identification of the selected peptide sequence by mass spectroscopy (Youngquist, R.S. et al., J. Amer. Chem. Soc. 117, 3900-3906 (1995)). The C34 variants selected, particularly those with the highest binding affinities for N36, are tested in syncytium and infection assays for gp41 inhibitory activity. Truncated versions of these C34 variants, containing only the cavity-binding region, can also be tested for inhibitory activity. Alternatively, a library of other peptides to be assessed can be synthesized to generate a library of beads, each containing (having bound thereto) a peptide to be assessed. This library is analyzed as described above for the C34 variants and resulting hits (members with appropriate binding affinities for N36) are further analyzed for gp41 inhibitory activity. As a second example, the N36 peptide or the soluble variants described earlier, such as IQN17, GCN4-N-helix peptide can be used as a target for phage display or mirror-image phage display techniques to identify peptides that bind to the cavity.

IQN17 can also be used to raise antibodies (monoclonal and/or polyclonal) that bind to the coiled-coil cavity. IQN17 can further be used, either alone or in combination with other materials, in a vaccine, which will elicit the production of antibodies that bind to the coiled-coil in the individual to whom it is administered (the vaccine), and thereby offer protection against infection and/or disease.

Peptides, both D-peptides and L-peptides, as defined in the claims appended hereto, which fit into a deep hydrophobic pocket in the trimeric N-helix coiled-coil of HIV-1 envelope glycoprotein gp41 are also the subject of this invention. The D-peptides are the first molecules that have been shown to bind exclusively to the gp41 hydrophobic pocket. The observation that these D-peptides inhibit gp41-mediated membrane fusion processes (syncytia formation and viral infection) provides the first direct demonstration that HIV-1 infection can be inhibited by molecules that bind specifically to pocket. The validation of the gp41 hydrophobic pocket as a drug target sets the stage for the development of a new class of orally bioavailable anti-HIV drugs, that work by inhibiting viral entry into cells. Such drugs would be a useful addition to the current regimen used to treat HIV-1 infection with combination therapies. D-peptides, such as the D-peptides described herein, portions, modification and variants thereof and larger molecules (e.g., polypeptide) which comprise all or a portion of a D-peptide described herein, are useful to inhibit HIV membrane fusion and, thus, HIV entry into cells. D-peptides, corresponding to the D-amino acid version of phage sequences identified as described herein, are inhibitors of HTV-1 infection and syncytia formation. The C-terminal residues in these D-peptide inhibitors have the sequence pattern: CXXXXXEWXWLCAA-am. (in the phage-display library, the positions corresponding to the C residues were encoded as either C or S, the positions corresponding to the AA residue were encoded as such and the other 10 positions (indicated by X) were randomly encoded. The -am represents a C-terminal amide, added as part of the peptide synthesis procedure.) The N-terminal residues in the D-peptide inhibitors are, for example, ac-GA, ac-KKGA, or ac-KKKKGA. The ac- represents an N-terminal acetyl group added as part of the peptide synthesis procedure. The C-terminal amide and the N-terminal acetyl group are optional components of D-peptides of this invention. Other N-terminal residues can be included, in place of or in addition to those in the previous sentence, as desired (e.g., to increase solubility). For example, D-peptides of the following sequences are also the subject of this invention:
ac-XXCXXXXXEWXWLCXX-am (SEQ ID NO: 28);
ac-KKXXCXXXXXEWXWLCXX-am (SEQ ID NO: 29);
ac-KKKKXXCXXXXXEWXWLCXX-am (SEQ ID NO: 30);
ac-XXCXXXXXEWXWLCXXX-am (SEQ ID NO: 31);
ac-KKXXCXXXXXEWXWLCXXX-am (SEQ ID NO: 32); and
ac-KKKKXXCXXXXXEWXWLCXXX-am (SEQ ID NO: 33).

The amino acid residues are represented by the single letter convention and X represents any amino acid residue (naturally occurring or non-naturally occurring) or other moiety, such as a modified amino acid residue.

Further, the ten amino acid residue "core" (the 10-mer which is flanked at each end by a cysteine residue) of the 12 amino acid residue peptide, as well as portions, modifications and variants of the 10-mers are also useful to inhibit membrane fusion and entry of HIV into cells. Variants, portions and modifications of these peptides are also useful as inhibitors. As described further herein, D-peptides which comprise a consensus sequence (e.g., WXWL (SEQ ID NO: 23), EWXWL (SEQ ID NO: 24), CXXXXXEWXWLC (SEQ ID NO: 63) or a portion thereof) have been shown to bind the N-helix coiled-coil and are useful to inhibit membrane fusion and entry of HIV into cells. The enantiomeric peptides (D-peptides) do not serve as efficient substrates for enzymes, such as proteases and, therefore, are more resistant to proteolytic degradation than are L-peptides; they are also less immunogenic than are L-peptides.

Specific embodiments of D-peptides of the present invention are:
(a) CDLKAKEWFWLC (SEQ ID NO: 3);
(b) CEARHREWAWLC (SEQ ID NO: 4);
(c) CELLGWEWAWLC (SEQ ID NO: 5);
(d) CLLRAPEWGWLC (SEQ ID NO: 6);
(e) CSRSQPEWEWLC (SEQ ID NO: 7);
(f) CGLGQEEWFWLC (SEQ ID NO: 8);
(g) CMRGEWEWSWLC (SEQ ID NO: 9);
(h) CPPLNKEWAWLC (SEQ ID NO: 10);
(i) CVLKAKEWFWLC (SEQ ID NO: 11);
(j) KKGACGLGQEEWFWLC (SEQ ID NO: 15);
(k) KKGACELLGWEWAWLC (SEQ ID NO: 16);
(l) KKKKGACELLGWEWAWLC (SEQ ID NO: 17);
(m) KKGACMRGEWEWSWLC (SEQ ID NO: 18);
(n) KKGACPPLNKEWAWLC (SEQ ID NO: 19);
(o) a D-peptide comprising WXWL (SEQ ID NO: 23);
(p) a D-peptide comprising EWXWL (SEQ ID NO: 24);
(q) a D-peptide comprising CXXXXXEWXWL (SEQ ID NO: 12)
(r) ac-GACEARHREWAWLCAA-am (SEQ ID NO: 34);
(r) ac-KKGACEARHREWAWLCAA-am (SEQ ID NO: 38);
(t) ac-KKKKGACEARHREWAWLCAA-am (SEQ ID NO: 43);
(u) ac-GACGLGQEEWFWLCAA-am (SEQ ID NO: 44);
(v) ac-KKGACGLGQEEWFWLCAA-am (SEQ ID NO: 64);
(w) ac-KKKKGACGLGQEEWFWLCAA-am (SEQ ID NO: 45)
(x) ac-GACDLKAKEWFWLCAA-am (SEQ ID NO: 35);
(y) ac-KKGACDLKAKEWFWLCAA-am (SEQ ID NO: 39);
(z) ac-KKKKGACDLKAKEWFWLCAA-am (SEQ ID NO: 46);
(a') ac-GACELLGWEWAWLCC-am (SEQ ID NO: 47);
(b') ac-KKGACELLGWEWAWLCAA-am (SEQ ID NO: 65);
(c') ac-KKKKGACELLGWEWAWLCAA-am (SEQ ID NO: 66);
(d') ac-GACSRSQPEWEWLCAA-am (SEQ ID NO: 36);
(e') ac-KKGACSKSQPHWHWLCAA-am (SEQ ID NO: 40);
(f') ac-KKKKGACSRSQPEWEWLCAA-am (SEQ ID NO: 48);
(g') ac-GACLLRAPEWGWLCAA-am (SEQ ID NO: 37);
(h') ac-KKGACLLRAPAWGWLCAA-am (SEQ ID NO: 41);
(i') ac-KKKKGACLLRAPEWGWLCAA-am (SEQ ID NO: 49);
(j') ac-GACMRGEWEWSWLCAA-am (SEQ ID NO: 50);
(k') ac-KKGACMRGEWEWSWCAA-am (SEQ ID NO: 18);
(l') ac-KKKKGACMRGEWEWSWLCAA-am (SEQ ID NO: 51);
(m') ac-GACPPLNKEWAWLCAA-am (SEQ ID NO: 52);
(n') ac-KKGACPPLNKEWAWLCAA-am (SEQ ID NO: 19);
(o') ac-KKKKGACPPLNKWAWLCAA-am (SEQ ID NO: 53);
(p') ac-GACXXXXXEWXWLCAA-am (SEQ ID NO: 54);
(q') ac-KKGACXXXXXEWXWLCAA-am (SEQ ID NO: 55);
(r') ac-KKKKGACXXXXXBWXWLCAA-am (SEQ ID NO: 56);
(s') ac-XXCXXXXXEWXEWLCXX-am (SEQ ID NO: 57);
(t') ac-KKXXCXXXXXEWLCXX-am (SEQ ID NO: 58);
(u') ac-KKKKXXCXXXXXEWXWLCXX-am (SEQ ID NO: 59);
(v') ac-XXCXXXXXEWXWLCXXX-am (SEQ ID NO: 60);
(w') ac-KKXXCXXXXXEWXWLCXXX-am (SEQ ID NO: 61); and
(x') ac-KKKKXXCXXXXXEWXWLCXXX-am (SEQ ID NO: 62):
wherein ac- at the N-terminus and -am at the C-terminus are optical.

D-peptides described herein, which are ligands shown to bind the N-helix pocket, are also useful in drug screens to identify compounds or molecules (e.g., from chemical libraries, recombinantly produced products, naturally-occurring substances, culture media or supernatants) which bind the N-helix pocket and thus, are also inhibitors of HIV. For example, a competitive assay can be carried out by combining a D-peptide which binds the N-helix cavity (e.g., a D-peptide described herein); IQN17 (e.g., in the natural L-handedness), or another fusion protein which is a soluble model that presents the N-helix cavity; and a candidate inhibitor (a compound or molecule to be assessed for its ability to bind the N-helix cavity). For example, D10pep5 or D10pep1, IQN17, and a candidate inhibitor (candidate drug) can be combined using buffer conditions and peptide concentrations appropriate for binding of D10pep5 or D10pep1 to IQN17. The extent to which binding of the D-peptide occurs is determined and compared to the extent to which binding occurs under the same conditions, but in the absence of a compound or molecule (referred to as a candidate drug or candidate inhibitor) to be assessed for its ability to bind the N-helix coiled-coil cavity of HIV gp41 envelope protein (in a control). If binding of D10pep5 or D10pep1 occurs to a lesser extent in the presence of the candidate inhibitor (test sample) than in its absence (control sample), the candidate inhibitor is a ligand which binds the N-helix coiled-coil cavity and, thus, is an inhibitor. Inhibitors identified in this manner can be further assessed for their activity in viral infectivity assays and synctia formation assays, such as those described herein. Those inhibitors which show activity in such assays can be further assessed in an appropriate animal model or in humans.

Any method by which binding of the D-peptide, known to bind the N-helix cavity, can be detected can be used to assess whether the candidate inhibitor interferes with binding. For example, the D-peptide can be detectably labeled and the extent to which the label appears on the N-helix cavity (as a result of binding of the D-peptide) detected, in the presence and in the absence of the candidate inhibitor. If less label appears on the N-helix cavity of IQN17 (or other appropriate fusion protein) in the presence of the candidate inhibitor (in the test sample) than in its absence (in the control sample), then the candidate inhibitor is a ligand which binds the N-helix cavity (and interferes with binding of the D-peptide). Alternatively, the D-peptide (e.g., D10peps or D10pep1) and IQN17 can be labeled with a fluorophore (e.g., with EDANS; 5-(2'aminoethyl)aminonaphthalene-1-sulfonic acid) with an appropriate quencher that quenches the fluorescent signal of the fluorophore when it is in close proximity (e.g., DABCYL; 4-(4'-dimethylaminophenylazo)benzoic acid). If the candidate inhibitor binds the N-helix cavity of IQN17, fluorescence is observed, since, as a result of binding of the candidate inhibitor, the D-peptide is not brought into sufficiently close proximity to the quencher to permit it to quench the reporter signal. Alternatively, the fluorescent reporter molecule can be on the IQN17 and an appropriate quencher on the D-peptide. In either case, the position of the reporter or quencher on IQN17 must be such that when the D-peptide binds the N-helix cavity, the reporter and quencher moieties are in sufficiently close proximity to each other that quenching occurs (Tyagi, S., et al., Nature Biotechnology 16:49 (1998)).

Also the subject of this invention are drugs (compounds and molecules) as defined in the claims appended hereto which bind the N-helix coiled-coil pocket of HIV gp41 and inhibit (partially or totally) HIV entry into cells. In one embodiment, these drugs can be identified as described herein or by other methods. Drugs which bind the N-helix coiled-coil pocket of HIV gp41 are useful as therapeutic agents (to prevent HIV entry into cells or reduce the extent to which it occurs), as research tools (e.g., to study the mechanism of HIV gp41 function) and to assess the rate of viral clearance by an individual (e.g., in an animal model or an infected human).

Also the subject of this invention are compositions, as defined in the claims appended hereto useful in methods of interfering with entry of HIV into a mucosal cell; these compositions comprise an appropriate carrier or base and at least one component selected from the group consisting of:
(a) C34 peptide;
(b) DP178;
(c) T649;
(d) T1249;
(e) a derivative of (a) - (d);
(f) a D-peptide which binds to the hydrophobic pocket of HIV gp41;
(g) a derivative of (f),
(h) is combination of two or more of (a)-(g); and
(i) a molecule that inhibits HIV infectivity by binding to the N-helix coiled coil.
The compositions can comprise one such component or two or more components.

A further subject of this invention are compositions (e.g., proteins or proteinaceous materials) as defined in the claims appended hereto that can be used to elicit an immune response (e.g., antibody production) that will protect (partially or totally) against HIV infection and/or disease. Such compositions are useful as protective agents (e.g., vaccines) and to obtain antibodies (monoclonal and/or polyclonal) that are useful as research tools, diagnostic tools, drug screening reagents, and to assess viral dynamics (rates of production and clearance of virus) in animal models or infected humans.

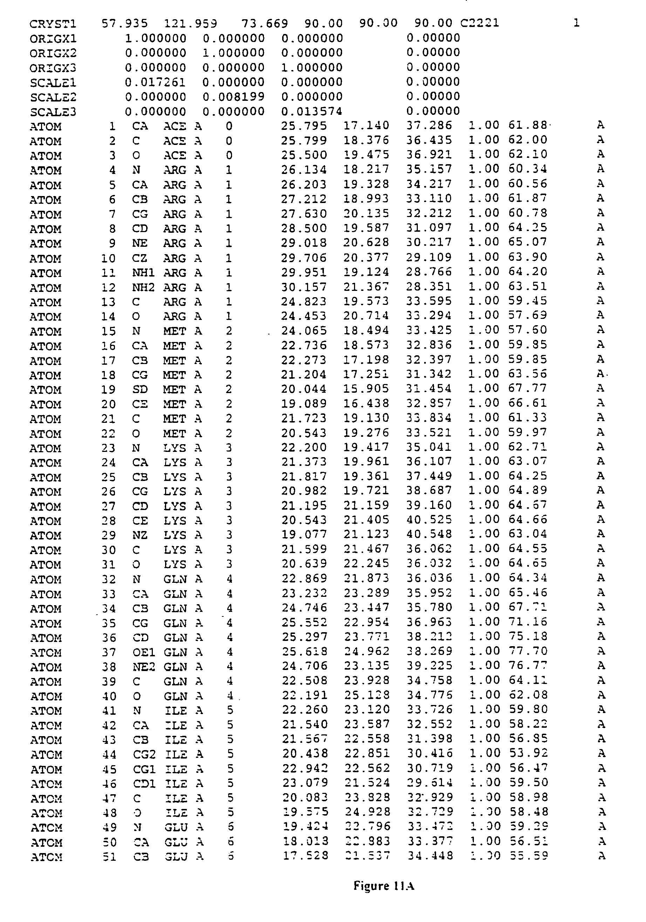

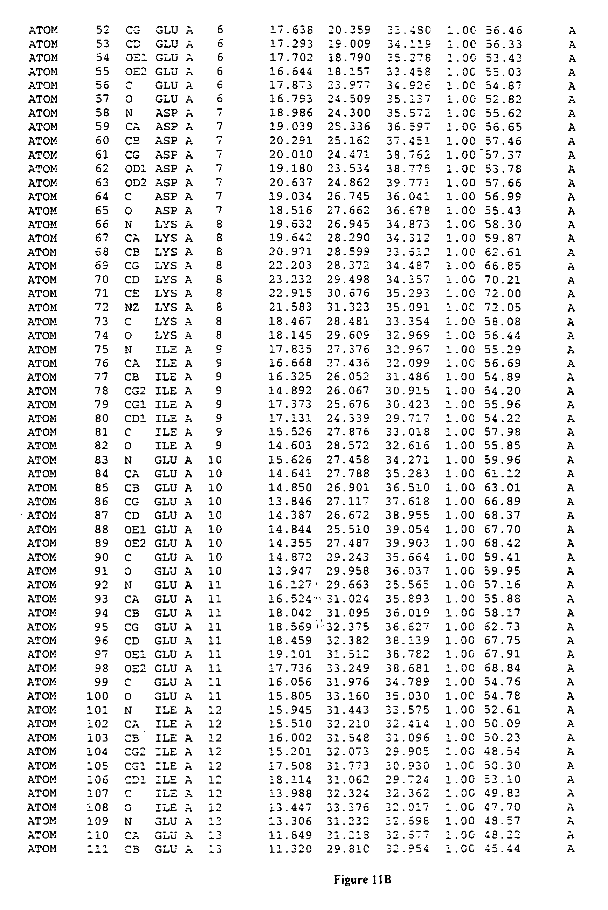

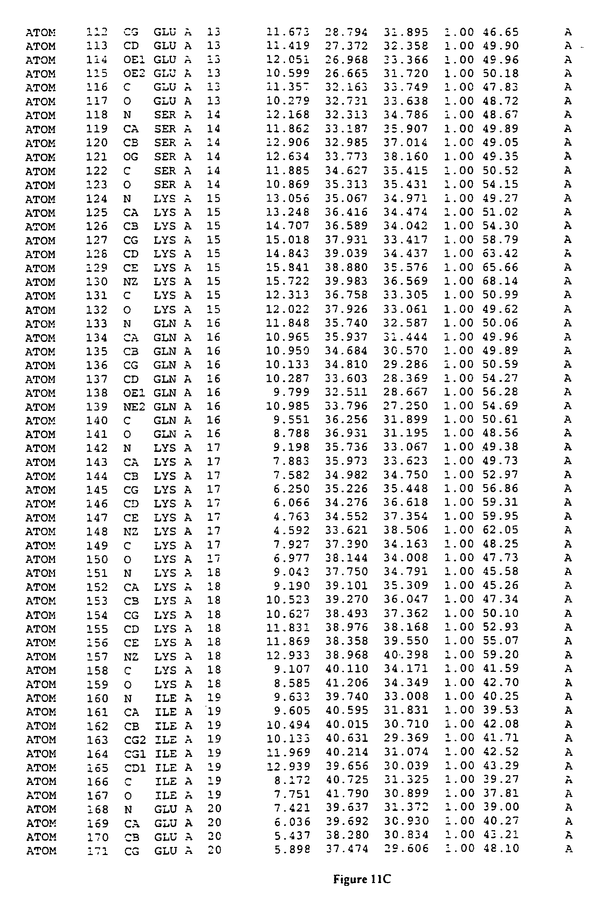

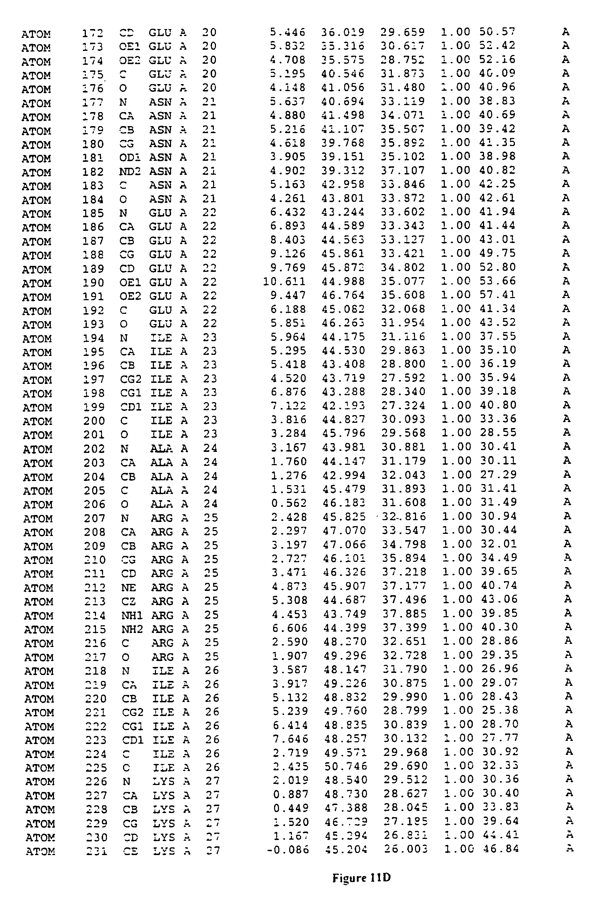

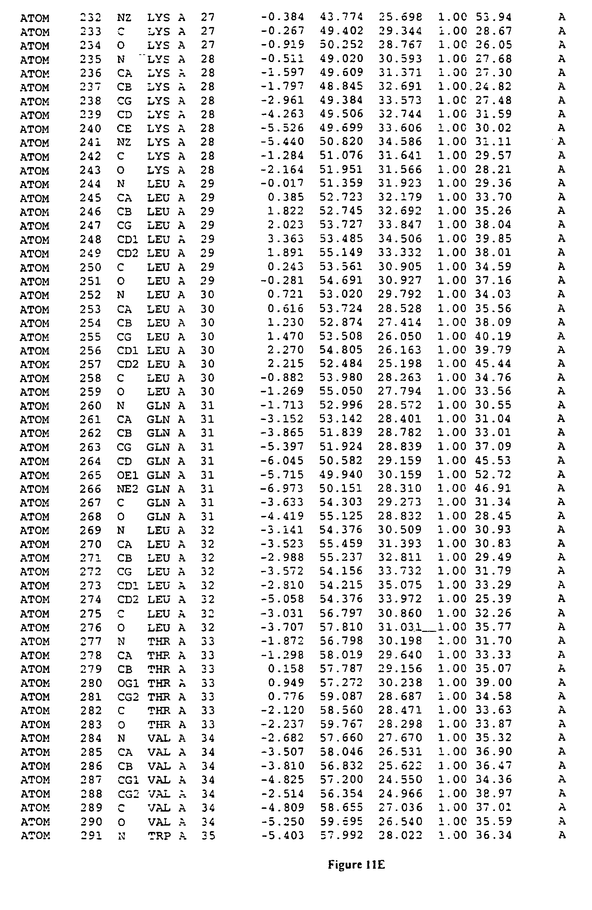

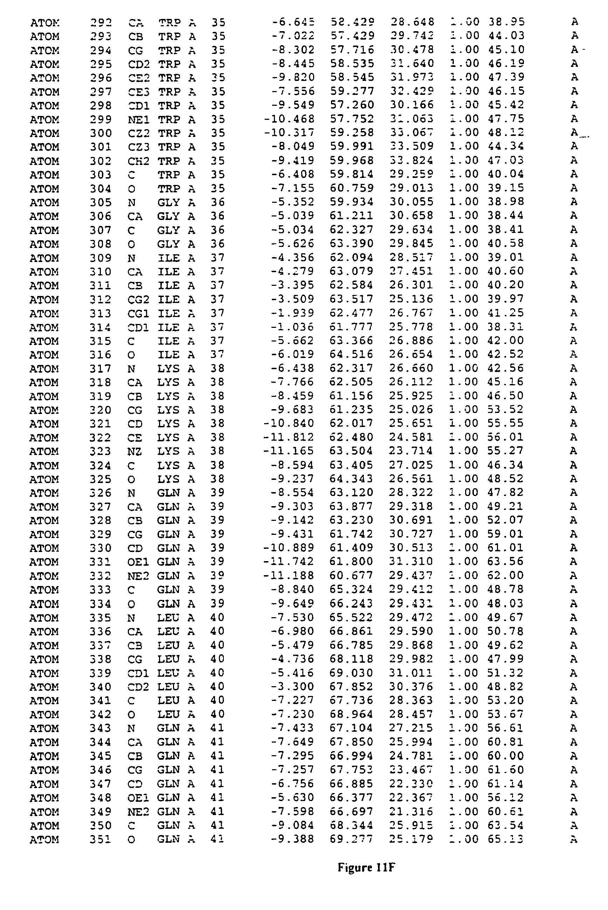

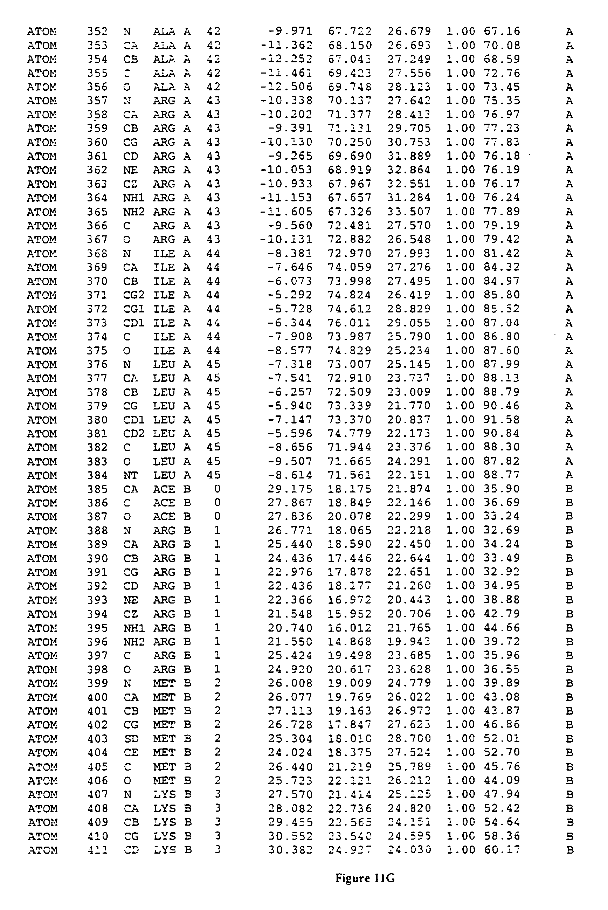

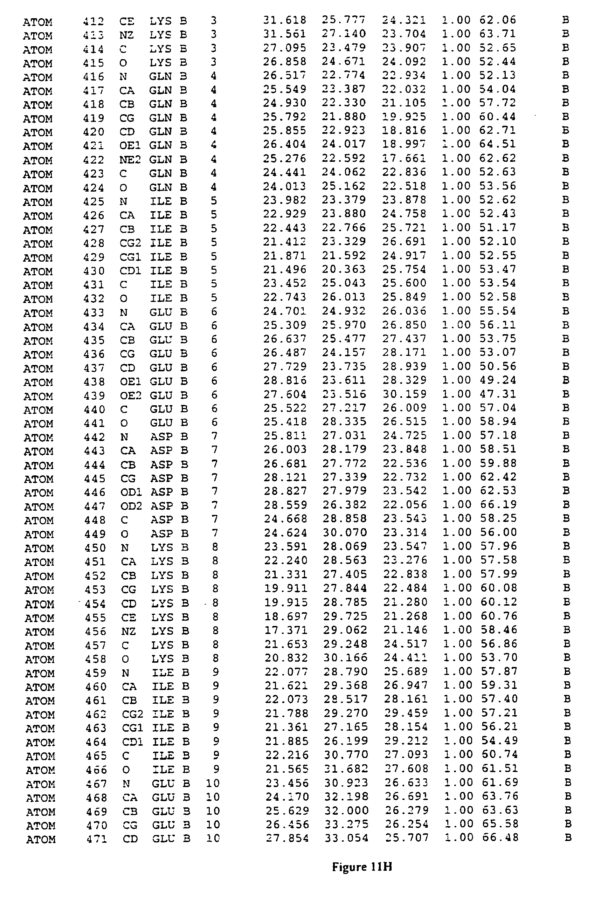

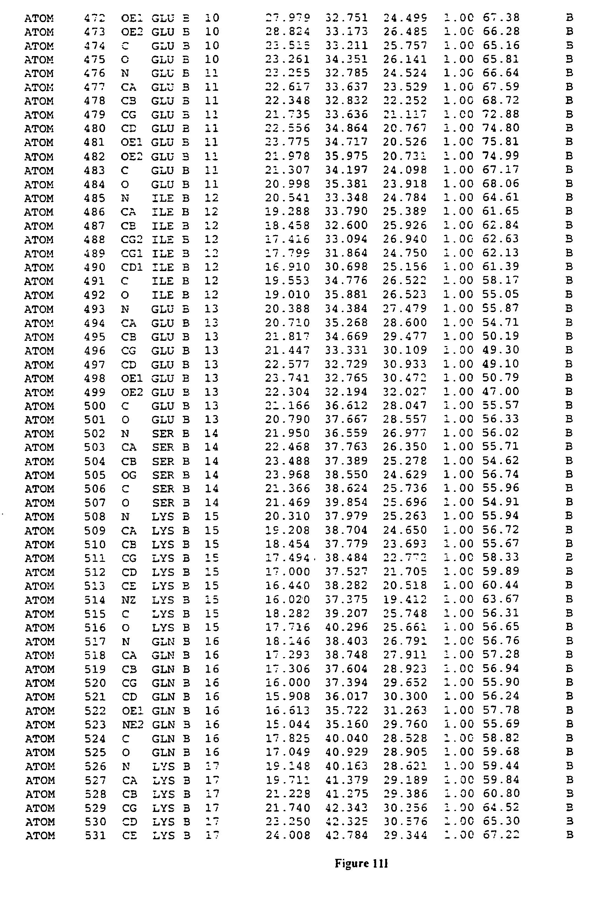

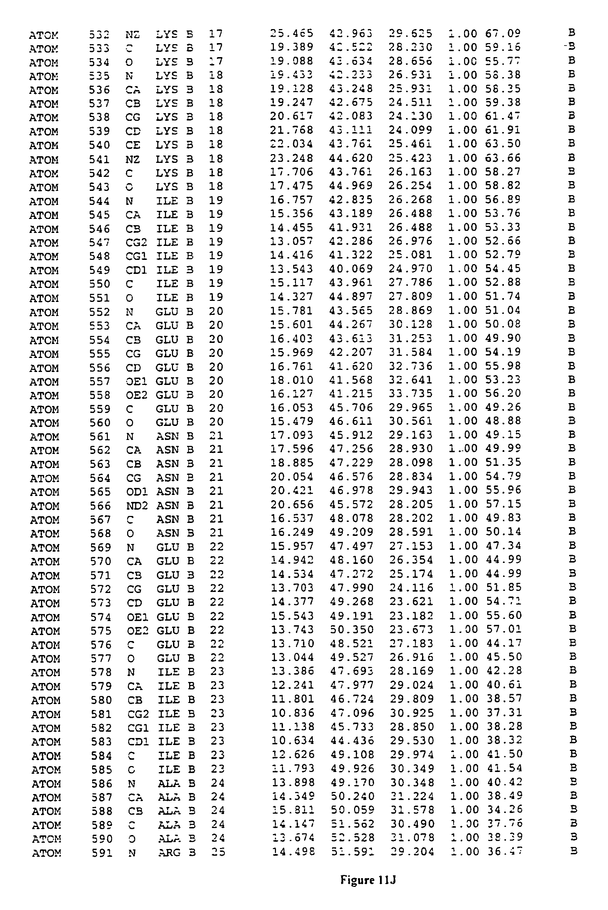

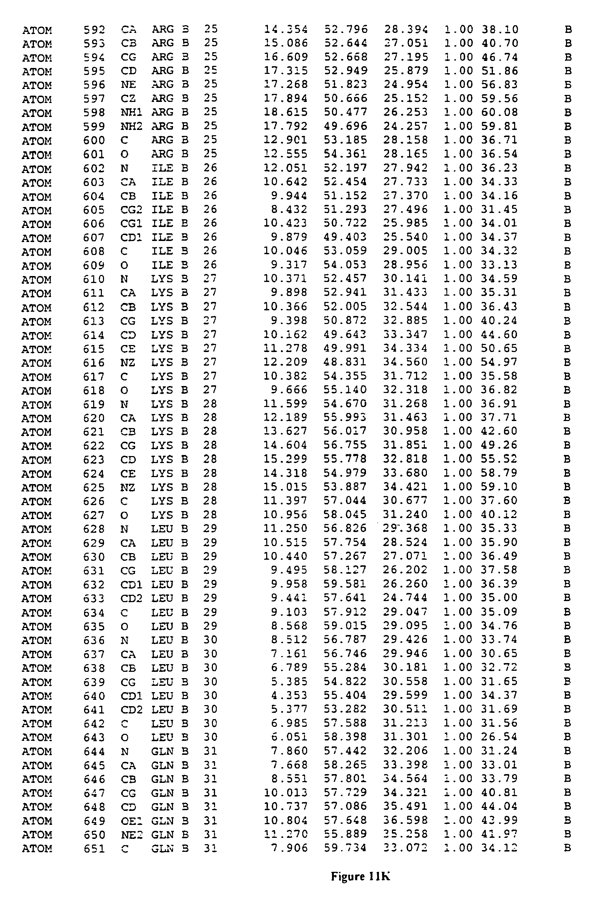

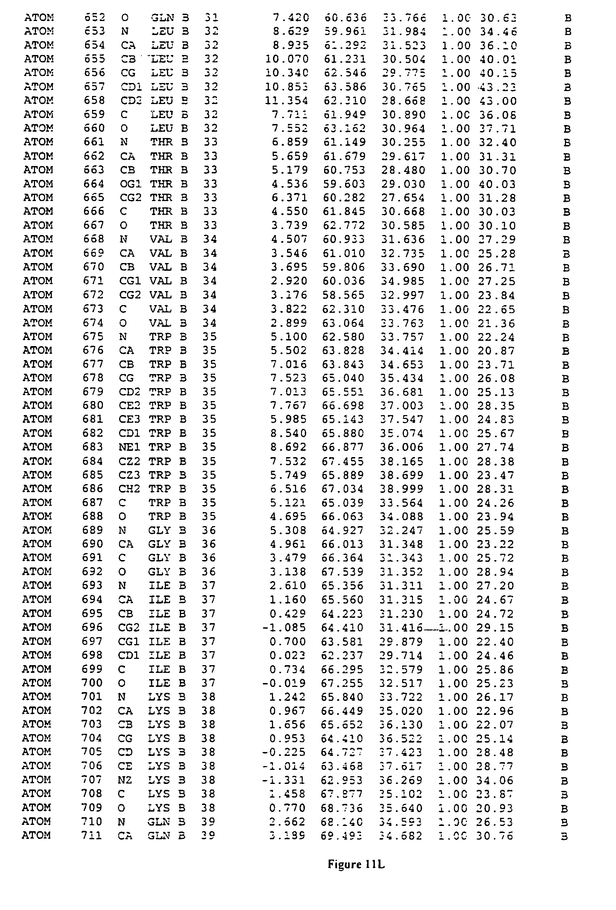

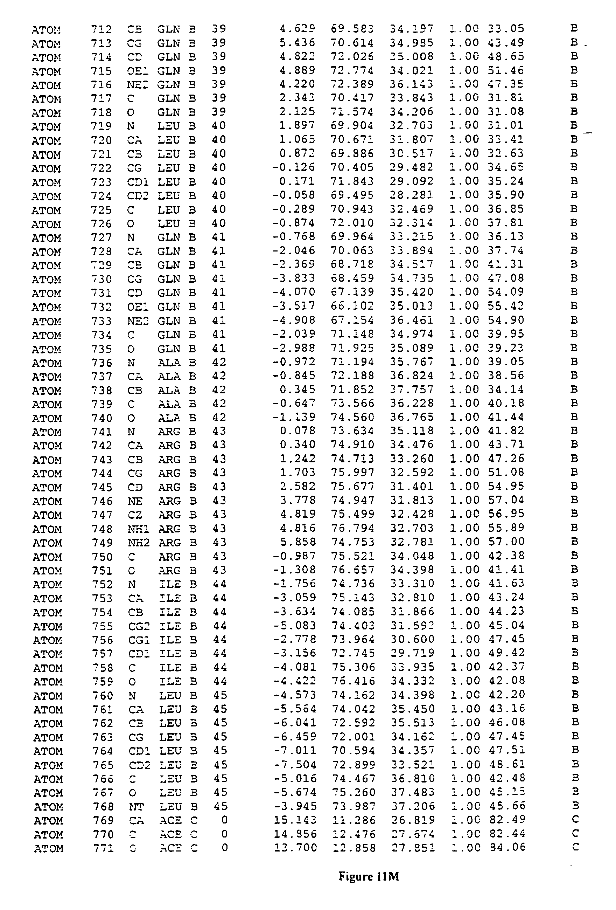

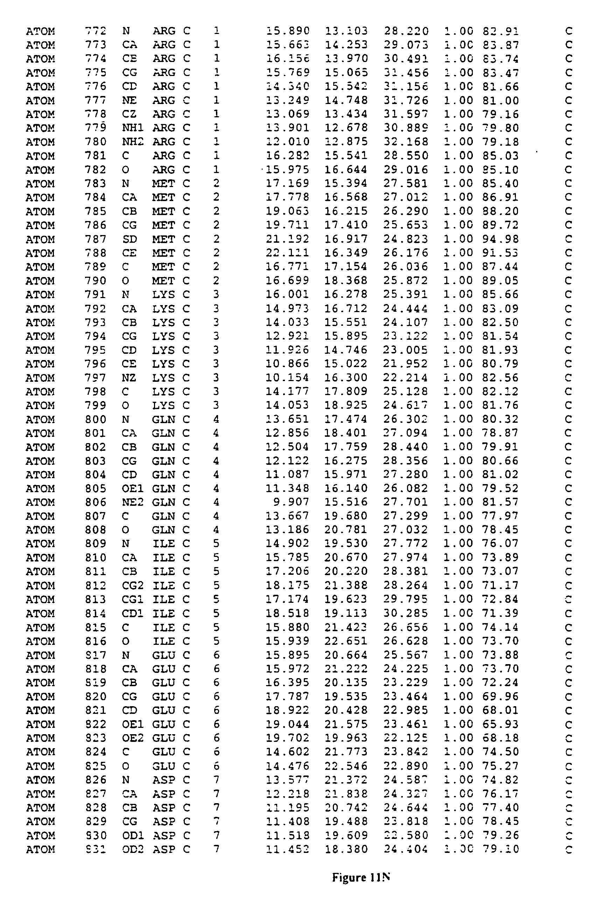

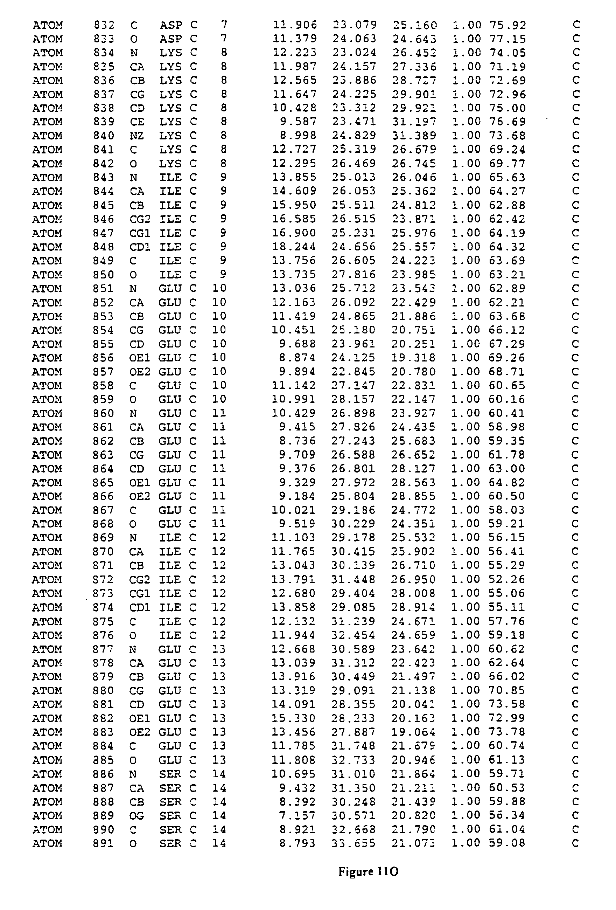

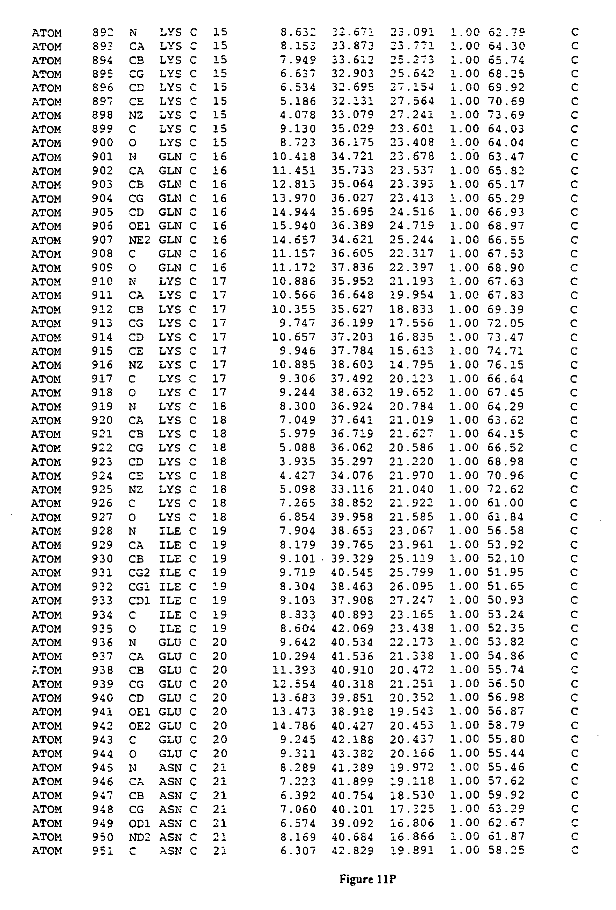

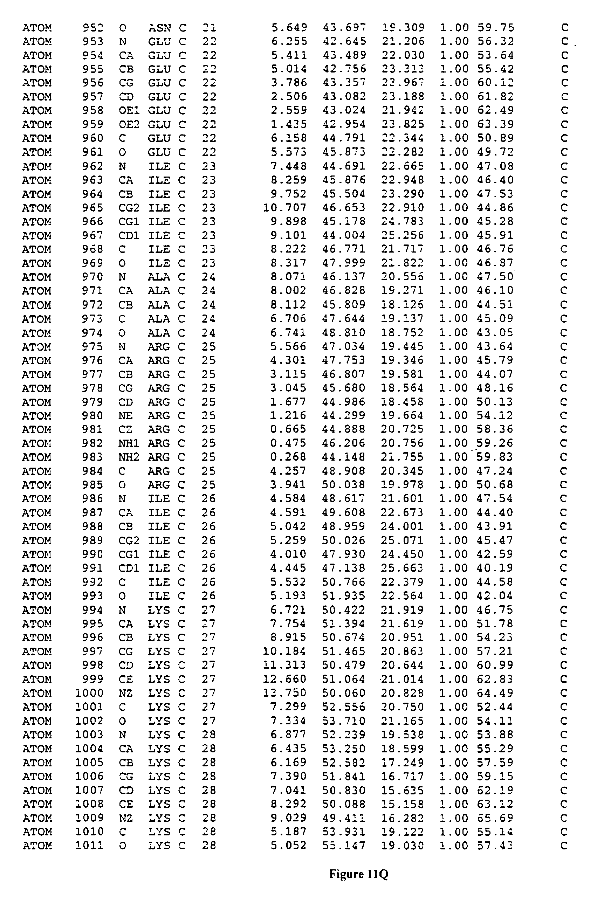

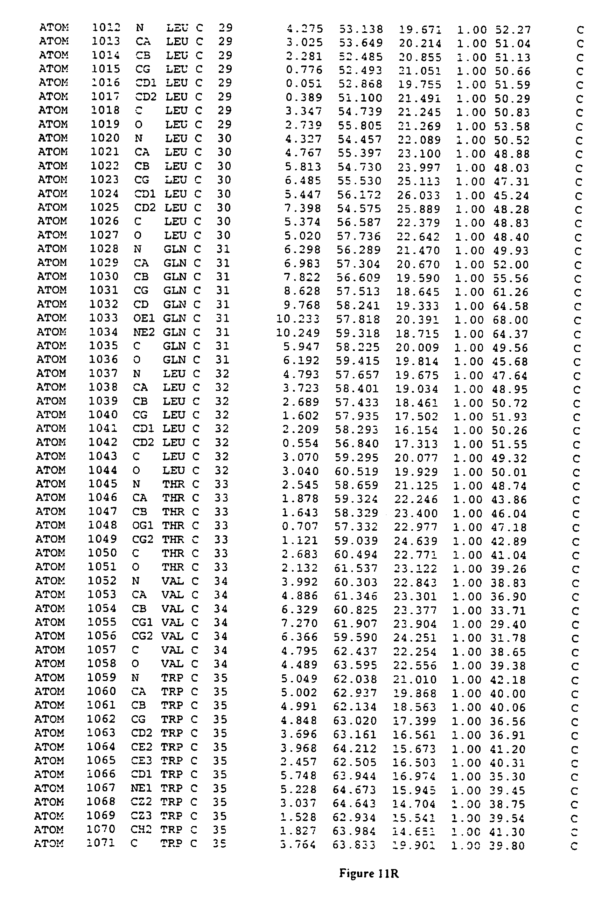

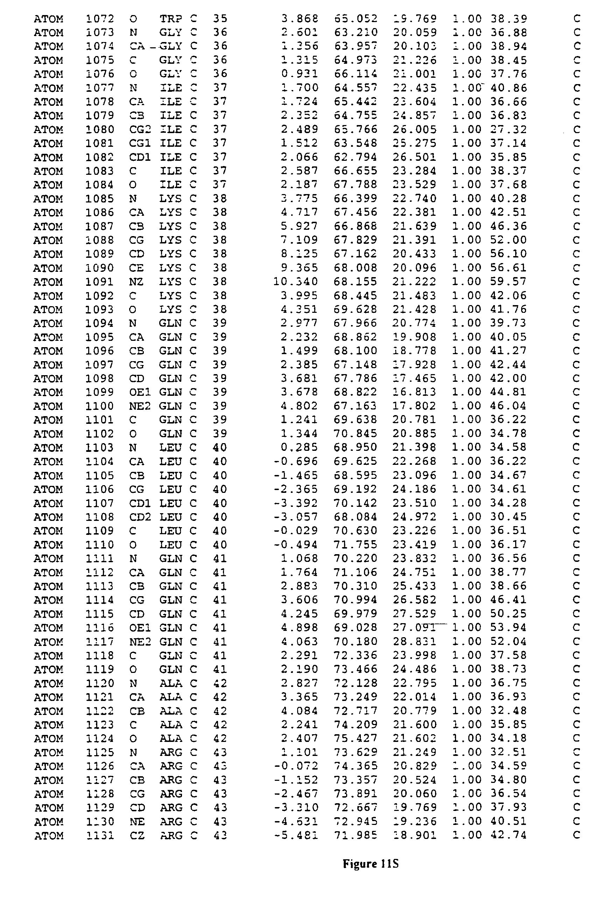

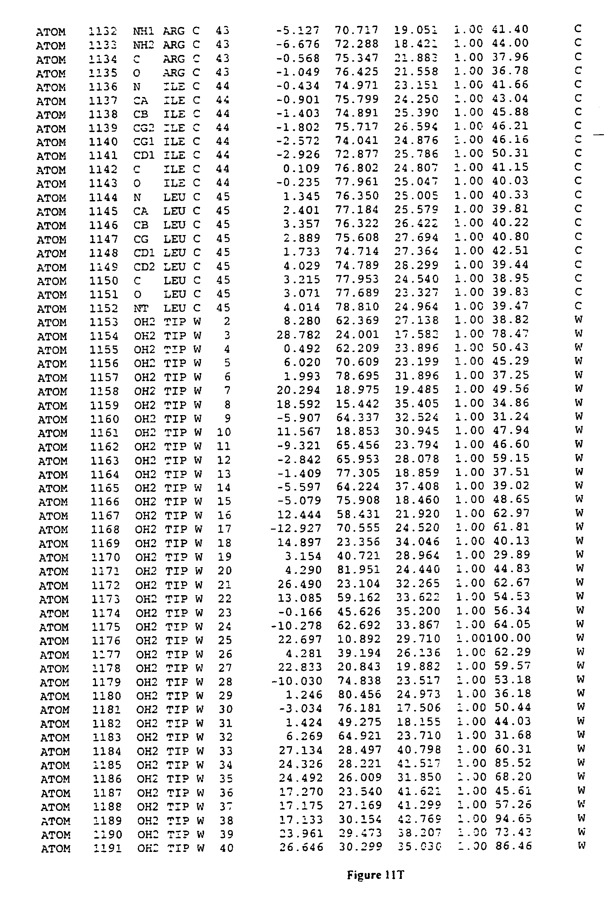

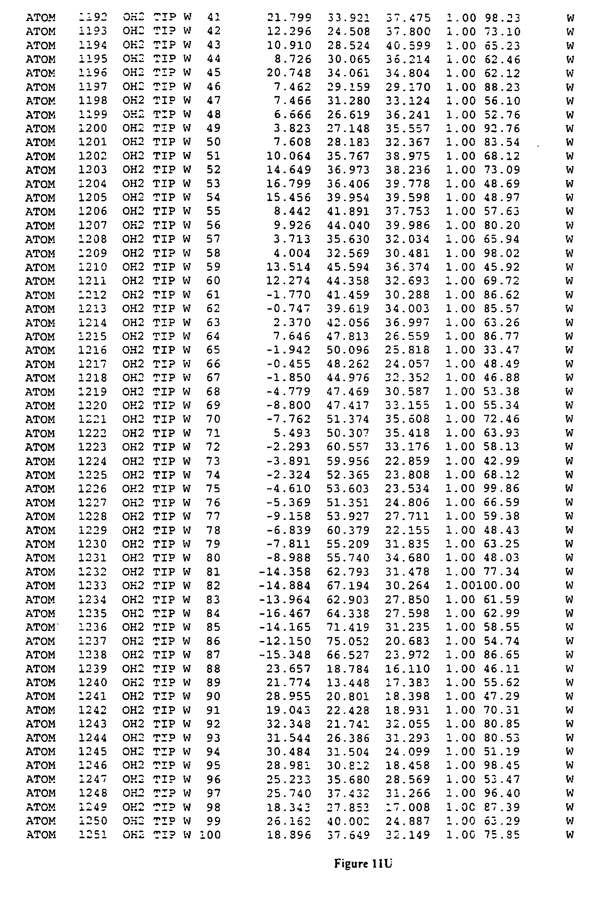

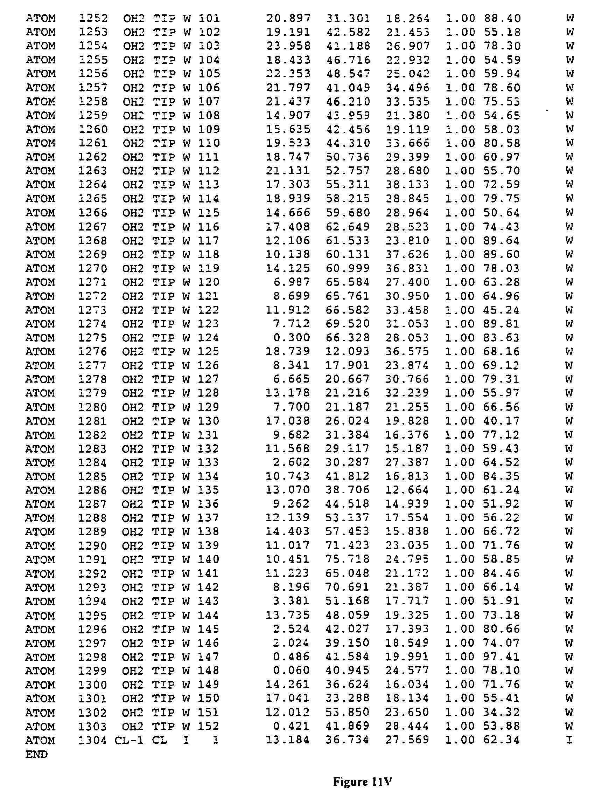

Also described herein is a list of atomic coordinates for the X-ray crystal structure of the complex between IQN17 and D10pep1; along with a list of coordinates for the X-ray crystal structure of IQN17. These coordinates can be used (e.g., as an electronic file for computer graphics programs) to create a model of the complex which indicates how D10pep1 binds to the N-helix coiled-coil cavity and models of the N-helix coiled-coil cavity. Such models can be used, in methods known to those of skill in the art such as in computer graphics modeling, to build new models to evaluate the likelihood of binding to the N-helix coiled-coil cavity by other Peptides, peptidomimetics, small molecules, drugs or other compounds. Such models can also be used to build new models for the structures of molecules (peptides, peptidomimetics, small organic molecules, drugs or other compounds) that bind the N-helix coiled-coil cavity (e.g., H. Kubinyi, Curr. Op. Drug Discov. Develop., 1:16 (1998); P.L. Wood, ibid, 1:34 (1998); J.R. Morphy, ibid. 1:59 (1998)). These models and the corresponding lists of atomic coordinates can be used to identify, evaluate, discover and design more effective and/or new D-peptides, L-peptides, peptidomimetics, other small molecules or drugs that inhibit HIV infectivity, using methods known to those of skill in the art. A further subject of this invention is a method of producing or identifying a drug which fits (packs into, binds) the N-helix coiled-coil pocket of HIV gp41 through the use of atomic coordinates of a crystal as defined in the claims appended hereto. The method comprises obtaining a crystal of the soluble model, such as the empty soluble model (not in complex with a D-peptide), obtaining the atomic coordinates of the crystal (e.g., of the crystal of the empty soluble model, such as IQN17); using the atomic coordinates obtained to define the N-helix coiled-coil pocket of HIV gp41; identifying a molecule or compound which fits the N-helix coiled-coil pocket and obtaining the molecule or compound; contacting the molecule or compound with the N-helix coiled-coil pocket (e.g., by contacting it with a polypeptide which comprises the pocket (e.g., IQN17 or a variant thereof or the N-peptide) to assess (determine) the ability of the molecule or compound to fit the pocket of HIV gp41, wherein in the molecule or compound fits the pocket, it is a drug which fits the N-helix coiled-coil pocket, whereby a drug which fits the pocket is produced. The atomic coordinates of the crystal can be obtained by X-ray diffraction studies or form a computer file or Protein Data Base (PDB), such as the PDB presented herein for IQN17 (Figures 11A-11V).

Similarly, the method can be carried out using a crystal of a soluble trimeric model in complex with a D-peptide (e.g., a D-peptide described herein, such as D10pep1) or a crystal of the N-peptide region of HIV gp41 which comprises the pocket of the N-helix coiled coil.

Drugs produces in this manner can be further assessed to conform their ability to fit into the pocket (e.g., by NMR) and can be assessed for their ability to inhibit HIV entry into cells (e.g., by a syncytia assay or infectivity assay).

### BRIEF DESCRIPTION OF THE DRAWINGS

Figure 1 is a schematic of HIV-1gp41 showing the N36 (SGIVQQQNNLLRAIEQQHLLQLTVWGIKQLQARIL) (SEQ ID NO:13) and C34 (WMEWDREINNYTSLIHSLIEESQNQQHKNHQELL) (SEQ ID NO: 14) peptides located within two regions containing 4,3 hydrophobic heptad repeats (labeled heptad repeat 1 and heptad repeat 2, also referred to as N-peptide region and C-peptide region, respectively). The underlined residues in C34 were mutated in this study. Three of these residues (W, W and I) project into the N36 cavity, whereas two of these residues (M and R) do not. FP, fusion peptide; S-S, disulfide bond; TM, transmembrane region; INTRA, intraviral region.

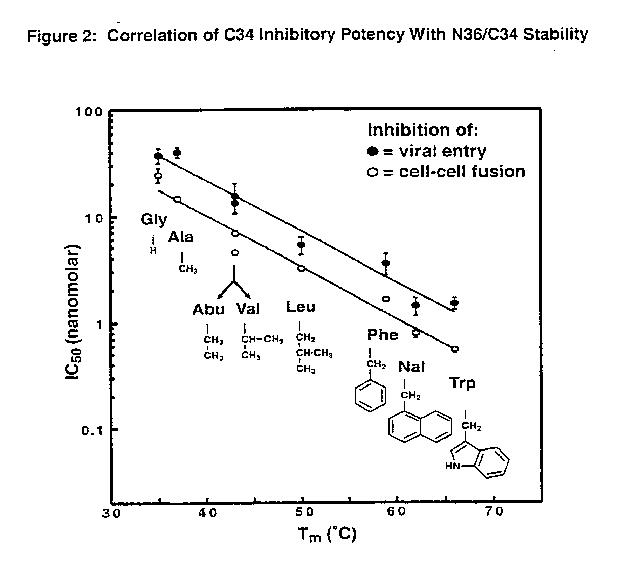

Figure 2 is a graph showing the correlation of C34 inhibitory potency with N36/C34 stability. C34 peptide variants containing substitutions at position Trp⁶³¹ were tested for inhibition of viral entry (filled circles) and cell-cell fusion (open circles). IC₅₀ values are plotted on a logarithmic scale against the _{Tm}, (melting temperature) of the corresponding N36/C34 complex. The identities and chemical structures of the substitutions are drawn under the corresponding data points. In order of increasing hydrophobic bulk, the substitutions were: glycine (Gly), alanine (Ala), L-α-aminobutyric acid (Abu), valine (Val), leucine (Leu), phenylalanine (Phe), the wildtype residue tryptophan (Trp), and L-β-(1-naphthyl) alanine (Nal). Error bars indicate the standard error from triplicate experiments.

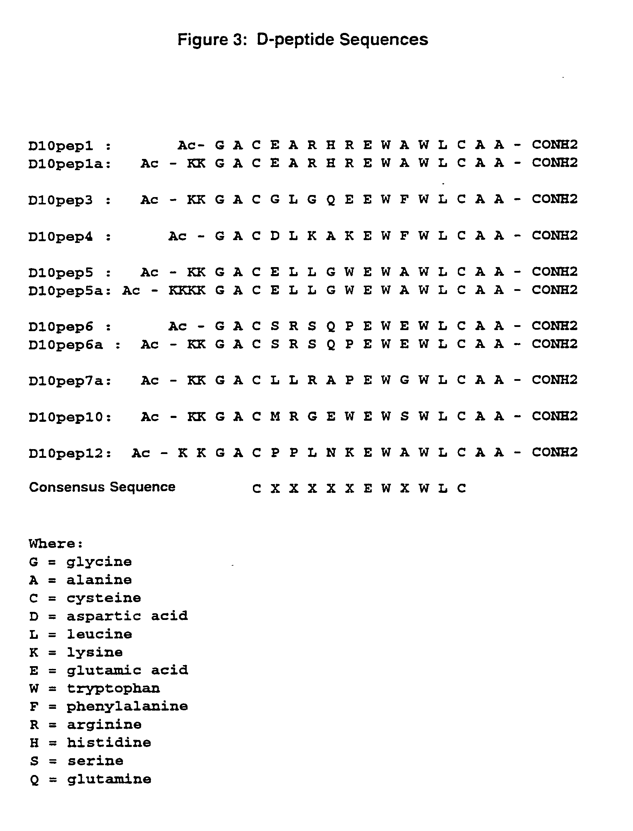

Figure 3 shows the amino acid sequences of D-peptides (SEQ ID NOS: 34, 38, 15, 35, 16, 17, 36, 40, 41, 18 and 19) and the consensus sequence (SEQ ID NO.: 12). As represented, each peptide is flanked by GA on the N-terminus and AA on the C-terminus, and comprises a blocking group at the N-terminus: (Acetyl-GA-C-10mer-C-AA-CONH₂; this can also be represented as ac-GA-C-10mer-C-AA-am). The single letter conventions which are used to represent amino acid residues are as follows: G=glycine; A=alanine; C=cysteine; D=aspartic acid; L=leucine; K=lysine; E=glutamic acid; W=tryptophan; F=phenylalanine; R=arginine; H=histidine; S=serine; and Q=glutamine.

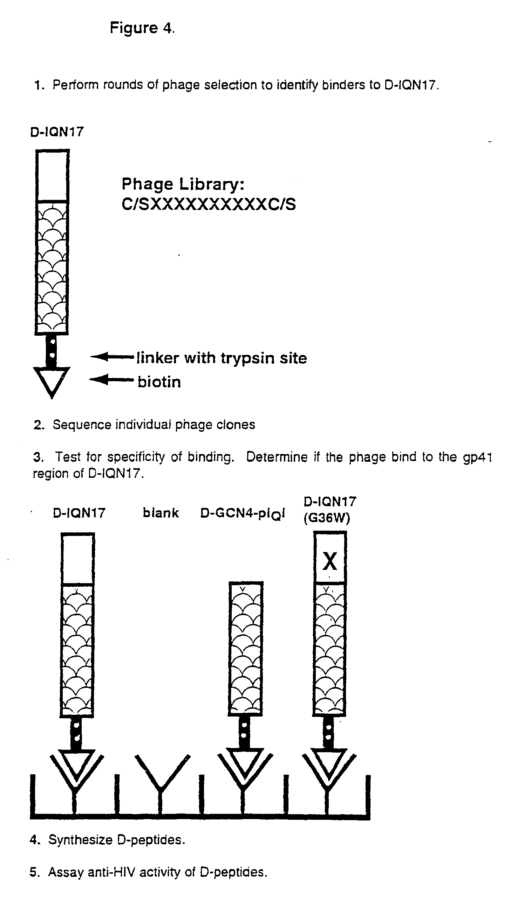

Figure 4 is a schematic representation of mirror-image phage display with the D-IQN17 target, in which: (1) rounds of phage selection are carried out to identify binders to D-IQN17; (2) individual clones are sequenced; (3) binding specificity is assessed by determining whether the phage bind to the gp41 region of D-IQN17; (4) D-peptides of those phage sequences which bind are produced; and (5) the anti-HIV activity of the D-peptides is assayed.

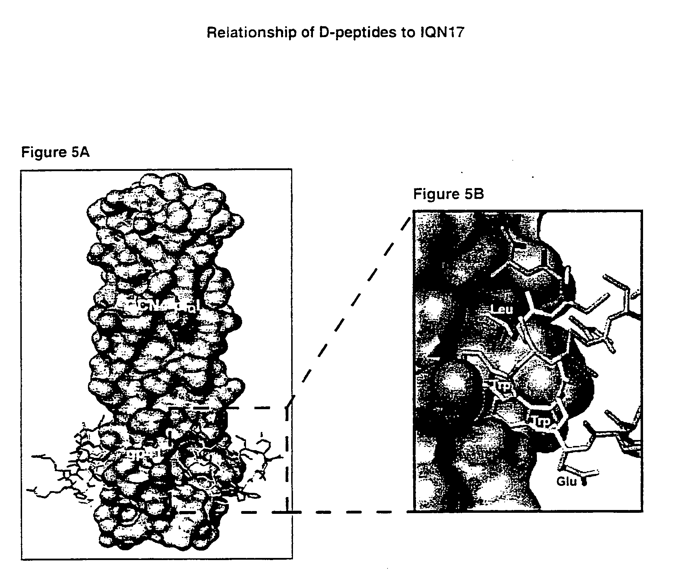

Figures 5A and 5B show the crystal structure of IQN17 bound to D10pep1. IQN17 is shown to be a continuous three-stranded coil, and binding of the conserved amino acid residues of D10pep1 is shown to be to the hydrophobic pocket of IQN17, formed by the 17 residues derived from HIV gp41. Figure 5A shows IQN17, consisting of GCN4-pI_{Q}I residues fused to HIV-1 gp41 residues and the binding of D10pep1 to the hydrophobic pocket of IQN17 (area within box). The D-peptide which binds to the pocket is represented by the branched extensions (i.e., stick representation). Figure 5B is an enlargement of the area within the box and shows the conserved residues that pack into the pocket (Trp, Trp Leu) as well as a glutamic acid (Glu).

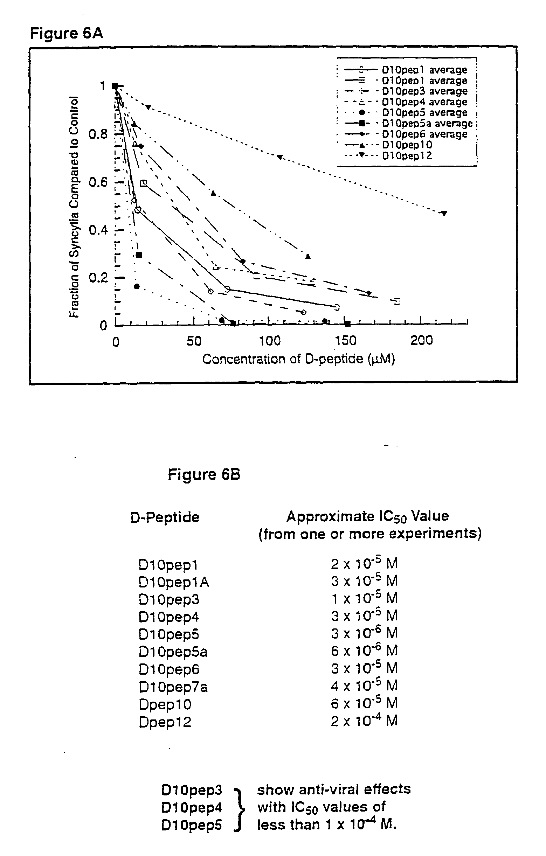

Figures 6A and 6B show results of syncytia assays, using the D-peptides described herein. Figure 6A is a graphic representation of results of syncytia assays. Figure 6B represents IC₅₀ data for D-peptides, with results from one or more experiments.

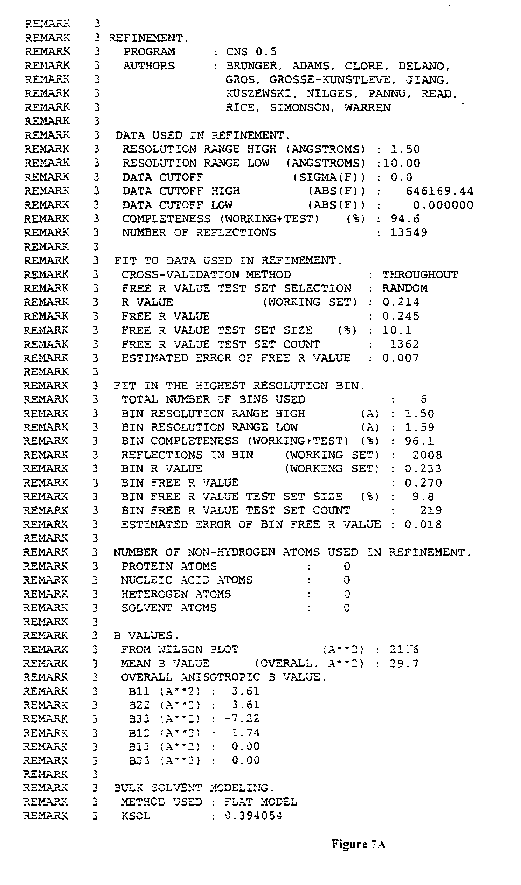

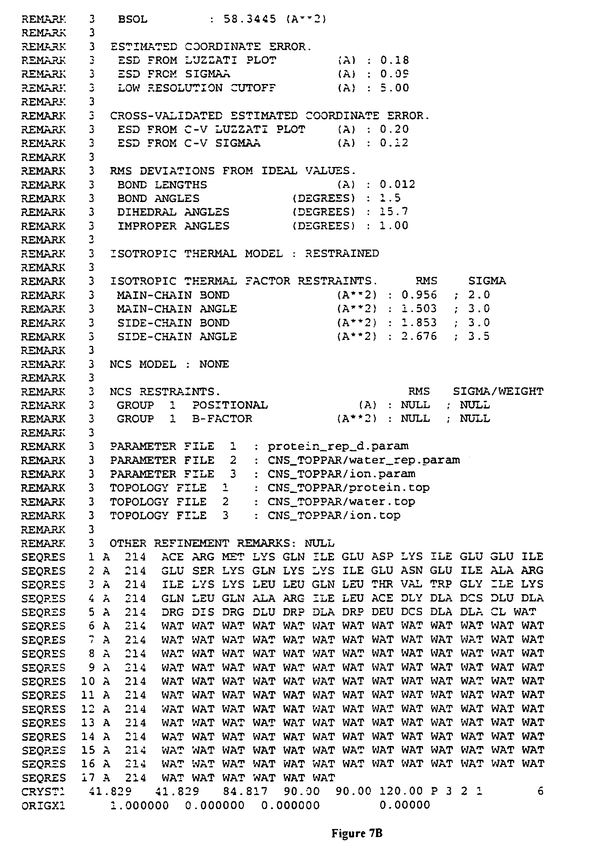

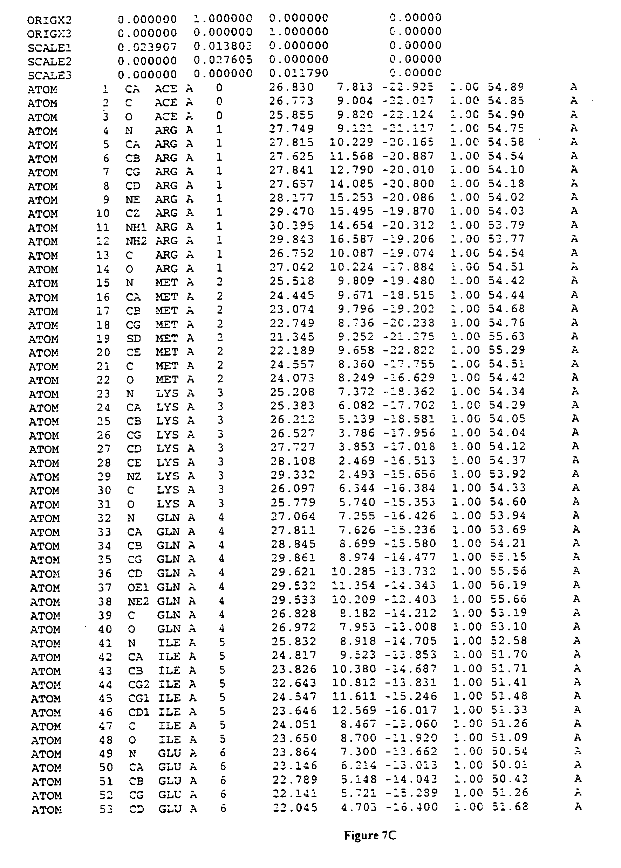

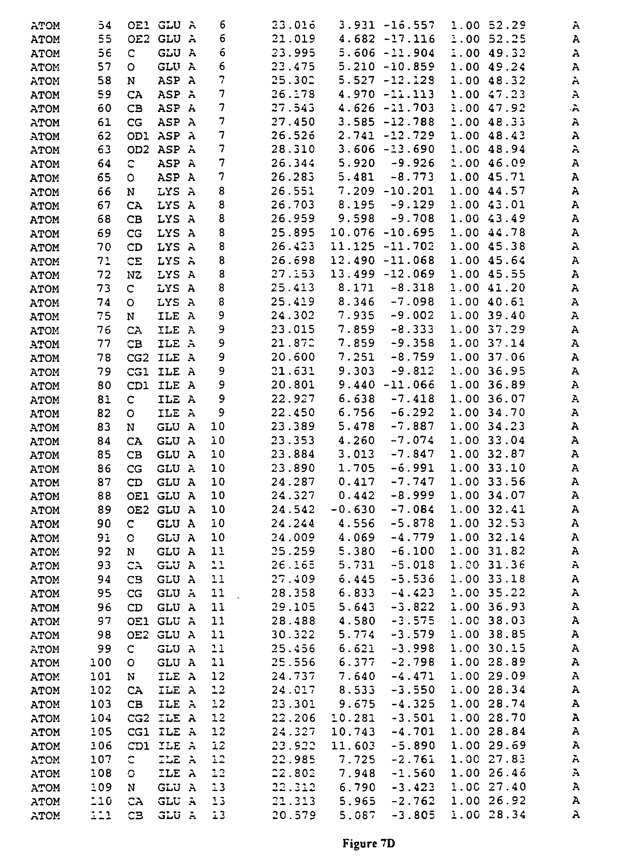

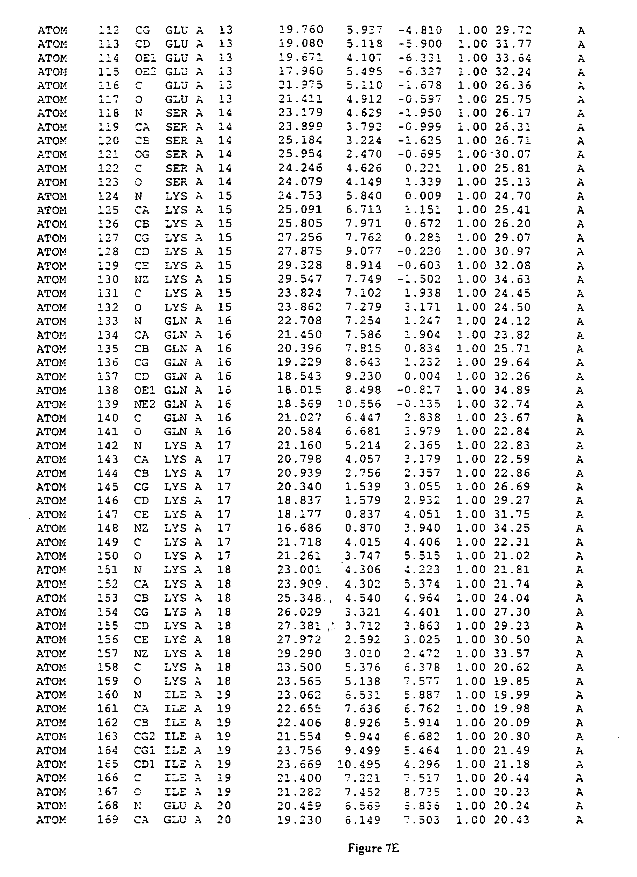

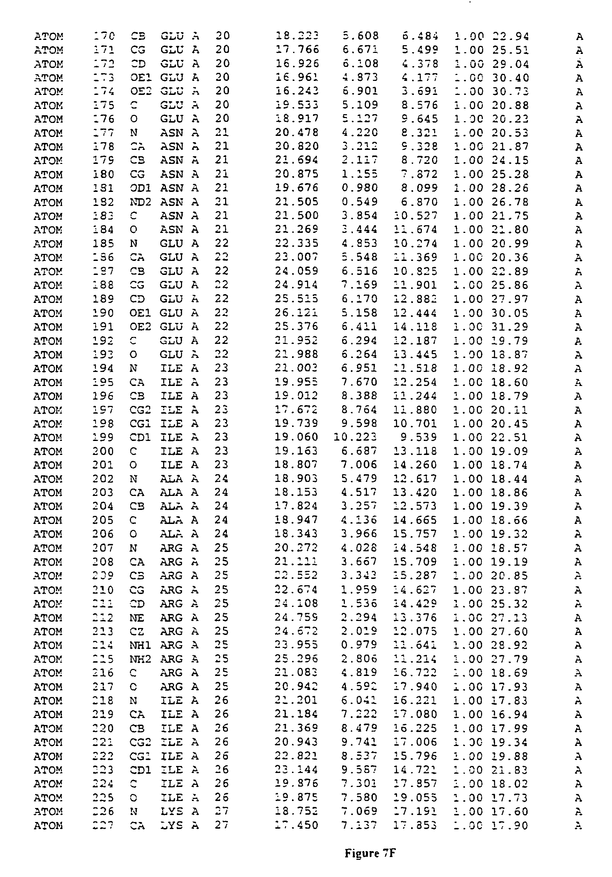

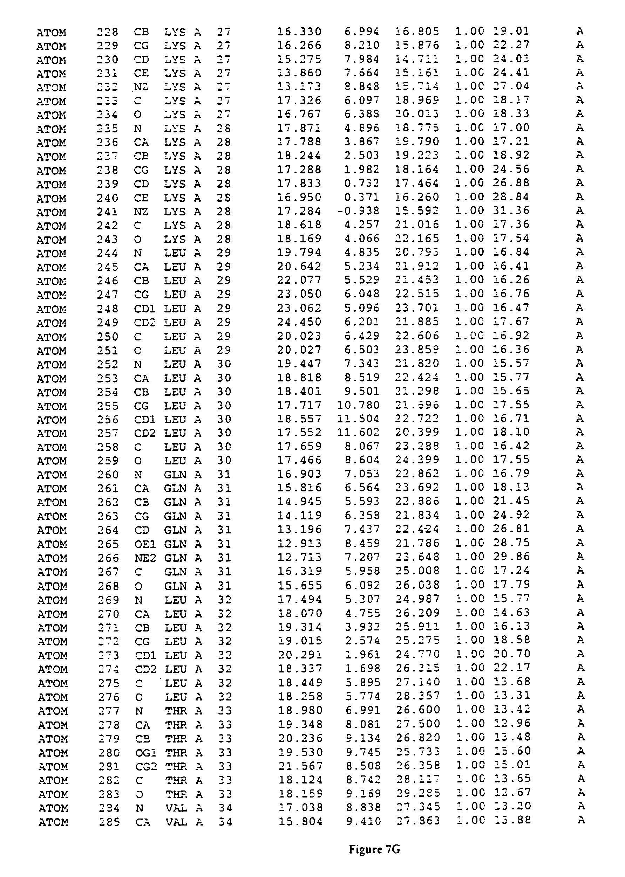

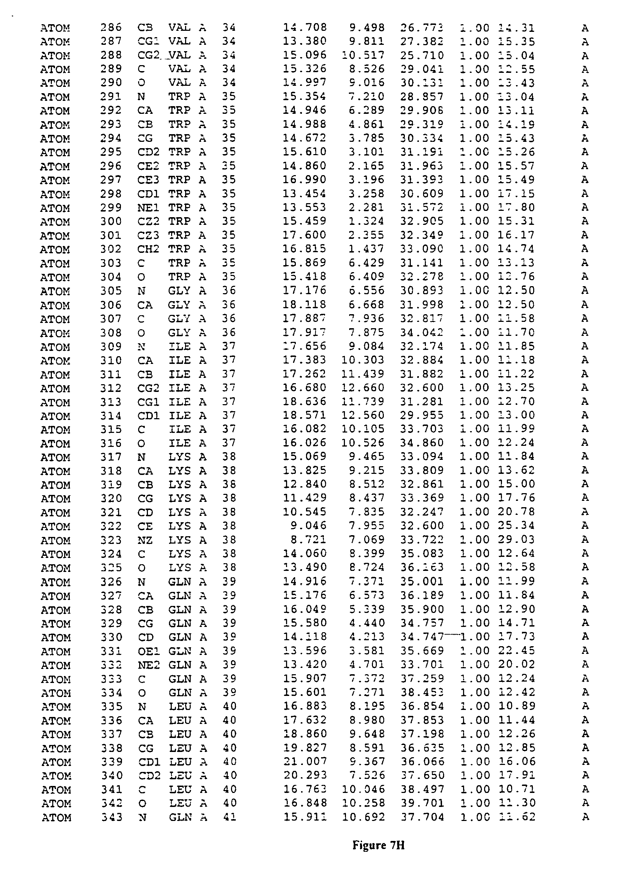

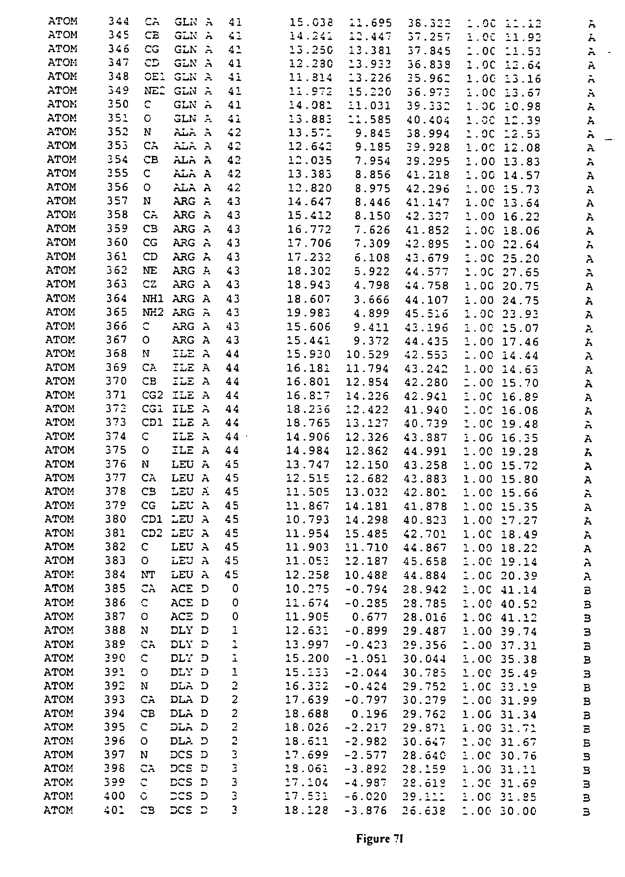

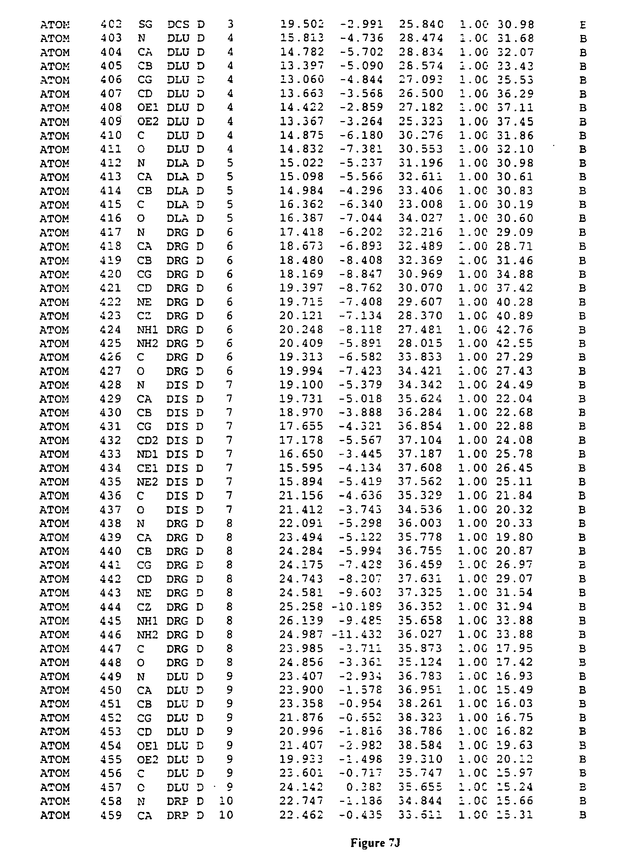

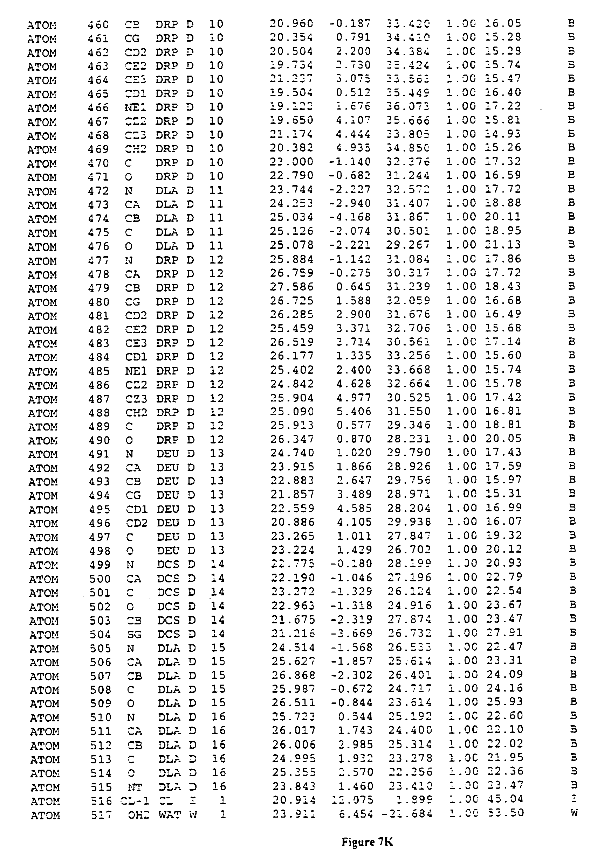

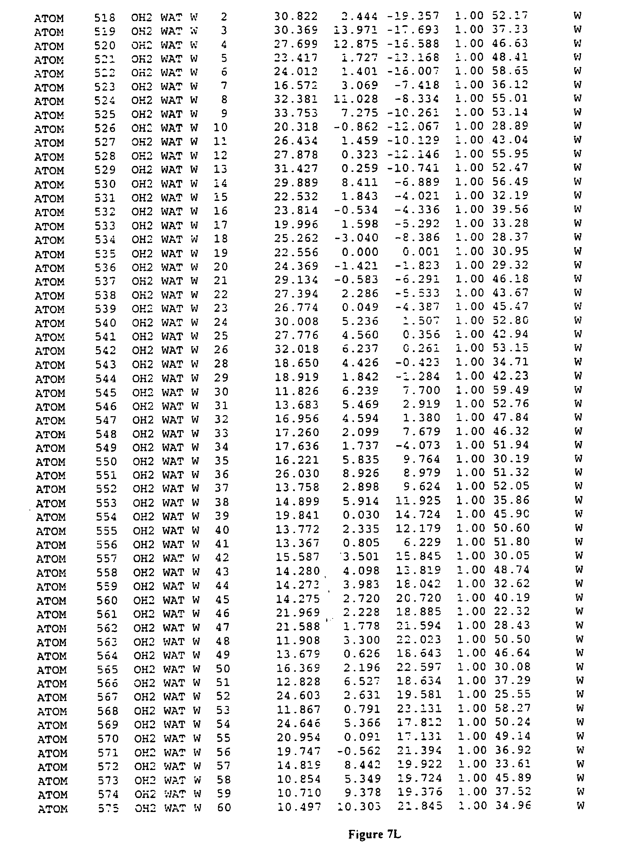

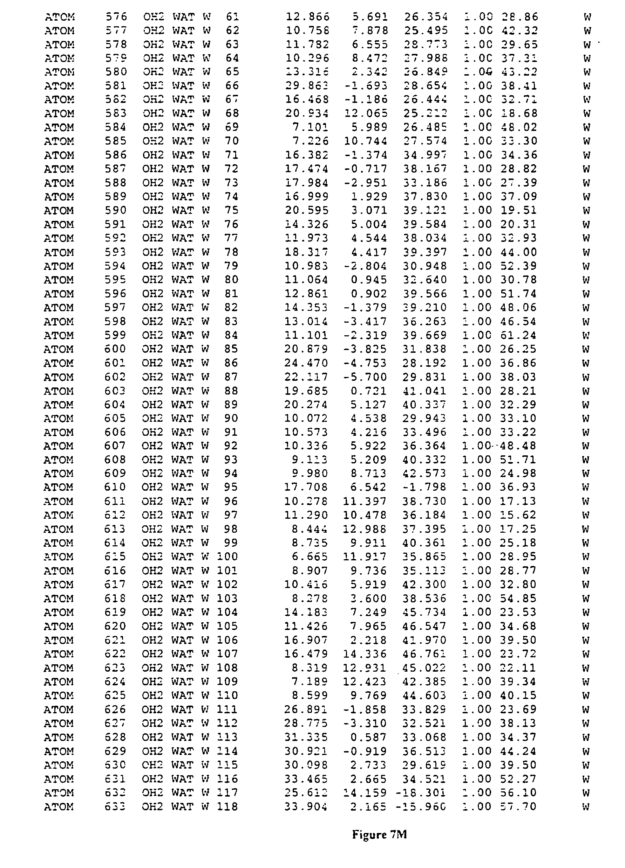

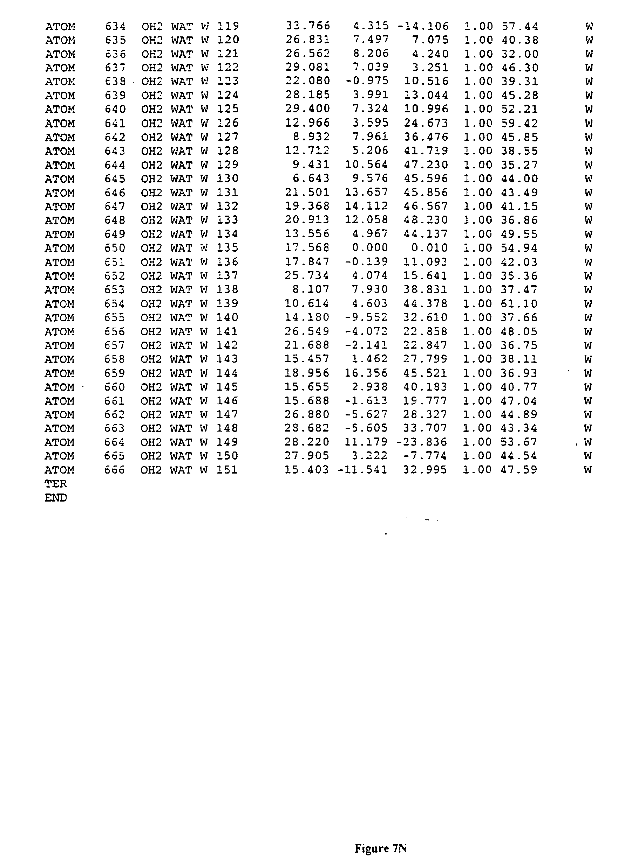

Figures 7A-7N are the PDB file which lists the atomic coordinates for the crystal structure of D10pep1 bound to IQN17, in which residues 0-28 of the A chain are derived from the GCN4-pI_{Q}I sequence (with three mutations), residues 29-45 of the A chain are derived from the HIVgp41 sequence, residues 0-16 of the D chain represent the D-peptide, ordered water molecules are represented as W, and a bound chloride ion as chain I. Residue 0 represents the acetyl group. The PDB file represents a monomer; the trimer is formed by crystallographic symmetry.

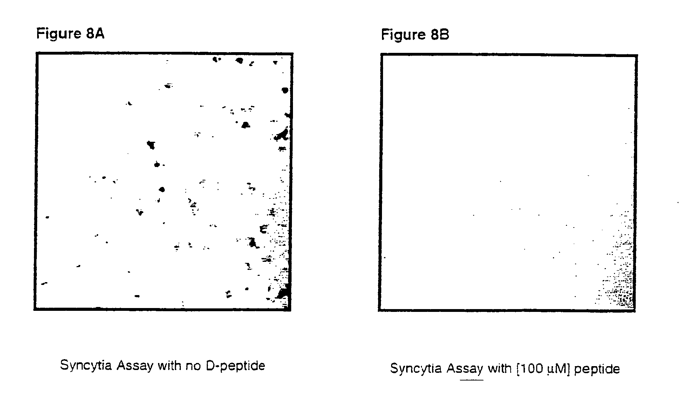

Figures 8A and 8B show results of assessment of inhibition of HIV-1 membrane fusion by a D-peptide. Figure 8A shows results of syncytia assay with no D-peptide. Figure 8B shows results of syncytia assay with D-peptide.

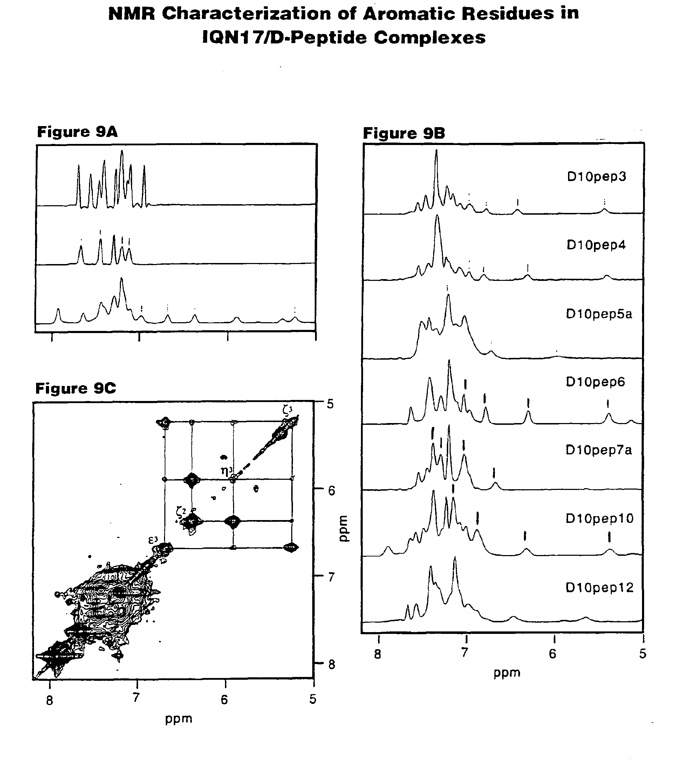

Figures 9A-9C show results of ¹H NMR experiments characterizing the aromatic residues of IQN17/D-peptide complexes. Figure 9A shows 1D-NMR spectra of D10pep1a (top), IQN17 (middle) and a 1:1 complex of D10pep1a and IQN17 (bottom). The x-axis is the same as for (C) below. Upfield peaks assigned to the four scalar-coupled aromatic ring protons of Trp-571 are indicated. The unmarked upfield peak of the bottom trace corresponds to an unassigned Hα resonance. Figure 9B shows 1D spectra of 1:1 complexes between IQN 17 and each D-peptide (as labeled). The same four protons are indicated in some spectra. Figure 9C shows a 2D-NMR TOCSY spectrum of IQN17/D10pep1a complex. Cross-peaks linking these four tryptophan protons are indicated, along with specific assignments. The TOCSY mixing time was 42ms.

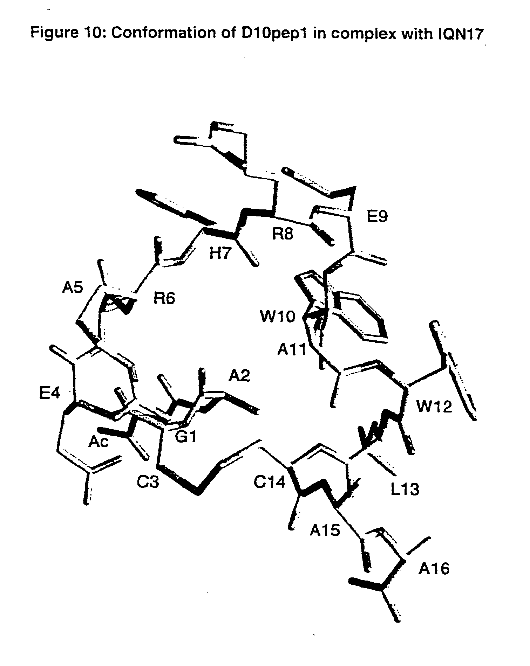

Figure 10 shows the conformation of the D10pep1 peptide as in the complex with IQN17, as determined by X-ray crystallography.
Figures 11A - 11V are the PDB file which lists the atomic coordinates for the crystal structure of IQN17, in which residues 0-28 of the A, B and C chains of the IQN17 trimer are derived from GCN4-pI_{Q}I sequence (with three mutations), residues 29-45 of the chains A, B, and C are derived from HIV gp41, ordered water molecules are represented as W, and a bound chloride ion as chain I. The PDB file represents a whole trimer in the crystallographic asymmetric unit.

### DETAILED DESCRIPTION OF THE INVENTION

The gp41 subunit of the HIV-1 envelope protein mediates fusion of viral and cellular membranes. The crystal structure of the gp41 ectodomain core is a six-helix bundle composed of three helical hairpins, each consisting of an N-helix paired with an antiparallel C-helix (D. C. Chan, D. Fass, J. M. Berger, P. S. Kim, Cell, 89:263 (1997),
W. Weissenhom, A. Dessen, S.C. Harrison, J. J. Skehel, D. C. Wiley, Nature, 387:426 (1997); K. Tan, J. Liu, J. Wang, S. Shen, M. Lu, Proc. Natl. Acad. Sci. USA, 94:12303 (1997). Three N-helices form an interior, trimeric coiled-coil, and three C-helices wrap around the outside of this N-helix coiled-coil along conserved, hydrophobic grooves. This structure likely corresponds to the core of the fusion-active state of gp41 (discussed in D. C. Chan, D. Fass, J. M. Berger, P. S. Kim, Cell, 89:263 (1997), and D.C. Chan and Peter S. Kim, Cell, 93:681 (1998)) and shows similarity to the proposed fusogenic structures of envelope fusion proteins from *influenza (*P. A. Bullough, F. M. Hughson, J. J. Skehel, D. C. Wiley, Nature, 371:37 (1994)), Moloney Murine Leukemia Virus (D. Fass, S. C. Harrison, P. S. Kim, Nat. Struct. Biol., 3:465 (1996)), and simian immunodeficiency virus (SIV). (V.N. Malashkevich, D.C. Chan, C.T. Chutkowski, P.S. Kim, Proc. Natl. Acad. Sci. USA, 95:9134 (1998), M. Caffrey et al., EMBO J., 17:4572 (1998)), and Ebola virus (W. Weissenhorn et al., Mol. Cell 2:605 (1998), V. N. Malashkevich et al., Proc. Natl. Acad. Sci. USA, 96:2662 (1999).)

Synthetic C-peptides (peptides corresponding to the C-helix), such as DP178 and C34, are potent inhibitors of HIV-1 membrane fusion and are effective against both laboratory-adapted strains and primary isolates (V. N. Malashkevich, D. C. Chan, C. T. Chutkowski, P. S. Kim, Proc. Natl. Acad. Sci. USA, 95:9134 (1998), DP178 corresponds to residues 638-673 of HIV-1 gp41 and is acetylated at the amino terminus and amidated at the carboxy terminus (C. T. Wild, D. C. Shugars, T. K. Greenwell, C. B. McDanal, T. J. Matthews, Proc. Natl. Acad. Sci. USA, 91:9770 (1994), S. Jiang, K. Lin, N. Strick, A.R. Neurath, Nature, 365:113 (1993)). A Phase I clinical trial with the C-peptide DP178 (also called T-20) indicates that it has antiviral activity *in vivo,* resulting in reduced viral loads (M. Saag, et al., abstract #771 presented at the Infectious Disease Society of America 35th Annual Meeting, San Francisco, CA, 16 September 1997; Kilby, J.M. et al. Nature Med. 4:1302-1307 (1998)). Based on the structural features of the gp41 core, these peptides are thought to act through a dominant-negative mechanism, in which exogenous C-peptides bind to the central coiled-coil of gp41 and lead to its inactivation (D.C. Chan and P.S. Kim, Cell, 93:681 (1998); R.A. Furuta et al., Nat. Struct. Biol., 5:276 (1998); D. C. Chan, D. Fass, J. M. Berger, P. S. Kim, Cell, 89:263 (1997), W. Weissenhorn, A. Dessen, S.C. Harrison, J. J. Skehel, D. C. Wiley, Nature, 387:426 (1997); K. Tan, J. Liu, J. Wang, S. Shen, M. Lu, Proc. Natl. Acad. Sci. USA, 94:12303 (1997), M. Lu, S. C. Blacklow, P. S. Kim, Nat. Struct. Biol., 2:1075(1995) and C. H. Chen, T. J. Matthews, C. B. McDanal, D. P. Bolognesi, M. L. Greenberg, J. Virol., 69:3771 (1995)). These peptides likely act on a pre-hairpin intermediate of gp41 that forms when the native gp41 structure (i.e., the nonfusogenic conformation present on free virions) is perturbed by gp120/CD4/coreceptor interactions. This pre-hairpin intermediate is proposed to have an exposed N-coiled-coil, thereby allowing C-peptides to bind and inactivate gp41 prior to the formation of the fusion-active hairpin structure (D. C. Chan, P. S. Kim, Cell, 93:681 (1998)). This model is further supported by immunoprecipitation experiments indicating that the C-peptide DP178 binds to gp41 (R. A. Furuta, C. T. Wild, Y. Weng, C. D. Weiss, Nat. Struct. Biol., 5:276 (1998)). In addition, viruses escaping DP178 inhibition show mutations in the central coiled-coil region of gp41 (L. T. Rimsky, D. C. Shugars, T. J. Matthews, J. Virol., 72:986 (1998)).

Recent crystallographic studies of gp41 facilitate the development of small-molecule peptidomimetic drugs which, in contrast to C-peptides, have the potential to be orally administered. Within each coiled-coil interface is a deep cavity, formed by a cluster of residues in the N-helix coiled-coil, that is an attractive target for the development of antiviral compounds. Three residues from the C-helix (Trp⁶²⁸, Trp⁶³¹, and Ile⁶³⁵) insert into this cavity and make extensive hydrophobic contacts. Mutational analysis indicates that two of the N-helix residues (Leu⁵⁶⁸ and Trp⁵⁷¹) comprising this cavity are critical for membrane fusion activity (J. Cao, et al., J. virol., 67:2747 (1993)). Therefore, it is reasonable to expect that compounds that bind with high affinity to this cavity and prevent normal N- and C-helix pairing will be effective HIV-1 inhibitors. In addition, residues in the cavity are highly conserved among diverse HIV-1 isolates. Because of the high structural conservation, drugs targeting this site would have broad activity against diverse HIV-1 isolates, and possibly HIV-2 isolates.

Although this hypothesis is tempting, until now, it had not been demonstrated that these cavity contacts are important for the potency of the C34 inhibitor. In fact, some C-peptides that lack the cavity-binding residues, such as DP178 (C. T. Wild, D. C. Shugars, T. K. Greenwell, C. B. McDanal, T. J. Matthews, ibid, 91:9770 (1994); Kilby, J.M. et al., Nature Med., 4:1302 (1998)), are highly effective inhibitors of HIV-1 membrane fusion. These concerns emphasize the need for systematic structure-function analysis to identify determinants of C-peptide activity.

To determine the role of cavity-contacts in inhibitory activity, structure-based mutagenesis was performed on C34. The core of the gp41 ectodomain (Figure 1) was reconstituted with two synthetic peptides called N36 and C34 (M. Lu, P. S. Kim, J. Biomol. Struct. Dyn., 15:465 (1997), D. C. Chan, D. Fass, J. M. Berger, P. S. Kim, Cell, 89:263 (1997)). Variants of the C34 peptide with single alanine substitutions were synthesized, and the helical content and thermal stability of mutant N36/C34 complexes were quantitated by circular dichroism. As expected, mutation of C34 residues (Met⁶²⁹, Arg⁶³³) that do not contact the N36 coiled-coil had little effect on mean residue ellipticity at 222 nm (a measure of helical content) or stability of N36/C34 complexes (Table 1). However, mutation of the three residues (Trp⁶²⁸ → Ala, Trp⁶³¹ → Ala or IIe⁶³⁵ → Ala) that project into the N36 coiled-coil cavity resulted in N36/C34 complexes with substantially decreased mean ellipticity and stability (Table 1). The greatest destabilization was observed with the mutant Trp⁶³¹ →Ala, which formed N36/C34 complexes with an apparent melting temperature (Tₘ) of 37°C, compared to 66°C for wildtype. These results demonstrate that C34 residues making hydrophobic contacts with the N36 coiled-coil cavity are important for stabilizing the helical-hairpin structure of the gp41 ectodomain core.

To determine the importance of these residues in the ability of C34 to inhibit membrane fusion, the activity of C34 peptides was tested in HIV-1 viral entry and syncytium assays (Table 1). Mutations (Met⁶²⁹ → Ala and Arg⁶³³ →Ala) that had little effect on the stability of the N36/C34 complex also had little effect on the inhibitory activity of wildtype C34 (IC₅₀∼2.1 nM and ∼0.55 nM for viral entry and syncytium formation, respectively). However, mutation of the strictly conserved Trp⁶²⁸ or Trp⁶³¹ to alanine resulted in a substantial decrease in activity of∼5 fold and ∼30-fold, respectively (Table 1). Mutation of the less well-conserved IIe⁶³⁵ resulted in only a ∼2-fold decrease in inhibitory activity. These results demonstrate for the first time, the C34 residues which make contact with gp41 pocket are important for the inhibitory potency of C34.

The relationship between the potency of mutant C34 peptides and the stability of mutant N36/C34 complexes was clarified by taking advantage of the greatly destabilizing effect of the Trp⁶³¹ mutation to construct a series of N36/C34 complexes with a gradation of stabilities. The Trp⁶³¹ position was used as a "guest site" and the tryptophan was substituted with natural and artificial amino acids representing a broad range of hydrophobic bulk. In order of increasing hydrophobic bulk, the substitutions were: glycine (Gly), alanine (Ala), L-α-aminobutyric acid (Abu), valine (Val), leucine (Leu), phenylalanine (Phe), the wildtype residue tryptophan (Trp), and L-β-(1-naphthyl) alanine (Nal). This approach resulted in a set of C34 peptides that form N36/C34 complexes with Tₘs ranging from 37°C to 66°C. The Tₘs and [θ]₂₂₂(10³deg cm²dmol⁻¹) for the N36/C34 variants (with IC₅₀ values (nanomolar) for virus entry and cell fusion, respectively, in parentheses) are: Trp⁶³¹→Gly, 35°C, 17.1 (38 ± 6.1, 25 ± 3.8); Trp⁶³¹→Ala, 37°C, -24.9 (40 ± 4.3,15 ± 0.8); Trp⁶³¹ →Abu, 43°C; -23.2 (16 ± 4.8,6.9 ± 0.4); Trp⁶³¹→ Val, 43°C, -23.9 (13 ± 2.8, 4.5 ± 0.09); Trp⁶³¹→Leu, 50°C, -26.7 (5.3 ± 1.0, 3.2 ± 0.1); Trp⁶¹¹→Phe, 59°C, - 26.3 (3.6 ± 0.8, 1.6 ± 0.05); wildtype, 66°C, -31.7 (1.5 ± 0.2, 0.55 ± 0.03); Trp⁶³¹→Nal, 62°C, -32.0 (1.4 ± 0.3, 0.79 ± 0.08). The concentration of the Trp⁶³¹→Nal peptide was measured by Nal absorbance using the extinction coefficient e = 6900 at 282 nm (J. Blake, C. H. Li, J. Med. Chem., 18:423-426 (1975)). In HIV-1 infection and syncytium assays, this series of peptides showed potencies that closely correlated with the Tₘ of the corresponding N36/C34 complex (Figure 2). The potency order of these mutants is wt∼Nal>Phe>Leu>Val-Abu>Ala-Gly, in close agreement with the hydrophobic bulk of the substitution and the stability of N36/C34 complexes. There is a striking linear relationship when the IC₅₀ is plotted on a logarithimic scale as a function of the Tₘ (Figure 2). Since ΔG= -RTInK (ΔG, change in free energy; R, gas constant; T, absolute temperature; and K, equilibrium constant) and ΔTₘ (T_{m, wildtype complex} -T_{m, mutant complex}) is proportional to Δ(ΔG) (ΔG _{wildtypecomplex}-ΔG _{mutant complex}) (W. J. Becktel, J.A. Schellman, *Biopolymers, 26*:1859 (1987)), the observed linear relationship strongly suggests that the potency of the C34 variants is directly related to their affinity for the N-helix coiled-coil, as predicted by a dominant-negative mode of inhibition. These results provide strong support for the proposal that the coiled-coil cavity in the gp41 core is an attractive drug target. Conserved residues projecting into the hydrophobic cavity clearly play a major role in the ability of C34 to inhibit HIV-1 infection, indicating that this inhibitor works by forming a high-affinity complex with the N-helix coiled-coil. Moving beyond traditional peptides, mirror-image phage display techniques (T. N. Schumacher, et al., Science, 271:1854 (1996)), selection-reflection aptamer techniques (K.P. Williams et al., PNAS, 94:11285 (1997); S. Kluβmann et al., Nat. Biotech., 4:1112 (1996); A. Nolte et al., Nat. Biotech., 14:1116 (1996), combinatorial chemistry (A. Borchardt, S. D. Liberles, S. R. Biggar, G.R. Crabtree, S.L. Schreiber, Chem. Biol., 4:961 (1997); J.C. Chabala, Curr. Opin. Biotechnol., 6:632 (1995)) and computational approaches in structure-based drug design (H. Kubinyi, Curr. Opin. Drug Discov. Develop., 1:16 (1998)), can be used to identify D-peptides, peptidomimetics, and small molecules that bind with high affinity to the coiled-coil cavity. The close correlation between N36/C34 stability and C34 inhibitory potency suggests that the effectiveness of such compounds will depend critically on the strength of their cavity-contacts. These results suggest that candidate compounds can be tested for the ability to form a stable complex with N36, thereby providing a basis for rapid, quantitative screens to identify and evaluate potential inhibitors of HIV-1 entry.

Small-molecule inhibitors directed against the cavity of the central coiled-coil target one of the most highly conserved regions of the HIV-1 envelope proteins. The analogous cavity in the SIV gp41 core has an essentially identical structure, with conservation of side chain conformations (V. N. Malashkevich, D. C. Chan, C. T. Chutkowski, P. S. Kim, Proc. Natl. Acad. Sci. USA, 95:9134 (1998)). This high degree of structural conservation explains the broad neutralizing activity of C-peptides, which are effective against laboratory-adapted strains as well as primary isolates (C. T. Wild, D. C. Shugars, T. K. Greenwell, C. B. McDanal, T. J. Matthews, Proc. Natl. Acad. Sci. USA, 91:9770 (1994), S. Jiang, K. Lin, N. Strick, A.R. Neurath, Nature, 365:113 (1993)). Remarkably, SIV C34 peptide is nearly as effective as HIV-1 C34 in inhibiting HIV-1 infection (V. N. Malashkevich, D. C. Chan, C. T. Chutkowski, P. S. Kim, Proc. Natl. Acad. Sci. USA, 95:9134 (1998)). In addition, a C-peptide (T649) containing the cavity-binding region is much less susceptible to the evolution of resistant virus (L. T. Rimsky, D. C. Shugars, T. J. Matthews, J. Virol., 72:986 (1998)) than DP178 (also called T-20), which lacks this region. These observations are evidence that high-affinity ligands targeting the coiled-coil surface, particularly its cavity, will have broad activity against diverse HIV isolates (including HIV-2) and will be less likely to be bypassed by drug-escape mutants.

These studies on the mechanism of C-peptide action also support the hypothesis that the trimeric hairpin structure of the gp41 core (Chan, D.C. et al., Cell, 89:263 (1997); Weissenhom, W. et al., Nature, 387:426 (1997); Tan, K. et al., Proc. Natl. Acad. Sci. USA. 94:12303 (1997)) corresponds to the fusion-active state of gp41. The work described herein shows that the inhibitory potency of C34 depends on its ability to bind to the N-coiled-coil of gp41. Since the hairpin structure of gp41 is extremely stable (with a melting temperature in excess of 90°C) (Lu, M. et al., Nat. Struct. Biol., 2:1075 (1995)), it is unlikely that nanomolar concentrations of C34 can disrupt this structure once it has formed, especially given the high effective concentration of the N- and C-helices within an intact gp41 molecule. Rather, C-peptides likely act prior to the formation of the gp41 hairpin by binding to a transient pre-hairpin intermediate, in which the central coiled-coil is exposed. Binding of C-peptides to this pre-hairpin intermediate inactivates gp41 and prevents its conversion to the fusion-active hairpin structure (D. C. Chan, P. S. Kim, Cell, 93:681 (1998)).

As described herein, the pocket on the surface of the N-helix coiled-coil of HIV-1 envelope protein gp41 subunit is a drug target. Similarly, cavities on other pathogens (e.g., HIV-2) which can cause AIDS or on pathogens which cause AIDS-like conditions in nonhuman mammals (e.g., SIV) are also drug targets. As described herein, available methods (e.g., mirror image phage display methods, combinational chemistry, computational approaches and other drug screening and medicinal chemistry methods) can be used to identify peptides, D-peptides, peptidomimetics and small molecules that bind the coiled-coil cavity of HIV-1 (and/or HIV-2) with sufficient affinity to interfere with viral entry into cells and, thus, inhibit viral infection. As further described herein (Example 3), mirror image phage display has been used to identify D-peptides which bind to a cavity on the surface of the N-helix coiled-coil of HIV-1 gp41.

As a result of the work described herein, screening assays which identify molecules or compounds (agents or drugs) that prevent C34/N36 complex formation and/or disrupt the complex once it has formed are available, as are methods of identifying molecules or compounds (agents or drugs) which bind the N-helix coiled-coil pocket of HIV gp41. Such drugs or agents are useful to inhibit (totally or partially) HIV entry into cells and, thus, infection by HIV.

Methods of screening for compounds or molecules (referred to as drugs or agents) that interfere with formation of a stable complex between C34 and N36 or disrupt a complex between the two and methods of screening for compounds or molecules that bind the N-helix coiled-coil pocket of HIV gp41 are also described herein.

Drugs which interfere with formation of a complex between C34 peptide and N36 peptide may be identified by combining a candidate drug (a compound or molecule to be assessed for its ability to interfere with formation of a complex between C34 and N36) with C34 and N36, thus forming a test sample, under conditions appropriate for formation of a complex between C34 and N36 and determining whether formation of C34/N36 complex is inhibited (partially or totally) in the test sample. Results of this assessment can be compared with the results of an appropriate control, which is the same combination as the test sample, except that the candidate drug is not present; the control is subjected to the same conditions as is the test sample. If C34/N36 complex is not formed or is formed to a lesser extent in the presence of the candidate drug (in the test sample) than in its absence, the candidate drug is a drug that interferes with formation of a stable complex between C34 and N36. Such a drug is also referred to herein as an inhibitor of C34/N36 complex formation. Inhibition of complex formation can be assessed by determining the extent to which binding of the two members of the complex occurs, such as by means of a fluorescence assay (e.g., FRET), in which C34 and N36 are each labeled by a member of a pair of donor-acceptor molecules or one end of one of the peptides (e.g., the N-terminus of C34) is labeled with one member of such a pair (EDANS) and the natural fluorophore tryptophan, present in the N36 peptide, is the other member of the donor/acceptor pair. Binding of the C34 and N36 is assessed by the extent to which light emission (FRET) occurs from the acceptor model and/or the wavelength spectrum of the light emitted is altered. Prevention of binding by the candidate drug alters the extent to which light is emitted and/or prevents the shift in wavelength that would occur if binding of C34 and N36 occurred. Alternatively, C34 can be labeled with a detectable label, such as a radiolabel (e.g., by synthesizing a variant C34 with a kinase recognition site that can be labeled with a kinase and radioactive ATP). The radiolabeled C34 and the candidate drug are combined with N36 immobilized to, for example, a solid surface (e.g., a bead or a plastic well), thus producing a test sample. The extent to which binding of labeled C34 with immobilized N36 occurs is determined and compared with the extent to which binding of labeled C34 to immobilized N36 occurs under the same conditions to which the test sample is subjected, but in the absence of the candidate drug (in a control sample). Typically, this assessment is carried out after the sample has been maintained for sufficient time and under appropriate conditions for C34/N36 binding to occur and a subsequent wash to remove any unbound C34 and candidate drug. If binding occurs in the test sample to a lesser extent than in the control sample, as evidenced by less radiolabel bound to the immobilized N36 in the test sample than in the control sample, the candidate drug is an inhibitor of binding of C34 and N36. Alternatively, the label or tag on C34 can be a member of a binding pair, the other member of which is used to detect binding to N36. For example, C34 can be tagged with biotin (through standard solid-state peptide synthesis, for example) and combined with N36, which can be in solution or bound to a solid surface, such as a bead, well or flat/planar surface, along with the candidate drug (test sample) or in the absence or the candidate drug (control sample). Binding of C34 to N36 is assessed by detecting the presence of biotin associated with N36, such as through the use of labeled streptavidin (e.g., streptavidin - HRP, streptavidin - AP or iodinated streptavidin), which binds the biotin on C34 and is then itself detected through its label. If binding occurs less in the presence of the candidate drug (in the test sample) than in the absence of the candidate drug (in the control sample), as indicated by the presence of less biotin detected on N36 in the test sample than in the control sample, the candidate drug is an inhibitor of C34/N36 binding. The candidate drugs can be obtained, for example, from a library of synthetic organic compounds or random peptide sequences, which can be generated synthetically or through recombinant technology.

In a similar fashion, the ability of a candidate drug to disrupt C34/N36 binding can be assessed, to identify inhibitors of C34/N36 and, thus, of HIV infection. Here, preformed C34/N36 complex is combined with a candidate drug, which is to be assessed for its ability to disrupt the complex, thus producing a test sample. The control sample is the same as the test sample, except that the control sample does not contain the candidate drug; it is treated in the same manner as the test sample. If C34/N36 binding is disrupted in the presence of the candidate drug and not in the control sample or if disruption of the complex occurs to a greater extent in the test sample than in the control sample, the candidate drug is an inhibitor (disrupter) of C34/N36. Detection of disruption of binding can be carried out as described above for detection of/prevention of/interference with binding of C34/N36 (e.g., by FRET or a fluorescence assay, by detecting a radiolabel or other detectable label, such as biotin.)

Results described herein demonstrate that hybrids (i.e., fusion proteins) can be made between a trimeric version of the coiled-coil region of a protein (such as GCN4) and the N-helix coiled-coil of HIV gp41, and that such hybrids are trimeric (i.e., not aggregated) and 100% helical. Results described herein also clearly indicate that such fusion proteins do not disrupt or alter the structure of the N-peptide large cavity (i.e., hydrophobic pocket), which is essentially the same in IQN17 (ligand-free and in complex with D10pep1; see Example 5) as it is in N36 (i.e., in complex with C34; Chan D.C. et al. Cell, 89, 263 (1997)).

Figures 5A, 5B and 6 present results of assessment of peptides described herein. In Figure 5A-5B, the IQN17 crystal structure is shown to be a continuous, three-stranded coiled-coil; the 17 residues derived from HIV gp41 form a hydrophobic pocket very similar to that found in the crystal structure of gp41. As shown, D10pep1 is bound to this pocket and the residues of D10pep1 that correspond to the conserved residues (leucine, tryptophan, tryptophan) found in all of the D-peptide inhibitors described herein are packed into this pocket, clearly indicating that other D-peptide inhibitors which comprise these conserved residues would bind to IQN17 in the same manner. Figure 6 shows results of syncytia assays carried out according to the method described by Chan *et al.* (Chan, D. C. et al. Proc. Natl. Acad. Sci., 95: 15613-15617 (1998)). In the experiments whose results are represented in Figure 6, D-peptides identified as described herein were used. In each instance, a blocking group (e.g., an acetyl group) was present at the N terminus and a CONH₂ (amide) was present at the C-terminus. Results of these assays showed a range of IC₅₀ concentrations, where IC₅₀ is the concentration at which one half of the number of syncytia are observed, compared to the control, in which no peptide is included. For example, D10pep5 with two lysines at the N-terminus has an IC₅₀ of approximately 6.

Also described herein is a method of identifying a drug that binds the N-helix coiled-coil cavity of HIV gp41. Here, too, the assay is based on assessing loss or decrease in binding, but unlike the C34/N36 complex assay described above, which is a more general assay in that it covers or detects interaction with any portion of the groove formed by the N-helical region of HIV gp41, this embodiment focuses on the HIV gp41 hydrophobic pocket (the N-helix Here coiled-coil cavity). Here, the method comprises combining a candidate drug to be assessed for its ability to bind the N-helix coiled-coil cavity of HIV gp41 with a fusion protein that comprises a trimeric version of the coiled-coil region of a protein and a sufficient portion of the N-peptide of HIV gp41 to include the HIV gp41 cavity, under conditions appropriate for presentation of the HIV gp41 cavity for binding by a peptide or other molecule and determining (e.g., in a high throughput screen) whether the candidate drug binds the fusion protein, If binding occurs, the candidate drug is a "hit" that may be a drug that binds the N-helix coiled-coil cavity of HIV gp41. If binding occurs, the candidate drug has bound the N-helix coiled coil and it can be determined if it binds to the coiled-coil cavity. Such "hits" can then be screened in secondary assays, such as the cell/cell fusion assay and HIV infectivity assay to determine if the candidate drug is a drug. Alternatively, or in addition, such "hits" can be assessed further by use of a counterscreen with other fusion proteins (or peptides), to which pocket-binding molecules will not bind. For example, GCN4-pl_{Q}I (with the same three surface mutations as in IQN17) or a version of IQN17 with a point mutation in the hydrophobic pocket, IQN17 (G39W), in which glycine 39 is mutated two tryptophan, resulting in a large protrusion into the pocket, can be used in a counterscreen. In this example, a candidate drug that binds to IQN17 but not to GCN4-pI_{QI} (with the same three surface mutations as in IQN17) or IQN17(G39W) is a drug that binds the N-helix coiled-coil cavity of HIV gp41.

A competitive assay may be carried out. Here, a peptide or protein that binds the N-helix coiled-coil cavity of HIV gp41 is combined with the candidate drug and the fusion protein and whether the candidate drug binds the HIV gp41 cavity is determined in the presence of the peptide that binds the N-helix coiled cavity of HIV gp41. If the candidate drug binds the fusion protein, it is a drug that binds the HIV gp41 cavity. For example, a fusion protein which comprises a trimeric version of the coiled-coil region of GCN4 and the C-terminus of the N peptide of HIV gp41 that includes the N-helix coiled-coil cavity (IQN17) is combined with a "reference" D-peptide (e.g., any of the D-peptides described herein or variants thereof) that binds the N-helix coiled-coil cavity and a candidate drug to be assessed for its ability to bind the N-helix coiled-coil cavity of HIV gp41, thus producing a test sample, which is maintained under conditions appropriate for binding of the D-peptide to bind to the cavity. A control sample, which includes the same components as the test sample, except for the candidate drug, and is handled in the same manner as the test sample, is also assessed. In both samples, binding of the reference D-peptide is assessed. If binding of the reference D-peptide occurs to a lesser extent in the presence of the candidate drug (in the test sample) than in its absence (in the control sample), the candidate drug is a drug that binds the N-helix coiled-coil cavity of HIV gp41. Detection of binding is assessed, for example, in a similar manner was described above for the C34/N36 embodiment of the invention. For example, the D-peptide is labeled with a detectable label, such as a radiolabel or a first member of a binding pair (e.g., biotin), and the extent to which the N-helix coiled-coil cavity bears the label (after the samples have been maintained under conditions appropriate for binding of the reference D-peptide to the cavity) is determined. In the case in which radiolabeling is used, the extent to which the fusion protein hears the radiolabel is assessed in the test sample and compared with the extent to which the fusion protein bears the radiolabel in the control sample. If the detectable label is a first member of a binding pair (e.g. biotin), the second member of the pair (a binding partner) is added to the samples in order to detect the extent to which the fusion protein is bound by the reference D-peptide. This can he done directly or indirectly (e.g., by adding a molecule, such as an antibody or other moiety which binds the second member of the binding pair). Less of the label will be present on the fusion protein (N-helix coiled-coil cavity) if the candidate drug has inhibited (totally or partially) binding of the D-peptide to the cavity. If binding occurs to a lesser extent in the test sample (in the presence of the candidate drug) than in the control sample (in the absence of the candidate drug), then the candidate drug is a drug that binds the N-helix coiled-coil cavity of HIV gp41.

IQN17, or a variant thereof, in the D-enantiomer, is useful to identify molecules or compounds which are members of a library or collection and bind the N-helix coiled-coil of gp41. For example, a library or collection of molecules or compounds, such as a phage display library, can be screened with IQN17 in the D-enantiomer to identify members that bind the pocket. This has been carried out successfully, as described herein. The mirror image of IQN17, or a variant thereof, is used as the target molecule. As used herein, the terms "D-enantiomer of a polypeptide" and "D-peptide" refer to the exact mirror image of the molecule in the natural handedness. Thus, for amino acid residues that contain a second chiral center, such as Ile and Thr, the exact mirror image of the naturally-occurring amino acid residue is used to create the D version of the polypeptide. Also as used herein, the terms "D-amino acids" and "L-amino acids" are both meant to include the non-chiral amino acid glycine. D-IQN17 can be immobilized to a solid surface, such as by addition of one member of a binding pair (e.g., biotin) to it and addition of the other member of the pair (e.g., streptavidin) to the solid surface. Binding of the two members results in immobilization of D-IQN17 on the solid surface, such as for phage panning. A linker which is an enzyme recognition site (e.g., an amino acid linker such as Gly-Lys-Gly, in which an L-lysine residue is used) can be placed between the D-IQN17 sequence and the binding pair member (between the biotin and D-IQN17) to provide an enzyme recognition site (here, a trypsin recognition site), so that bound phage can be eluted by a trypsin digestion, rather than by non-specific elution, such as acid addition. The phage display library can be a library of L-amino acid peptides of any appropriate length fused to an appropriate phage gene. In one embodiment, it is a phage display library of L-amino acid peptides fused to the gIII gene of M13 phage. The peptides, in one embodiment, comprise 10 randomly encoded amino acid residues flanked by either a cysteine or a serine on both sides. Typically, several rounds of panning are carried out. D-IQN17-specific binding phage are identified. Phage that bind only the gp41 region of D-IQN17 can be identified by post-panning assessment, such as by screening against wells that lack the antigen and then further testing against a panel of molecules. For example, specific pocket-binding phage include those that bind D-IQN17 but not D-GCN4-pI_{Q}I (with the same three surface mutations as in IQN17) or a version of D-IQN17 with a point mutation in the hydrophobic pocket, D-IQN17(G39W), in which glycine 39 is mutated to tryptophan, resulting in a large protrusion into the pocket. D-peptides identified in this manner can be assessed for their ability to inhibit HIV gp41, using known assays, such as the cell/cell fusion assay and HIV infectivity assay. The mirror-image phage display method described herein has demonstrated the value of IQN17 and IQN17(G39W), and their D-enantiomers in identifying inhibitors of HIV-1 entry that bind the gp41 pocket. Of nine specific pocket-binding phage sequences identified (phage that bind to D-IQN17 but not to D-IQN17(G39W), eight contain a consensus EWXWL sequence and inhibit HIV-1 gp41-induced syncytia formation when tested as D-peptides. The ninth peptide was toxic to cells and was not investigated further.

The D-versions of IQN17 and IQN17(G39W) can be used in a similar manner with other biologically encoded libraries, to discover other pocket-binding molecules that are not subject to enzymatic degradation by natural enzymes. For example, other phage-display libraries can be used to identify new D-peptide inhibitors (e.g., with a different number of residues between the flanking Cys residues and/or with randomly encoded amino acid residues outside the regions flanked by cysteine residues and/or with more than two cysteine residues). Strategies for encoding peptide libraries without phage (e.g., in which the encoding mRNA is attached to the peptide) can be used to identify D-peptide inhibitors. RNA or DNA libraries can be used (e.g., with SELEX methods) to identify L-ribose- or L-deoxyribose-based RNA or DNA aptamers, respectively, that bind to the hydrophobic pocket and are not substrates for natural nucleases (see e.g., Williams et al., PNAS, 74:11285 (1997)).

Although the versions of IQN17 and IQN17(G39W) of natural L-handedness can also be used in similar manner with biologically encoded libraries, the most likely applications will be with other, non-biologically encoded libraries. For example, chemical combinatorial libraries on beads (of the one-bead, one-compound variety) can be screened with labeled IQN17 (e.g., radioactive or with a chromophore) to identify beads containing molecules that bind to IQN17. In this example, IQN17(G39W) can be used as a counterscreen to determine if the molecules on the bead bind to the pocket of IQN17. (If they bind to IQN17(G39W), then they are not likely to be pocket-binding molecules). As another example, beads to which IQN17 had been previously attached can be incubated with a mixture of potential pocket-binding molecules (e.g., a mixture of chemicals, or a natural product extract). IQN17 (bound to the beads) can then be separated from the mixture, washed, and then subjected to conditions (e.g., organic solvent, low pH, high temperature) that elute molecules bound to the IQN17 on the beads. The eluted molecules (i.e., potential pocket-binding molecules) could be identified by analytical chemistry methods (e.g., HPLC, mass spectrometry). A counterscreen with IQN17(G39W) is useful to help to identify true pocket-binding molecules.

Drugs identified by the methods described above arc then further tested for their ability to inhibit (totally or partially) HIV gp41 function (membrane fusion) and, thus entry into cells, using further *in vitro* assays, such as the syncytium assays and/or infectivity assays described herein or others known to those of skill in the art, and/or *in vivo* assays in appropriate animal models or in humans.

Also described herein is a method of identifying a drug that binds the N-helix coiled-coil of HIV gp41, particularly the N-helix coiled-coil pocket. The method comprises combining a candidate drug to be assessed for its ability to bind the N-helix coiled-coil pocket of HIV gp41 and peptide which comprises a soluble, trimeric coiled-coil and a sufficient portion of the N-peptide of HIV gp41 to include the HIV gp41 pocket, under conditions appropriate for presentation of the HIV gp41 pocket for binding by a molecule or compound (e.g., a drug) and determining whether the candidate drug binds the HIV gp41 pocket. If binding of the candidate drug with the HIV gp41 pocket occurs, the candidate drug is a drug which binds the N-helix coiled-coil pocket of HIV gp41. Optionally, binding of the candidate drug can be assessed in the assay as described above, except that a peptide that binds the N-helix coiled-coil pocket (a peptide previously identified as one which binds the pocket) is combined with the candidate drug and the peptide. In this competitive assay, binding of the candidate drug to the N-helix coiled-coil pocket is assessed in the presence of a known binding moiety (a molecule or compound which binds the pocket). If binding of the candidate drug occurs in the presence of the known binding moiety, the candidate drug is a drug which binds the N-helix coiled-coil pocket with sufficient affinity to successfully compete with the known binding moiety. The fusion protein used comprises a soluble, trimeric version of a coiled-coil, such as a soluble, trimeric version of the coiled-coil region of a protein (e.g., a non-HIV protein, such as that of GCN4 or GCN4-pIQI, although an HIV protein can be used) and a sufficient portion of the N-peptide of HIV gp41 to include the HIV gp41 cavity. For example, this portion can comprise SEQ ID NO.: 20 or a sufficient portion to comprise the cavity and, when present in an appropriate fusion protein or other soluble model, present the cavity in such a manner that it is available for binding. Alternatively, a variant of the HIV gp41 sequence present herein, a sequence from another strain of the human virus (e.g., HIV-2) or a sequence from another species (e.g., SIV, feline immunodeficiency virus, Visna virus (M. Singh et al., J. Mol. Biol., 290:1031 (1999)) can be used in the fusion protein or soluble model. The fusion protein can comprise a soluble, trimeric version of the coiled-coil of any protein, provided that when it is in the fusion protein with the HIV component, the HIV cavity is presented in such a manner that it is available for binding. It can be, for example, that of GCN4-pI_{Q}I, GCN4-pII, Moloney Murine Leukemia Virus (Mo-MLV) or the ARC heterotrimer. The fusion protein may be IQN17 in the D- form or in the natural L-handedness.

In the competitive assay format, any peptide known to bind the N-helix coiled-coil cavity can be used as the known binding moiety. For example, any of the peptides described herein (SEQ ID NOS.: 3-12, 15, 17-19, 23, 24) or a variant or portion thereof can be used. Also, any non-peptide pocket-binding molecule can be used in the competitive assay format. The competitive assay can be performed in solution, on a bead, or on a solid surface.

The candidate drug may be detectably labeled and binding of the candidate drug to the HIV gp41 N-helix coiled-coil determined by detecting the presence of the detectable label on the HIV gp41 N-helix coiled-coil (as a result of binding of the labeled candidate drug to the N-helix coiled-coil). Detection of the label on the helix coiled-coil pocket of the soluble model is indicative of binding of the candidate drug to the N-helix coiled-coil pocket and demonstrates that the candidate drug is a drug which binds the N-helix coiled-coil pocket. If the labeled candidate drug is detected on the fusion protein, the candidate drug is a drug which binds the N-helix coiled-coil cavity.

In another method of identifying a drug that binds the N-helix coiled-coil pocket of the HIV gp41, a soluble model that presents the pocket in such a manner that it is available for binding by a drug is combined with a candidate drug and whether binding of the candidate drug with the N-helix coiled-coil of the soluble, model occurs is determined. If binding occurs, the candidate drug is a drug which binds the pocket. Here, too, a competitive assay format can be used. The components of the competition assay (e.g., IQN17 and a D-peptide) can be labeled, with any of a variety of detectable labels, (including fluorophore/quencher combinations. The candidate drug can be labeled, as described above, with any of a variety of detectable labels. The components of the soluble model (fusion protein) used and the competing moiety which is used in a competitive assay format can also be as described above.

The invention finds application in a method of producing a drug that binds the N-helix coiled-coil pocket of HIV gap41. The method is carried out as follows: A soluble model that presents the N-helix coiled-coil pocket of HIV gp41 or a fusion protein which comprises a soluble, trimeric coiled-coil (e.g., of a protein, such as a non-HIV protein, such as GCN4-pI_{Q}I, GCN4-pII, Mo-MLV, ABC heterotrimer or an HIV protein) is combined with a candidate drug to be assessed for its ability to bind the N-helix coiled-coil pocket of HIV gp41 and inhibit entry into cells, under conditions appropriate for presentation of the HIV gp41 pocket for binding by a drug. Whether the candidate drug binds the HIV gp41 pocket is determined, wherein if binding of the candidate drug to the N-helix coiled-coil pocket of HIV gp41 occurs, the candidate drug is a drug which binds the N-helix coiled-coil cavity of HIV gp41. In this embodiment, the fusion protein comprises a soluble, trimeric coiled-coil (e.g., of a protein such as a non-HIV protein, such as a soluble, trimeric coiled coil of GCN4, GCN4-pIQI, GCN4-pII, Mo-MLV, ABC heterotrimer or an HIV protein) and a sufficient portion of the N-peptide of HIV gp41 to include the HIV gp41 N-helix coiled-coil pocket (e.g., all or a portion of SEQ ID NO.: 20, a variant or modification thereof or a sequence from another strain or species). IQN17, described herein, can be used in this method; the D enantiomer of IQN17 can also be used (e.g., in mirror-image phage applications). The ability of the drug produced to inhibit HIV entry into cells is assessed, for example, in a syncytium assay and/or an infectivity assay, as described herein. It can be further assessed in an appropriate animal model or in humans.

The invention also finds application in a method of producing a drug that binds the N-helix coiled-coil pocket of HIV gp41. The method comprises: producing or obtaining a soluble model of the N-helix coiled-coil pocket of HIV gp41 (e.g., a fusion protein as described herein and particularly IQN17 or a variant thereof); combining a candidate drug (a molecule or compound) to be assessed for it ability to bind the N-helix coiled-coil pocket of HIV gp41 and the soluble model of the N-helix coiled-coil pocket of HIV gp41 and determining whether the candidate drug hinds the N-helix coiled-coil pocket of HIV gp41. If the candidate drug binds the N-helix coiled-coil pocket of HIV gp41, the candidate drug is a drug which binds the N-helix coiled-coil pocket of HIV gp41; as a result, a drug which binds the N-helix coiled-coil cavity of HIV gp41 is produced. The fusion protein used in this embodiment is described herein and can be, for example, IQN17, the D enantiomer of IQN17, or variants thereof. Alternatively, a drug that binds the N-helix coiled-coil pocket of HIV gp41 and inhibits entry of HIV into cells can be produced by a method comprising: producing or obtaining a soluble model of the N-helix coiled coil pocket of HIV gp41, as described herein; combining the soluble model and a candidate drug to he assessed for its ability to bind the N-helix coiled-coil pocket of HIV gp41; determining whether the candidate drug binds the N-helix coiled-coil pocket of the soluble model (fusion protein), wherein if binding occurs, the candidate drug is a drug which binds the N-helix coiled-coil of HIV gp41; and assessing the ability of the drug which binds the N-helix coiled-coil to inhibit HIV entry into cells, wherein if the drug inhibits HIV entry into cells, it is a drug which binds the N-helix coiled-coil pocket of HIV gp41 and inhibits HIV entry into cells. Its ability to inhibit HIV entry into cells can be assessed *in vitro* (e.g., in a syncytium assay, an infectivity assay) or *in vivo* (e.g. in an appropriate animal model or in humans). The soluble model can be a peptide which comprises a soluble, trimeric coiled-coil, such as that of a protein (e.g., GCN4-pI_{Q}I) and a sufficient portion of the N-peptide of HIV gp41 to include the HIV gp41 pocket.

Drugs which inhibit HIV gp41 can also be designed or improved with reference to the X-ray crystal structure of the complex between IQN17 and a D-peptide which binds the N-helix coiled-coil cavity presented by IQN17, such as with reference to the X-ray structure of the complex between IQN17 and D10pepl, presented herein. Alternatively, or in addition, drugs which inhibit HIV gp41 can also be designed or improved with reference to the X-ray crystal structure of free IQN17, presented herein.

Compounds and molecules (drugs) identified as described herein inhibit (partially or totally) entry of HIV into cells, and thus are useful therapeutically in uninfected individuals (humans) and infected individuals (e.g., to prevent or reduce infection in an uninfected individual, to reduce or prevent further infection in an infected individual) and as research reagents both to study the mechanism of gp41-induced membrane fusion and to assess the rate of viral clearance by an individual and as reagents to discover or develop other compounds and molecules (drugs) that inhibit entry of HIV into cells. D-peptides described herein (e.g., D10pep5, D10pep1) have been shown, using the infectivity assay described herein, to inhibit infection of cells. Other D-peptides can be similarly assessed for their ability to inhibit infectivity.

The drugs can be administered by a variety of route(s), such as orally, nasally, intraperitoneally, intramuscularly, vaginally or rectally. In each embodiment, the drug is provided in an appropriate carrier or pharmaceutical composition. For example, a cavity-binding drug can be administered in an appropriate buffer, saline, water, gel, foam, cream or other appropriate carrier. A pharmaceutical composition comprising the drug and, generally, an appropriate carrier and optional components, such as stabilizers, absorption or uptake enhancers, flavorings and/or emulsifying agents, can be formulated and administered in therapeutically effective dose(s) to an individual (uninfected or infected with HIV). In one embodiment, drugs which bind the N-helix coiled-coil of gp41 (e.g., those described herein, DP178 (C. T. Wild, D. C. Shugars, T. K. Greenwell, C. B. McDanal, T. J. Matthews, ibid, 91:9770 (1994)), T649 which corresponds to residues 117-152 of HIV-1 gp41 (HXB2 strain) and is acetylated at the amino terminus and amidated at the carboxy terminus) (L. T. Rimsky, D. C. Shugars, T. J. Matthews, J. Virol., 72:986 (1998), are administered (or applied) as microbicidal agents and interfere with viral entry into cells. For example, a drug or drugs which bind(s) the HIV cavity can be included in a composition which is applied to or contacted with a mucosal surface, such as the vaginal, rectal or oral mucosa. The composition comprises, in addition to the drug, a carrier or base (e.g., a cream, foam, gel, other substance sufficiently viscous to retain the drug, water, buffer) appropriate for application to a mucosal surface or to the surface of a contraceptive device (e.g., condom, cervical cap, diaphragm). The drug can be applied to a mucosal surface, such as by application of a foam, gel, cream, water or other carrier containing the drug. Alternatively, it can be applied by means of a vaginal or rectal suppository which is a carrier or base which contains the drug or drugs and is made of a material which releases or delivers the drug (e.g., by degradation, dissolution, other means of release) under the conditions of use (e.g., vaginal or rectal temperature, pH, moisture conditions). Such compositions can also be administered orally (e.g., swallowed in capsule, pill, liquid or other form) and pass into an individual's blood stream. In all embodiments, controlled or time release (gradual release, release at a particular time after administration or insertion) of the drug can be effected by, for example, incorporating the drug into a composition which releases the drug gradually or after a defined period of time. Alternatively, the drug can be incorporated into a composition which releases the drug immediately or soon after its administration or application (e.g., into the vagina, mouth or rectum). Combined release (e.g., release of some of the drug immediately or soon after insertion, and over time or at a particular time after insertion) can also be effective (e.g., by producing a composition which is comprised of two or more materials: one from which release or delivery occurs immediately or soon after insertion and/or one from which release or delivery is gradual and/or one from which release occurs after a specified period). For example, a drug or drugs which bind the HIV cavity can be incorporated into a sustained release composition such as that taught in U.S. Patent 4,707,362. The cream, foam, gel or suppository can be one also used for birth control purposes (e.g., containing a spermicide or other contraceptive agent), although that is not necessary (e.g., it can be used solely to deliver the anti-HIV drug, alone or in combination with another non- contraceptive agent, such as an antibacterial or antifungal drug or a lubricating agent). An anti-HIV drug of the present invention can also be administered to an individual through the use of a contraceptive device (e.g., condom, cervical cap, diaphragm) which is coated with or has incorporated therein in a manner which permits release under conditions of use a drug or drugs which bind the HIV gp41 N-helix coiled coil. Release of the drug(s) can occur immediately, gradually or at a specified time, as described above. As a result, they make contact with and bind HIV and reduce or prevent viral entry into cells.

A drug which interferes with HIV entry into cells by a mechanism other than binding to the gp41 N-helix coiled-coil cavity (e.g., a drug which interferes with viral entry by interfering with gp120 binding at the CD4 stage) may be administered or applied to a mucosal surface as described above for drugs which bind to the gp41 N-helix coiled coil.

Fusion proteins described herein may comprise a soluble, trimeric form or version of a coiled-coil, such as a soluble, trimeric form or version of a coiled-coil region of a protein (of non-HIV origin or of HIV origin) and a sufficient portion of the C-terminal end of the N peptide of HIV gp41 to include (comprise) the HIV coiled-coil cavity or hydrophobic pocket (the pocket-comprising residues of the N-peptide). The N peptide of HIV gp41 can be that of HIV-1, HIV-2. another HIV strain or a strain from another species (e.g., simian immunodeficiency virus (SIV), feline immunodeficiency virus or Visna virus). For example, HIV-2 sequence LLRLTVWGTKNLQARVT (SEQ ID NO: 26), SIV sequence LLRLTVWGTKNLQTRVT (SEQ ID NO: 27) or a sequence comprising invariant residues in HIV-1, HIV-2 and SIV (represented LLXLTVWGYKXLQXRXX (SEQ ID NO: 42), wherein amino acid residues L, T, V, W, G, K, Q, and R arc the single letter code used for amino acid residues and X can be any amino acid residue). Also the subject of this invention is a soluble trimeric model of the HIV gp41 hydrophobic pocket, which can be a D-peptide or an L-peptide and comprises a soluble trimeric coiled coil and a sufficient portion of the N peptide region of HIV gp41 to comprise the amino aicd residues which form the pocket of the N-helix coiled-coil region of HIV gp41. The D- or L-peptide can comprise as the soluble, trimeric coiled coil the coiled coil of GCN4-pl_{Q}I, of GCN4-pII, of Moloney Murine Leukemia Virus or of the ABC heterotrimer. The component which is a sufficient portion of the N peptide of HIV gp41 to comprise the amino acid residues of the pocket can comprise, for example: LLQLTVWGIKQLQARIL of HIV-1 (SEQ ID NO:: 20); LLRLTVWGTKNLQARVT of HIV-2 (SEQ ID NO: 26); LLRLTVWGTKNLQTRVT of SIV (SEQ ID NO: 27) or the invariant residues of these, which are: LLXLTVWGXKXLQXRXX (SEQ ID NO: 42).

Also described herein are fusion proteins between a trimeric version of the coiled-coil region of a protein (such as GCN4-pI_{Q}I) and the N-helix coiled-coil of HIV gp41 that include all, part or none of the N-helix cavity. That is, a fusion protein can comprise a trimeric form of the coiled-coil region of GCN4-pI_{Q}I and a portion of the N-peptide of HIV-1 gp41, wherein the portion of the N-peptide of gp41 comprises part, or all, or none of the N-helix cavity of HIV-1 gp41. For example, a fusion protein can be made that contains residues from GCN4-pI_{Q}I and residues from N36. The fusion protein, denoted IQN24n, contains 29 residues of GCN4-pI_{Q}I, including three mutations for increased solubility, and 24 residues from the N-terminal end of N36 (SGIVQQQNNLLRAIEAQQHLLQLT) (SEQ ID NO 21); for recombinant expression in E. coli, an extra Met residue is include at the N-terminus. For example, a fusion protein can comprise a portion of the N-peptide of HIV gyp41 comprising the amino acid sequence of (SEQ ID.: 21). The sequence of IQN24n is: MRMKQIEDKIEEIESKQKKIENEIARIKKLISGIVQQQNNLLRAIEAQQHLLQL T (SEQ ID.: 22). This fusion protein can he made by a variety of methods, including chemical synthesis or recombinant DNA methods or by recombinant expression in E. coli, in which case the N- and C-termini are not blocked. Because the superhelix parameters of the GCN4-pI_{Q}I coiled coil are nearly identical to the HIV gyp41 N-helix coiled coil, the resulting fusion protein molecule (IQN24n) is predicted to form a long trimeric coiled coil, which presents part of the gyp41 N-helix coiled coil as a trimer (not aggregated).

The invention finds application in a method of eliciting an immune response in an individual. The strategy used to create a soluble, trimeric model for part of the gp41 N-terminal region coiled coil is also helpful to develop HIV vaccine candidates. One goal for a potential HIV vaccine is to elicit a neutralizing antibody response that binds to the "pre-hairpin" intermediate of the HIV-1 gp120/gp41 envelope protein complex. In this transient form, the N-Helix region of gp41 is exposed, but the C-helix region is not. Although it seems reasonable to use an N-peptide (such as N36, N51 or DP-1 07) as an immunogen to elicit an antibody response against the N-helix region of gp41, the isolated N-peptides are aggregated and do not properly present the gp41 N-helix coiled-coil trimer. Accordingly, the same strategy described herein to solve this problem for the gp41 hydrophobic pocket can be applied towards the development of soluble, trimeric models of the gp41 N-helix coiled-coil region, in general. Such trimeric models (including IQN 17, but also including, for example, peptides that do not contain the pocket residues of gp41) can be used as immunogens to elicit an antibody response to the pre-hairpin intermediate, thereby inhibiting HIV-1 infection. For example, an individual to be immunized can be administered a fusion protein comprising a trimeric form of a coiled-coil region of a protein and a portion of an N-peptide from HIV-1 gp41, wherein the portion from gp41 comprises part of, all of, or none of the N-helix coiled-coil cavity in a pharmaceutically acceptable carrier. For example, IQN24n can be used, either alone or in combination with other materials, in a vaccine, which will elicit the production of antibodies that bind to the coiled coil in the individual to whom it is administered (the vaccinee), and thereby offer protection against infection and/or disease. IQN24n can also be used to identify (from humans, other animals or antibody libraries) and/or raise antibodies (monoclonal and/or polyclonal) that bind to the N-helix coiled coil. This provides the basis for a diagnostic method in which IQN24n (or IQN17 or other soluble trimeric model) is used to assess the presence/absence/level of antibodies that bind the N-helix coiled coil in a biological sample (e.g., blood).

Any of a wide variety of variations can be made in the GCN4-pI_{Q}I component of fusion proteins described herein (e.g., IQN17 or IQN24n) and used in the method, provided that these changes do not alter the trimeric state of the coiled-coil. Changes can also be made in the amino acid composition of the fusion protein component which is the portion from the HIV gp41 N36 peptide, to produce variants (e.g., variants of IQN17 or IQN24n). There is no limit to the number or types of amino acid residue changes possible, provided that the trimeric state of the coiled-coil and the structure of the surface of the fusion protein corresponding to the N-peptide coiled coil of HIV gp41 are maintained. The fusion protein component which is the portion of the HIV gp41 N-peptide can include all, part, or none of the N-helix cavity. For example, other parts of N51, N36, DP-107, or other regions of the HIV gp41 N-helix region can be fused to GCN4-pI_{Q}I (or another trimeric version of the coiled-coil region of a protein) to generate trimeric (not aggregated) helical coiled-coil fusion proteins and used in the method. There is no limit to the number or types of fusion proteins that can be designed and generated, provided that the trimeric state of the coiled-coil and the structure of the surface of the fusion protein corresponding to the N-peptide coiled coil of HIV gp41 are maintained. Such fusion proteins can be designed and generated using methods known to those of skill in the art, such as evaluating heptad-repeat positions or superhelix parameters of coiled coils,

Described herein are peptides, which can be D-peptides or L-peptides, which bind to a cavity on the surface of the N-helix coiled-coil of HIV envelope glycoprotein gp41 (e.g., HIV-1, HIV-2). Such peptides can be of any length, provided that they are of sufficient length to bind the cavity in such a manner that they interfere with the interaction of the N-helix coiled-coil cavity and amino acid residues of the C-peptide region of HIV gp41 and prevent HIV entry into the cells. For example, D- or L-peptides comprise at least two amino acid residues and generally will be from about two to about 2 amino acid residues. That is, they can comprise any number of amino acid residues from about two to about 21. The amino acid residues can be naturally occurring or non-naturally occurring or modified, as described below. The peptides can be linear or circular.

Examples of D-peptides, identified as described herein, are shown in Figure 3. Because of library design, each peptide, in addition to the amino acid residue shown, is flanked by GA on the N-terminus and AA can the C-terminus. N-terminal lysine residues were added to improve water solubility.

Described herein are compounds which inhibit the binding of the N-helix coiled coil to the C-helix of HIV-1 gp41 envelope protein. Such compounds are of use in a method of treating a patient infected by, or potentially subject to infection by, HIV. These compounds are also of use in a method of assessing the ability of a second compound to bind to the N-helix coiled coil cavity.

Described herein are the compounds which inhibit the binding of the N-helix coiled coil to the C-helix of HIV-1 gp41 envelope protein of Formula I, 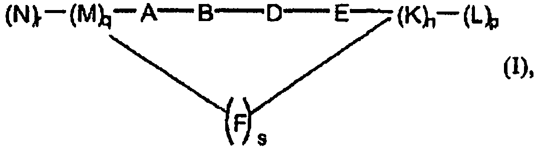 wherein A, B, D and E are each, independently, a D-amino acid residue, an L-amino acid residue, or an N-substituted glycyl residue. Natural or nonnatural amino acid residues can be used. K, L, M and N are each, independently, an amino acid residue or a polypeptide group of from 2 to about 6 amino acid residues which can be the same or different, and n, p, q and r are each, independently, 0 or 1. F is a direct bond or a difunctional linking group and s is 0 or 1.

In one subset of the compounds of Formula I, A is a D- amino acid residue, an L-amino acid residue or an N-substituted glycyl residue of the formula 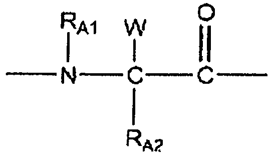 where one of R_{A1}, and R_{A2} is a substituted or unsubstituted aryl, heteroaryl, arylmethyl, heteroarylmethyl, benzo-fused aryl, benzo-fused heteroaryl, benzo-fused arylmethyl, benzo-fused heteroarylmethyl, cycloalkyl or bicycloalkyl; and the other is hydrogen. W is hydrogen, methyl, trifluoromethyl or halogen, for example, fluorine, chlorine, bromine or iodine.

B is a glycyl residue or D-amino acid or N-substituted glycyl residue of the formula 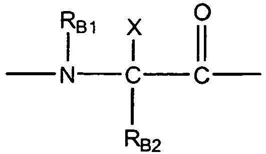 where one of R_{B1} and R_{B2} is a substituted or unsubstituted linear, branched or cyclic alkyl, aryl, arylalkyl, heteroaryl or heteroarylalkyl group; and the other is hydrogen. X is hydrogen, methyl, trifluoromethyl or halogen, such as fluorine, chlorine, bromine or iodine.

D is a D- amino acid residue or N-substituted glycyl residue of the formula 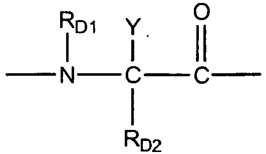 where one of R_{D1} and R_{D2} is a substituted or unsubstituted aryl, heteroaryl, arylmethyl, heteroarylmethyl, benzo-fused aryl, benzo-fused heteroaryl, benzo-fused arylmethyl; benzo-fused heteroarylmethyl, cycloalkyl or bicycloalkyl; and the other is hydrogen. Y is hydrogen, methyl, trifluoromethyl or halogen, such as fluorine, chlorine, bromine or iodine.

E is a D-amino acid residue or N-substituted glycyl residue of the formula 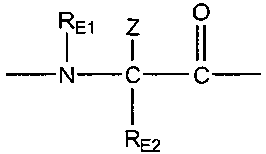 where one of R_{E1} and R_{E2} is a substituted or unsubstituted, linear, branched or cyclic alkyl, aryl or arylalkyl group; and the other is hydrogen. Z is hydrogen, methyl, trifluoromethyl or halogen, such as fluorine, chlorine, bromine or iodine.

K, L, M and N are each, independently, composed of from 1 to about 6 (which can be the same or different), D-amino acid residues, L-amino acid residues, N-substituted glycyl residues or a combination thereof. Natural or nonnatural amino acid residues can be used. One or more of the amino acid residues or N-substiuted glylcyl residues can, optionally, be substituted at the α-carbon by a methyl or trifluoromethyl group, or a halogen, such as a fluorine, chlorine, bromine or iodine atom.

One of R_{A1} and R_{A2} and one of R_{D1} and R_{D2} may be, independently, a phenyl, substituted phenyl, naphthyl, substituted naphthyl, naphthyl methyl substituted naphthylmethyl, benzyl or substituted benzyl group, or a group of the formula 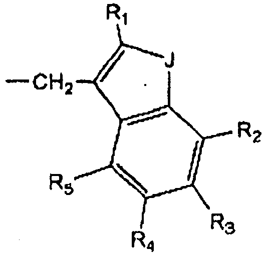 where J is O, S or NR, where R is H or linear, branched or cyclic C₁-C₄-alkyl, preferably methyl, R₁, R₂, R₃, R₄ and R₅ are independently selected from the group consisting of hydrogen, halogen and alkyl, preferably, linear, branched or cyclic C₁-C₄-alkyl, such as methyl, Suitable phenyl, naphthyl, naphthyl methyl and benzyl substituents include alkyl, preferably linear, branched or cyclic C₁-C₄-alkyl, such as methyl and halogen, such as flourine, chloride, bromine or iodine. More preferably, R_{A1} and R_{D1} are both hydrogen, and R_{A7} and R_{D2} are each, independently, one of the foregoing groups.

Preferably, one of R_{B1} and R_{B2} is hydrogen, substituted or unsubstituted linear, branched or cyclic C₁-C₄-alkyl, phenyl, benzyl, naphthyl or naphthylmethyl, Suitable substituents include linear, branched or cyclic C₁-C₄-alkyl groups and halogens, such as fluorine, chlorine, bromine or iodine. More preferably, Rₘ is hydrogen and Rₘ is one of the foregoing groups.

Preferably, once of R_{E1} and R_{E2} is a substituted or unsubstituted, linear, branched or cyclic C₁-C₆-alkyl group or a substituted or unsubstituted phenyl or naphthyl group. Suitable substituents include lineal, branched or cyclic C₁-C₄-alkyl groups, such as methyl, and halogens, such as fluorine, chlorine, bromine and iodine. More preferably, R_{E1} is hydrogen and R_{E2} is one of the foregoing groups.

In a preferred subset of the compounds of formula I, A and D are each a D-tryptophan residue and E is a D-leucine residue.

Preferably, K is a D-amino acid residue or an N-substituted glycyl residue comprising an amino-, carboxyl- or sulfhydryl substituted side chain, such as a cysteine, glutamic acid, aspartic acid or lysine residue, and L is a polypeptide comprising 2 or 3 D-amino acid residues, L-amino acid residues (the D- or L-amino acid residues can be the same or different) or N-substituted glycine residues. For example, in one embodiment, L comprises 2 or 3 residues selected from among D-glycine, D-alanine or D-α-C₁-C₄-alkylglycine.

Preferably, M is a polypeptide group comprising from 2 to about 8 D-amino acid residues, of which at least one comprises an amino-, carboxy- or sulfhydryl substituted side chain, such as a cysteine, glutamic acid, aspartic acid or lysine residue. N is, preferably, a polypeptide group comprising from 1 to about 6 amino acid residues, of which at least one is a lysine residue.

The identity of divalent linking group F is not critical, as long us it is of a suitable length to position residues A to E to interact with the N-helix coiled coil cavity (J.R. Morphy, Curr. Op. Drug Discov. Develop., 1:59-65 (1998)). For example, F preferably has a length from about 2 to about 40 atoms. In one embodiment, F is a direct bond or a polypeptide linking group of the formula -Pₙ- wherein n is 1 to about 12 and each P is independently an L- or D- amino acid or N-substituted glycyl residue residue, a glycyl residue or an N-substituted glycyl derivative.

F may be a substituted or unsubstituted C₄-C₄₀-alkylene group, such as a polymethylene group of the formulate -(CH₂)ₘ-, wherein m is from about 4 to about 40; an alkylene group which is interrupted at one or more points by a heteroatom, such as a nitrogen, oxygen or sulfur atom. For example, F can he a group (CH₂CH₂O)_{q}-, wherein q is from 1 to about 20. F can also he an alkylene group which is interrupted at one or more points by a phenylene or heteroarylene group, or a polysaccharide group, for example, a glycoside or poly(glycoside) group comprising one or more glycoside groups, for example, from 1 to about 10 glycoside groups. Suitable glycosides include glucoside, lactoside, mannoside, galactoside, fucoside, fructoside, guloside, alloside, altroside, taloside, idoside and others, such as pyranosides and furanosides, which are known in the art.

In compounds of Formula I having a C-terminal amino acid residue, the C-terminal residue can be, for example, in the form of an amide, an N-substituted amide or a carboxylic acid protecting group, as is known in the art. The nitrogen atom of an N-terminal residue can be acylated, for example, acetylated, or substituted with an amino protecting group, as is known in the art.

The term "D-amino acid residue", as used herein, refers to an α-amino acid residue having the same absolute configuration as D-glyceraldehyde. When the amino acid residue includes a first non-hydrogen α substituent and a second α substituent selected from methyl and halogen, the absolute configuration is the same as that of D-glyceraldehyde with the second α substituent taking the place of the hydrogen atom at the glyceraldehyde α-carbon.

The peptides, portions of the peptides, variations/derivatives of the peptides or portions of the variations/derivatives described herein can be used as inhibitors of HIV entry into cells. The peptides represented in Figure 3 or a portion of a peptide sufficient to fit into the hydrophobic pocket at the C-terminal end of the coiled-coil and prevent interaction of the C-peptide region with the N-peptide region of gp41 are useful to inhibit HIV infection. A portion of any of the peptides represented or of a derivative thereof can be from 2 to 20 (any number of residues from 2 to 20) amino acid residues in size. D-peptides which comprise the consensus sequence tryptophan-tryptophan-leucine or the sequence tryptophan-tryptophan-leucine-glutamate, described herein, and additional residues, can be used; the other residues present in such D-peptides and the size of the D-peptides can be selected with reference to peptides described herein or can be designed independent of those peptides, provided that these three or four residues are positioned in such a manner that the peptide can fit into the hydrophobic pocket and act as an inhibitor. Additional amino acid residues can also be present at the N-terminus, the C-terminus or both of the D-peptides described herein, thus producing a larger peptide. Alternatively, there can be other amino acid residues selected, for example, to enhance binding affinity. Alternatively, a peptide which comprises the conserved amino acid residues of the D-peptides of Figure 3 can be used. For example, such a peptide can be 16 amino acid residues in size and include the conserved amino acid residues, which can be at the same positions as those at which they occur in the peptides shown in Figure 3. The intervening amino acid residues can be different from the amino acid residues at these positions in any of the peptides shown in Figure 3 (e.g., can be isoleucine or asparagine or other amino acid residue which does not appear in the peptides represented in Figure 3) or can be substituted for or replaced by an amino acid residue represented at a specific position in another peptide shown in Figure 3 (e.g., the aspartic acid residue in D10pep1 can be replaced by a serine residue). Amino acid residues other than the D-versions of the 20 L-amino acids found in natural proteins can be used. Such changes can be made, for example, to enhance bioavailability, binding affinity or other characteristic of the peptide. A D-peptide can comprise the conserved amino acid residues present in the peptides shown in Figure 3, but they can be separated by fewer (or more) amino acid residues than the number of intervening amino acid residues shown in Figure 3. For example, fewer than five amino acid residues (e.g., Tarrago-Litvak, L. et al., FASEB, J., 8:497 (1994); Tucker, T.J. et al., Methods Enzymol., 275:440 (1996), Tarrago-Litvak, L. et al., FASEB, J., 8:497 (1994); Tucker, T.J. et al., Methods Enzymol., 275:440 (1996)), can be present between the first cysteine and the glutamic acid in the consensus sequence shown in Figure 3. Alternatively, these two residues can be separated by more than five amino acid residues. Internal modifications can also be made (e.g., to enhance binding or increase solubility of a peptide). For example, the first tryptophan of D10pep5 can be replaced by an arginine to increase solubility. A D-peptide can have additional moieties or amino acids at its N-terminus. For example, a moiety which blocks the N terminus or gets rid of the charge otherwise present at the N-terminus can be added. The moiety can be, for example, a blocking moiety, such as an acetyl group linked directly to the glycine (G), or an acetyl group linked to one or more additional amino acid residues linked to the N-terminal of G, such as an acetyl group linked to one or more lysine residues, which, in turn, are linked to the N terminal G. In one embodiment, two lysine residues are linked to the N-terminal G (KKGAC ....), for example to increase the solubility of the peptide; a blocking moiety, such as an acetyl group, can be linked to the terminal lysine (acetyl group KKGAC ....). In another embodiment, four lysine residues are linked to the N-terminal G. In addition, a D-peptide can have additional and/or altered moieties or amino acids at its C-terminus. For example, one or both of the alanine residues at the C-terminus can be altered and/or one or more residues can be added at the C-terminus, for example to enhance binding. Alternatively, functional (chemical) groups other than amino acid residues can be included to produce an inhibitor of the present invention. For example, these additional chemical groups can be present at the N-terminus, the C-terminus, both termini or internally. In addition, two or more D-peptides can be linked via an appropriate linker (e.g., a linker of amino acid residues or other chemical moieties) to increase the effectiveness of inhibition. Alternatively, one or more D-peptides can be linked via an appropriate linker to a molecule (drug) that binds to HIV gp120, CD4, CCR5, CXCR4, or a non-pocket region of HIV gp41 to increase the effectiveness of inhibition.

The D-peptides (or L-peptides or peptides with both D- and L-amino acids) can be produced using known methods, such as chemical methods or recombinant technology. The polypeptide backbone can be altered (e.g., N-methylation) or replaced with alternative scaffolds (e.g., peptoids) at one or more positions of the peptides. Additional components can be included in the peptides, such as, for example, linkers (chemical, amino acid) which are positioned between amino acids or amino acid portions of the peptide (e.g., to provide greater flexibility or to provide greater rigidity). As described herein, the D-peptides of the present invention are flanked by GA at the N-terminus and AA at the C-terminus, due to the design of the library used in identifying the D-peptides. Some or all of these four amino acid residues may be altered, replaced or deleted in order to produce D-peptides with, for example, altered absorption, distribution, metabolism and/or excretion. In one embodiment, the C-terminus is modified by the addition of a glycine residue immediately before the C-terminal amide. In another embodiment, the most C-terminal A is altered/modified or replaced by a different amino acid residue or deleted.

D-peptides, which are of the opposite handedness from the handedness of naturally-occurring peptides, do not serve as efficient substrates for enzymes, such as proteases, and, therefore, are not as readily degraded as L-peptides. In addition, there is no effective immune response which targets D-peptides and therefore, they do not elicit an immune response comparable to that elicited by L amino acid peptides.

The present invention is illustrated by the following examples, which are not intended to be limiting in any way.

### Example 1 Synthesis of Variants of the C34 Peptide

Mutant peptides were synthesized by solid-phase FMOC peptide chemistry and have an acetylated amino terminus and an amidated carboxy terminus. After cleavage from the resin, peptides were desalted with a Sephadex G-25 column (Pharmacia), and then purified by reverse-phase high-performance liquid chromatography (Waters, Inc.) on a Vydac C18 preparative column using a linear water-acetonitrile gradient and 0.1% trifluoroacetic acid. Peptide identities were verified by MALDI mass spectrometry (Voyager Elite, PerSeptive Biosystems). Peptide concentrations were measured by tryptophan and tyrosine absorbance in 6 M GuHCl [H. Edelhoch, Biochemistry, 6:1948 (1967)].

### Example 2 Quantitation of Helical Content and Thermal Stability of Mutant N36/C34 Complexes

CD measurements were performed in phosphate-buffered saline (50 mM sodium phosphate, 150 mM NaCl, pH 7.0) with an Aviv Model 62DS spectrometer as previously described (M. Lu, S. C. Blacklow, P. S. Kim, Nat. Struct. Biol., 2:1075 (1995)). The apparent melting temperature of each complex was estimated from the maximum of the first derivative of [θ]₂₂₂ with respect to temperature. The mean residue ellipticities ([θ]_{222'} 10³deg cm² dmol⁻¹) at 0°C were as follows: wildtype, -31.7; Met⁶²⁹→Ala; -32.0; Arg⁶³³→Ala, -30.7; IIe⁶³⁵→Ala, -25.9; Trp⁶²⁸→Ala, -27.0; Trp⁶³¹→Ala, -24.9. In the case of the Trp⁶²⁸→Ala and Trp⁶³¹→Ala mutations, the decrease in [θ]₂₂₂ is likely to overestimate the actual reduction in helical content. The removal of tryptophan residues from model helices has been reported to significantly reduce the absolute value of [θ]₂₂₂ even when there is little change in helical content (A. Chakrabartty, T. Kortemme, S. Padmanabhan, R. L. Baldwin, Biochemistry, 32:5560 (1993)).

### Example 3 Identification of Peptides Which Bind to a Pocket on the Surface of the N-helix Coiled-Coil of HIV-1 gp41.

Methods are available to identify D-peptides which bind to a cavity on the surface of the N-helix coiled-coil of HIV envelope glycoprotein gp41. As described in detail below, D-peptides which bind to a cavity on the surface of the N-helix coiled-coil of HIV-1 envelope glycoprotein gp41 were identified by mirror-image phage display. This method involves the identification of ligands composed of D-amino acids by screening a phage display library. D-amino acid containing ligands have a chiral specificity for substrates and inhibitors that is the opposite of that of the naturally occurring L-amino ligands. The phage display library has been used to identify D-amino acid peptide ligands which bind a target or desired L-amino acid peptide (Schumacher et al. Science, 271:1854-1857 (1996)).

D-peptides that bind to the hydrophobic pocket of gp41 were identified using a target that is an enantiomer ofIQN17, a hybrid molecule containing 29 residues of GCN4-pI_{Q}I on the N-terminal end and 17 residues of gp41 on the C-terminus. The phage library used for selection is described in U.S. Patent 5,780,221 and Schumacher et al. Science, 271:1854-1857 (1996). The complexity of the library is greater than 10⁸ different sequences. The sequences are flanked on either end by either a cysteine or a serine, with ten random residues in the middle. These sequences are located in the pIII gene of the phage, a coat protein that is expressed as approximately five copies on the outer surface of the phage.

The following experimental procedures were used in the examples described herein.

### Phage Display

Neutravidin (Pierce, 10 µg in 100 µL of 100 mM NaHCO₃) was added to individual wells of a 96-well high-binding styrene plate (Costar) and incubated overnight on a rocking platform at 4°C. The neutravidin was removed and the wells were washed four times with a TBS/Tween solution. Biotinylated D-IQN17 (100 µL of a 10 µL peptide solution in 100mM NaHCO₃) was added to the wells and incubated for one hour at 25°C. The biotinylated target was removed and a blocking solution (30 mg/ml nonfat dried milk in 100 mM NaHCO₃) was added to the wells and incubated for two hours, with rocking, at 4°C. The blocking solution was removed and the wells were coated again with the biotinylated target as above. The target was removed and the unliganded neutravidin was blocked by the addition of the blocking solution with 5 mM biotin. After removing the biotin, the wells were washed six times with the TBS/Tween solution. The phage stock was then added to the wells (50 µL of phage stock plus 50 µL of phage-binding buffer: TBS, 0.1% Tween-20, 1 mg/ml milk, 0.05% sodium azide). The incubation time of the phage stock in the wells decreased in increasing rounds of selection. After incubation, the phage solution was removed and the wells were washed twelve times with TBS/Tween to remove the unbound phage. Odd numbered washes were performed quickly, with no incubation time; even numbered washes were incubated for increasing amounts of time each round of phage selection. The phage were eluted by the addition of two micrograms of trypsin in 100 µL of phage-binding buffer and 2.5 mM CaCl₂ with an hour incubation at 37°C. To determine recovery, a dilution of the eluted phage was used to infect K 91 kan cells. After a one hour incubation, 100 µL of cells were removed and 1:10, 1:100, and 1:100 dilutions in LB were plated on LB/tetracycline plates. Phage recovery was determined as a ratio of transducing units recovered (the titer of the eluted phage) to the input number of transducing units (the titer of the phage stock used that round). Transducing units were determined by counting the number of tetracycline-resistant colonies on the LB/tetracycline plates. Non-specific phage recovery generally has a ratio in the order of magnitude of 10⁻⁸ to 10⁻⁹, whereas specifically amplified phage have a ratio 10⁻⁷ or greater. Individual clones were amplified and sequenced. They were assayed in the binding assay to determine binding specificity.

D10pep7 was identified after five rounds of phage selection. D10pep1, D10pep3, D10pep4, D10pep5, and D10pep6 were identified after seven rounds of phage selection. The phage selection was performed again, with shorter incubation times and longer washes, and D10pep10 and D10pep12 were identified after three rounds of selection. (A ninth D-peptide was identified but was not further investigated once it was shown to be toxic to cells.)

To test the specificity of binding of identified phage clones to the pocket of D-IQN17, the phage clones were added to wells of 96-well plates coated as above with D-INQ17, D-GCN4-pI_{Q}I (with the three mutations), D-IQN17(G39W = glycine36 substituted with tryptophan), or wells with no target. The phage were incubated on the plates and washed for the same lengths of time as in the round from which they were identified. Eluted phage were used to infect K91 kan cells and the recovered transducing units were determined as above. These sequences bound specifically to the wells with D-IQN17.

### Peptide Purification

IQN17 and the D10 peptides were synthesized by FMOC peptide chemistry. They have an acetylated N-terminus and a C-terminal amide. IQN17 contains 29 residues derived from GCN4-pI_{Q}I on the N-terminus and 17 residues from the C-terminus of N36 on the C-terminus. There is one residue overlap between GCN4-pI_{Q}I and the N36 region, making the peptide 45 residues long. To improve solubility, three amino-acid substitutions were made in the GCN4-pI_{Q}I region of IQN17, as compared to the original GCN4-pI_{Q}I sequence (Eckert, D.M. *et al., J. Mol. Biol., 284*:859-865 1998). These substitutions are L13E, Y17K, and H18K. Thus, the sequence of IQN7 is:
ac-RMKQIEDKIEEIESKQKKIENEIARIKKLLQLTVWGIKQLQARIL-am
(ac- represents an N-terminal acetyl group and -am represents a C-terminal amide),

with the HIV portion underlined. For mirror-image phage display, IQN17 was synthesized using D-amino acids (for amino acid residues that contain a second chiral center, such as Ile and Thr, the exact mirror image of the naturally occurring amino acid residue is used to create the D-version of the target). In addition, the N-terminus of the peptide was biotinylated using NHS-LC-biotin II (Pierce, catalog #21336). Between the biotin and the IQN17 sequence was a three amino acid linker of GKG, with the lysine in the naturally-occurring L-form. This lysine was inserted as a trypsin recognition site.

The sequences of the D-peptides are as follows (with all amino acids in the D-enantiomer, using the exact mirror image of naturally occurring amino acid residues for Ile and Thr, which contain a second chiral center):

| | |
|---|---|
| D10pep1 ; | Ac-GACEARHREWAWLCAA-CONH₂ (SEQ ID NO: 34); |
| D10pep3: | Ac-KKGACGLGQEEWFWLCAA-CONH₂ (SEQ ID NO: 64); |
| D10pep4: | Ac-GACDLKAKEWFWLCAA-CONH₂ (SEQ ID NO: 35); |
| D10pep5: | Ac-KKGACELLGWEWAWLCAA-CONH₂ (SEQ ID NO: 65); |
| D10pep6: | Ac-GACSRSQPEWEWLCAA-CONH₂ (SEQ ID NO: 36); |
| D10pep7: | Ac-GACLLRAPEWGWLCAA-CONH₂ (SEQ ID NO: 37); |
| D10pep10: | Ac-KKGACMRGEWEWSWLCAA-CONH₂ (SEQ ID NO: 67); and |
| D10pep12: | Ac-KKGACPPLNKEWAWLCAA-CONH₂ (SEQ ID NO: 68). |

After cleavage from the resin, the peptides were desalted on a Sephadex G-25 column (Pharmacia) and lyophilized. The lyophilized peptides were purified by reverse-phase high performance liquid chromatography (Waters, Inc.) on a Vydac C18 preparative column. The D-peptides were then air-oxidized by dissolving the lyophilized powder in 20 mM Tris, pH 8.2, and stirring at room temperature for several days. The oxidized peptides were HPLC purified as before. The expected molecular weights of the peptides were verified using MALDI-TOF mass spectrometry (PerSeptive Biosystems). Peptide concentrations were determined using tyrosine, tryptophan and cysteine absorbance at 280 nm in six molar GuHCl (Edelhoch, 1967). Peptide stock solutions were prepared in DMSO.

The N-terminal lysines on D10pep3, D10pep5, D10pep7a, D10pep10 and D10pep12 were added to increase the water solubility of the peptides. To investigate the effect of the added lysines on the inhibitory activity of the peptides, D10pep1 was synthesized with two N-terminal lysines (denoted D10pep1a) and compared to D10pep1 without lysines: D10pep1a was found to have an IC₅₀ for inhibition of syncytia formation approximately 2-fold higher than D10pep1 (i.e., without lysines). In addition, D10pep5 was synthesized with two additional N-terminal lysines (for a total of four lysines to generate a peptide denoted D10pep5a). The IC₅₀ for inhibition of syncytia formation of D10pep5a was approximately 2-fold higher than D10pep5. The addition of N-terminal lysine residues to the D-peptides results in only a modest decrease of inhibitory activity.

D-peptides that had additional D-Lys residues added to the N-termini, that were synthesized for study are indicated with the addition of "a" to the peptide name and include the following:

| | |
|---|---|
| D10pep1a: | Ac-KKGACEARHREWAWLCAA-CONH₂ (SEQ ID NO: 38); |
| D10pep4a: | Ac-KKGACDLKAKEWFWLCAA-CONH₂ (SEQ ID NO: 39); |
| D10pep5a: | Ac-KKKKGACELLGWEWAWLCAA-CONH₂ (SEQ ID NO: 66); |
| D10pep6a: | Ac-KKGACSRSQPEWEWLCAA-CONH₂ (SEQ ID NO: 40); and |
| D10pep7a: | Ac-KKGACLLRAPEWGWLCAA-CONH₂ (SEQ ID NO: 41). |

These sequences are also represented in Figure 3. The 12 amino acid "core" of each D-peptide (which, in turn comprises a 10-mer and the consensus sequences described herein) are as follows:

| | |
|---|---|
| CDLKAKEWFWLC | (SEQ ID NO: 3) |
| CEARHREWAWLC | (SEQ ID NO: 4) |
| CELLGWEWAWLC | (SEQ ID NO: 5) |
| CLLRAPEWGWLC | (SEQ ID NO: 6) |
| CSRSQPEWEWLC | (SEQ ID NO: 7) |
| CGLGQEEWFWLC | (SEQ ID NO: 8) |
| CMRGEWEWSWLC | (SEQ ID NO: 9) |
| CPPLNKEWAWLC | (SEQ ID NO: 10) |
| CVLKAKEWFWLC is an alternative sequence for peptide SEQ ID NO: 3. (SEQ ID NO: 11). | |

It is readily apparent that there is a highly conserved consensus sequence in these peptides. The 12 amino acid peptide represented in Figure 3 can be represented as: CXXXXXEWXWLC (SEQ ID NO: 63), where amino acid residues common to the peptides are shown and X represents an amino acid residue which is not conserved among the peptides.

### Example 4 Assessment of Activity of C34 Peptides and D-Peptides

The potency of C34 peptides in inhibiting viral infection and the HIV-1 infection inhibitory activity of the D-peptides were assayed using recombinant luciferase-expressing HIV-1 (Chen, B.K. et al., J Virol., 68:654 (1994); Malashkevich, V.N., et al. Proc. Natl. Acad. Sci., USA, 95:9134 (1998)). The virus was produced by co-transfecting an envelope-deficient HIV genome NL43LucR-E-(Chen, B.K. et al., J Virol., 68:654 (1994) and the HXB2 gp 160 expression vector pCMVHXB2gp160 (see Chan, D.C. et al., Proc. Natl. Acad. Sci., 95:11513 (1998)) into 293T cells. Low-speed centrifugation was used to clear the viral supernatants of cellular debris. The supernatant was used to infect HOS-CD4/Fusion cells (N. Landau, NIH AIDS Reagent Program) in the presence of the D-peptides, with concentrations ranging from 0 to 500 µM. Cells were harvested 48 hours post-infection, and luciferase activity was monitored in a Wallac AutoLumat LB953 luminometer (Gaithersburg, MD). The IC₅₀ is the peptide concentration that results in a 50% decrease in activity relative to control samples lacking peptide. The IC₅₀ was calculated from fitting the data to a Langmuir equation [y=k/(1+([peptide]/IC₅₀) + x], where y = luciferase activity and k and x are scaling constants.

### Cell/Cell Fusion Assay

Inhibition of cell/cell fusion (i.e., syncytia formation) was assayed by co-culturing Chinese hamster ovary cell expressing HXB2 envelope (K. Kozarsky, *et al*., *J. Acquir. Immune. Defic. Syndr., 2*:163 (1989) and the HeLa-CD4-LTR-Beta-gal cells (M. Emerman, NIH AIDS Reagent program) in the presence of varying concentration of peptide. When mixed, these cells form syncytia, or multi-nucleated cells, which express β-galactosidase. Approximately twenty hours after co-culturing the cells, the monolayers were stained with 5-bromo-4-chloro-3-indolyl-β-D-galactoside to visualize the syncytia. The syncytia are visualized with a microscope and counted manually (a syncytia is scored as a fused cell containing three or more nuclei). The IC₅₀ was calculated from fitting the data to a Langmuir equation [y = k/(1 + [peptide]/IC₅₀) + x], where y = number of syncytia and k and x are scaling constants.

**Table 1 Stability of mutant N36/C34 complexes and the inhibitory potency of C34 mutants.**

| | | | |
|---|---|---|---|
| Peptide | Tₘ (°C) | IC₅₀ (nM) viral entry | IC₅₀ (nM) cell fusion |
| | | | |
| Wildtype | 66 | 2.1 ± 0.31 | 0.55 ± 0.03 |
| | | | |

| Cavity-binding | | | |
|---|---|---|---|
| Trp⁶²⁸→Ala | 53 | 10 ± 2.0 | 3.8 ± 0.33 |
| Trp⁶³¹→Ala | 37 | 61 ± 16 | 15 ± 0.82 |
| Ile⁶³⁵→Ala | 55 | 4.1 ± 0.91 | 0.96 ± 0.12 |
| | | | |

| Control residues | | | |
|---|---|---|---|
| Met⁶²⁹→Ala | 66 | 2.0 ± 0.27 | 0.74 ± 0.03 |
| Arg⁶³³→Ala | 65 | 2.6 ± 0.89 | 0.76 ± 0.07 |

Mutant C34 peptides (10 µM) were complexed with the N36 peptide (10 µM) in phosphate-buffered saline (pH 7.0) for circular dichroism (CD) measurements. The apparent melting temperatures (Tₘ) were estimated from the thermal dependence of the CD signal at 222 nm. Inhibition of viral entry was measured in a cell-culture infection assay using recombinant luciferase-expressing HIV-1. Inhibition of cell-cell fusion was measured in a syncytium assay. The means and standard errors are from triplicate trials.

Similarly, the activity of the D-peptides described was assessed using the two assays described above. Results are shown in figures 6A-6B and 8A-8B.

### Example 5: Crystallization of the IQ17/D10pep1 Complex and Ligand-Free IQN17

### Peptide Purification, Crystallization

Peptides IQN17 and D10pep1 were synthesized by FMOC peptide chemistry, as described above.

A 10 mg/ml stock of a mixture of IQN17 and D10pep1 was prepared in water. The final concentration of IQN17 was about 1.37 nM, and the final concentration of D10pep1 was about 1.51 mM. Initial crystallization conditions were found using Crystal Kits I and II (Hampton Research), and then optimized. To grow the best diffracting crystals, one microliter of this stock was added to one microliter of the reservoir buffer (10% PEG 4000, 0.1 M NaCi pH 5.6, 20 % 2-propanol) and allowed to equilibrate against the reservoir buffer. Crystals belong to a space group P321 (a=b=41.83Å; c=84.82Å, α=β=90°, γ=120°) and contain one IQN17/D10pep1 monomer in the asymmetric unit. A useful osmium derivative was produced by increasing the concentration of PEG 4000 in the reservoir solution by 4%, adding (NH₄)₂OsCl₆ to the reservoir solution to a final concentration of 5 mM and adding five microliters of the resulting solution to the drop containing the protein crystal. Prior to data collection native and heavy-atom derivative crystals were transferred into cryosolution containing 20% PEG 4000, 0.1 M NaCi PH 5.6, 20% 2-Propanol and flash-frozen using X-stream cryogenic crystal cooler (Molecular Structure Corporation).

The best diffracting crystals of ligand-free IQN17 were grown with a similar technique as above: on microliter of a 10 mg/ml solution of IQN17 in water was added to one microliter of the reservoir buffer (1.0 M K,Na Tartrate, 0.1 M NaHEPES pH 7.0) and allowed to equilibrate against the reservoir buffer. Before flash freezing, the crystals were transferred into buffers consisting of the reservoir solution with increasing amounts of glycerol, up to a final concentration of 23% glycerol. Crystals belong to the space group C222₁ (a= 57.94 Å, b=121.96 Å, c= 73.67 Å; α=β=γ=90°) and contain one IQN17 trimer in the asymmetric unit.

### X-Ray Data Collection and Processing

Initial data were collected on a Rigaku RU300 rotating-anode x-ray generator mounted to an R-axis IV area detector (Molecular Structure Corporation). Diffraction data for IQN17 were collected at 100 K using a Quantum-4 CCD detector and the 5.0.2 beamline at the Advanced Light Source (Berkeley, USA). Final native and multiwavelength anomalous diffraction (MAD) data for IQN17/D10pep1 were collected at the Howard Hughes Medical Institute Beamline X4A at Brookhaven National Laboratory using a Raxis-IV detector. For MAD data, four wavelengths near the osmium L-III absorption edge were selected based on the fluorescence spectrum of the Os derivative crystal (Table 2). The four wavelengths were: 1.1398 Å, 1.1403 Å, 1.1393 Å, 1.1197 Å. Data sets were collected in 20° batches, allowing the same batch to be collected at each wavelength before moving to the next batch, in order to minimize the crystal decay between data sets. Reflections were integrated and scaled with the programs DENZO and SCALEPACK (Otwinowski, Z., (1993) in Data Collection and Processing, eds. Sawer, L., Isaacs, N. & Bailey, S. (SERC, Daresbury Laboratory, Warrington, England), pp. 55-62).

Further diffraction data processing, phase determination and map calculations were performed using the CCP4 suite of programs (CCP4, Acta Cryst. D50:760-763 (1994)). Intensities were reduced to amplitudes with the program TRUNCATE, and the data sets for the wavelengths closest to the Os L-III absorption edge (λ1, λ2, λ3) were scaled with SCALEIT to the remote wavelength (λ4) data set (Table 2).

### Phase Determination and Crystallographic Refinement

Initially, phase determination for IQN17/D10pep1 crystals was attempted with the molecular replacement technique using the theoretical model ofIQN17 build from the published GCN4-pI_{Q}I and HIV gp41 structures (Eckert, D.M., et al. (1998) J. Mol. Biol. 284:859-865; Chan, D.C., et al. (1997) Cell 89, 263-273) with sidechains truncated to a polyserine chain. The resulting molecular replacement solutions were ambiguous and the electron density map did not reveal conformation of the D10pep1 peptide. The molecular replacement phases were good enough, however, for determining the coordinates of a single Os atom in the corresponding derivative using difference and anomalous fourier maps. The heavy atom binds on the cryallographic three-fold axis (0.333, 0.667, 0.047). MAD phases were then generated with the program MLPHARE (Table 2) and extended to higher resolution with the program DM. The quality of MAD electron density map at 1.5 Å resolution was exceptional, and revealed structural details of IQN 17 and D10pep1 peptide with clarity. Electron density map interpretation and model building was done with the program O (Jones, T.A. et al. (1991) Acta Crystallogr. D47, 110-119). The structure of IQN17-D10pep1 complex was refined using the program CNS (Brünger, A.T. et al., Acta Crystallogr. D54, 905-921 (1998)). The correctness of the structure was checked with simulated annealing omit maps and with the program WHAT CHECK (Hoff, R.WW. et al., Nature 381: 272 (1996)). All residues of IQN17 and the D10pep1 peptide (when converted into its mirror image) occupy most preferred areas of the Ramachandran plot. The conformations of the majority of the residues are well defined except for the two most N-terminal residues of IQN17 and the side chains of Arg-6 and Arg-8 of the D10pep1 peptide.

The structure of ligand-free IQN17 was solved by molecular replacement using the program AMORE (Navaza, J. (1994) Acta Crystallogr. A50, 157-163) and the IQN17 part of the refined IQN17/D10pep1 structure as a test model. Three-fold noncrystallographic averaging, solvent flattening and histogram matching with the program DM was used for phase improvement. Electron density map interpretation and model building was done with the program O (Jones et al., Acta Crystallogr. D54, 905-921 (1991). The structure of the IQN17/D10pep1 complex was refined using the program CNS (Brunger, A.T. et al., Acta Crystallogr. D54, 905-921 (1998)).

The crystal structure can be used to design more effective and/or new D-peptides, peptidomemetics or other small molecules that inhibit HIV infectivity.

### Example 6 Nuclear magnetic resonance (NMR) methods for identifying compounds which bind to the N-helix hydrophobic pocket of gp41

### A. Assaying specific binding between the IQN17 hydrophobic pocket and D-peptides

NMR experiments were used to assay the binding of each D-peptide to IQN17. The single tryptophan residue of IQN17 (denoted Trp-571) provides an excellent probe of specific binding to the hydrophobic pocket of gp41. In deuterium oxide (deuterated water) buffers, the simple homonuclear one-dimensional ¹H NMR spectrum ofIQN17 (Figure 9A, middle) shows five signals from the Trp-571 indole, extremely well-resolved from all other signals in the molecule. To test a compound for binding to the gp41 pocket, two one-dimensional ¹H NMR measurements were made on samples in deuterated buffers. First, a reference (control) spectrum of IQN17 was taken, identifying the Trp571 chemical shifts in the unbound form. A second spectrum was acquired on a sample containing both IQN17 and the compound in question. An optional third spectrum of the D-peptide (or other small molecule, or mix of molecules) was also taken. ¹H NMR experiments were performed on a Bruker AMX 500 spectrometer. Data was processed in Felix 98.0 (MSI) on Silicon Graphics computers, and all spectra were referenced to DSS. All experiments were performed at 25° C in 100 mM NaCl, 50 mM sodium phosphate (pH 7.5). All buffers used were >99.7% D₂O to remove overlapping resonances from exchangeable backbone and side chain protons. Solute concentrations ranged from 0.3-1.0 mM for individual peptides, 0.8-1.0 mM for 1:1 commplexes of IQN17 with each D-peptide.

Simple binding of two or more components is expected to result both in broader peaks (due to the increased size of the complex) and in changes in chemical shifts (due to the different chemical environments experienced by nuclei in free and bound forms). Specific binding to the hydrophobic pocket is indicated by a change in the Trp-571 chemical shifts, as well as by a broadening of peaks. Binding can also be indicated by similar changes in the chemical shifts and peak widths of the molecule (peptides and small organic molecules, for example) assayed. Figure 9A shows an example of these effects: the NMR spectrum of the IQN17/D10pep1a complex displays broader peaks and dramatically different chemical shifts than the spectra for either of the two separate components. All IQN17/D-peptide complexes studied gave similar results, though varying in the degree of chemical shift dispersion (Figure 9B). Thus, binding was indicated in all cases.

The x-ray crystallographic finding that the two conserved Trp residues, and the conserved Leu residue, in D10pep1 are directly involved in the binding of the IQN17 pocket, strongly suggests that these conserved residues participate in a similar manner when the other D-peptides bind the pocket. These conserved trypophan residues, and Trp-571 of IQN17, provide an opportunity to study the binding interfaces in greater detail. In the IQN7/D1pep1 crystal structure, the Trp-571 sidechain of IQN17 is in close contact with Trp-10 of D10pep1, with several protons of Trp-571 (H_{ζ2}, H_{η2}, H_{ζ3}, H_{∈3}; the four scalar-coupled protons of the aromatic ring) above the plane of the Trp-10 indole group. In this position, aromatic ring current interactions (F.A. Bovey, Nuclear Magnetic Resonance Spectroscopy (1988)) are expected to alter the chemical shifts of some of those protons, moving peaks upfield in the manner seen (Figure 9A, bottom). Use of the structure-based chemical shift prediction program SHIFTS (version 3.0b2, K. Osapay, D. Sitkoff, D. Case) also predicted that only protons from Trp-571 will experience a large upfield shift, expecially the H_{ζ3} proton. If the other D-peptides bind to the IQN17 pocket in the same fashion as D10pep1, a similar juxtaposition of Trp-571 and Trp-10 should occur, resulting in upfield-shifted peaks. All of the D-peptide/IQN17 complexes studied displayed such peaks, though varying in the extent of the shift (Figure 9B). The D10pep1 complex showed the most extreme upfield shifts, and the D10pep7a complex the least. The magnitude of these changes is very large, ranging from roughly 0.5 to 2 ppm for the most upfield-shifted proton (H_{ζ3}, in all cases where it could be assigned). In comparison, chemical shift differences often used to detect binding in SAR by NMR experiments (Shuker, S.B., Hajduk, P.J., Meadows, R.P., Fesik, S.W., Science 274:1531-1534 (1996)) are frequently in the range of 0.05 to 0.2 ppm.) Though a broad range of upfield chemical shifts was observed, ring-current effects can be highly sensitive to distance and orientation, so that small structural differences may give rise to substantial variations in chemical shift. (All of the upfield shifts observed are consistent with the approximate orientation ofTrp side chains expected from the x-ray crystal structure.) Also, the upfield-shifted peaks are somewhat broadened compared to others in these NMR spectra (most likely due to some type of exchange process) an effect particularly pronounced for the complexes with D10pep5a and with D10pep7a.

To confirm that the strongly upfield-shifted peaks all correspond to a single sidechain (almost certainly Trp-571), two-dimensional NMR (TOCSY) experiments were performed on each of the IQN17/D-peptide complexes. As expected, the TOCSY experiments indicate that in each complex, the strongly upfield-shifted resonances all belong to the same aromatic side chain, identified as a group of four scalar-coupled protons. One example TOCSY spectrum is shown in Figure 9C. For several of the complexes studied, NOESY experiments also indicate contact between this sidechain and other (unassigned) aromatic groups, as expected from the IQN17/D10pep1 structure. Not all of the potential NOE crosspeaks could be resolved, due to intense spectral overlap in the 6.8-7.6 ppm region. 2D NOESY and TOCSY experiments as described in J. Cavanaugh, W.J. Fairbrother, A.G. Palmer, N.J. Skelton, Protein NMR Spectroscopy: Principles and Practice (1996) were performed on samples of IQN17 and of each complex, with mixing times ranging between 30-90 ms (NOESY) and 30-70 ms (TOCSY). Spectral widths of 11,111 Hz and 5555 Hz were used in the acquisition (t₂) and indirect (t₁) dimensions, respectively. TOCSY experiments employed the DIPSI-2rc mixing sequence (J. Cavanaugh, M. Rance, J. Magn. Reson. Serv. A., 105:328 (1993)).

We conclude that all D-peptides assayed clearly bind the hydrophobic pocket of IQN17. Additionally, in the majority of these IQN17 complexes (i.e., D10pep1, D10pep3, D10pep4, D10pep6, D10pep10, and D10pep12) the D-peptides contact the pocket with very similar binding interfaces, bringing Trp-571 in close contact with the aromatic ring of Trp-10. In the cases of complexes with D10pep5a and D10pep7a this conclusion also seems very likely, although the more limited chemical shift dispersion and broader peaks raise a remote possibility of some other mode of binding.

The binding assay employed here can also be employed to assay binding of other molecules to the hydrophobic pocket of gp41 (e.g., such as found in IQN17). The assay is especially easy to interpret in a case where an aromatic group binds the pocket, as with the set of D-peptides described above. However, any pocket-binding molecules should also perturb the chemical shifts of Trp-571, an easily noticeable effect. In addition, new NMR signals generated by the small molecules themselves upon binding, are also indicative of binding.

The use of one-dimensional homonuclear ¹H NMR provides significant advantages over multidimensional heteronuclear NMR to determine specific binding: (1) Sensitivity is higher, allowing samples to be assayed more quickly; alternately the higher sensitivity makes possible the use of lower concentrations of IQN17 and of putative binding agents, allowing screening for higher-affinity compounds, and more of them simultaneously. (2) Non-isotopically labeled proteins are simpler to produce, and more cost-effective. However, two-dimensional NMR experiments, either homonuclear or heteronuclear (with ¹⁵N and/or ¹³C isotopic labeling) could also be employed.

### B. Screening chemical libraries

The binding assay described in (A) above can be used to screen large numbers of compounds present in a chemical library. Simple one-dimensional homonuclear ¹H NMR experiments are sufficient to assess binding, with no requirement for isotopic labeling. Two-dimensional NMR experiments, either homonuclear or heteronuclear (with ¹⁵N and/or ¹³C isotopic labeling) could also be employed. Single compounds can be screened one at a time in this process. However, multiple compounds can also be combined in the same assay with IQN17 (or any representation of the gp41 N-helix coiled coil) and screened simultaneously. Binding to the pocket by any component of the mixture is indicated by a change in the Trp-571 chemical shifts. NMR signals from a large number of compounds together have the potential to obscure signals from Trp-571; these signals from unbound molecules can be eliminated using pulsed field gradient techniques well known in the art. With use of these techniques and a commercially available NMR tube sample changer, the automated screening of large numbers of compounds is straightforward.

### C. Evaluating the products of multiple combinatorial syntheses

The screening process described in (B) above can also be extended to take advantage of combinatorial organic synthetic methods. Such methods are currently being used to generate whole families of compounds, with each family containing a diverse number of chemically related compounds. By the simple assay described above, the products of an entire combinatorial synthesis can be screened simultaneously. If no binding is indicated, then there is no need to invest further attention in any member of that family of compounds. If binding is indicated, then a particular family of promising compounds can be targeted for more detailed investigation. Simple one-dimensional homonuclear ¹H NMR experiments are sufficient to assess binding, with no requirement for isotopic labeling. Two-dimensional NMR experiments, either homonuclear or heteronuclear (with ¹⁵N and/or ¹³C isotopic labeling) could also be employed.

**Table 2. Data collection and refinement statistics**

| Data collection | | | | | | | | | |
|---|---|---|---|---|---|---|---|---|---|
| Crystal | λ (A) | Completeness (%) | | Rₛᵥₘ¹ (%) | Resolution (A) | | | | |
| IQN17 | 1.0000 | 89.5 | | 3.7 | 2.1 | | | | |
| IQN17/D10 | 1.1197 | 93.8 | | 4.8 | 1.5 | | | | |
| Os λ1 | 1.1403 | 98.6 | | 6.3 | 2.0 | | | | |
| Os λ2 | 1.1399 | 96.8 | | 9.7 | 2.0 | | | | |
| Os λ3 | 1.1393 | 96.9 | | 7.9 | 2.0 | | | | |
| Os λ4 | 1.1197 | 97.0 | | 8.4 | 2.0 | | | | |

| MAD phasing statistics (22.0-2.0 A) | | | | | | | | | |
|---|---|---|---|---|---|---|---|---|---|
| Derivative | Rᵢₛₒ² (%) | R_{cullis}³ Acentric | R_{cullis}³ Centric | R_{cullis}³ Anom. | Ph. Power⁴ Acentric | Ph. Power⁴ Centric | | Occ.⁵ | Anom. Occ.⁵ |
| Os λ1 vs. λ4 | 7.3 | 0.75 | 0.61 | 0.47 | 1.41 | 1.21 | | -0.039 | 0.337 |
| Os λ2 vs. λ4 | 5.2 | 0.83 | 0.71 | 0.44 | 1.04 | 1.15 | | -0.027 | 0.533 |
| Os λ3 vs. λ4 | 3.3 | 0.97 | 0.97 | 0.49 | 0.35 | 0.28 | | -0.005 | 0.295 |

| Overall figure of ment (before solvent flattening): 0.68 | | | | | | | | | |
|---|---|---|---|---|---|---|---|---|---|
| **Refinement statistics** | | | | | | | | | |
| | | | | | | | | | |

| Crystal | Non-hydrogen protein atoms | Waters | Ions | Resolution (Å) | Reflections total | R_{cryst}⁶ | R_{free}⁶ | R.m.s. bonds (°) | deviations (A) angles |
|---|---|---|---|---|---|---|---|---|---|
| IQN17/D10 | 516 | 150 | 1 | 10.0-1.5 | 13549 | 0.214 | 0.245 | 0.012 | 1.498 |
| IQN17 | 1143 | 160 | 1 | 5.0-2.5 | 7541 | 0.282 | 0.352 | 0.009 | 1.252 |

| | | | | | | | | | |
|---|---|---|---|---|---|---|---|---|---|
| ¹R_{sym} = ∑∑ⱼ∥ⱼ-<\|>\| ∑∑ⱼ\|<\|>\|, wnere \|ⱼ is the recorded intensity of the reflection j and <\|> is the mean recorded intensity over multiple recordings. ²Rᵢₛₒ = ∑∥F(λ1) = F(λ4)\| - \|F_{(λ1)}∥ / ∑\|F_{(λ4)}\|, where F_{(λ1)} is the structure factor at wavelength λ1 and F_{(λ4)} is the structure factor at the reference wavelength λ4. ³R_{cullis} = ∑∥F_{(λ)} ± F_{(λ4)}\|- \|F_{h(λ1),c}∥ /∑\|F_{(λ)} ± F_{(λ4)}\|, wnere F_{h(λ1),c} is the calculated heavy atom structure factor. ⁴Phase power = <F_{h(λ1)}> /E, where <Fh_{(λ1)}> is the root-mean-square heavy atom structure factor an E is the residual laCK of ciosure error. ⁵Occupancies are values output from MLPHARE. ⁶R_{cryst. tree} = ∑∥F_{obsl} - \|F_{calc∥}/ \|F_{obsl}, wnere the crystallographic and free R factors are calculated using the working and test sets, respectively. Test set contained 10% of reflections. | | | | | | | | | |

While this invention has been particularly shown and described with references to preferred embodiments thereof, it will be understood by those skilled in the art that various changes in form and details may be made therein without departing from the scope of the invention as defined by the appended claims.

## Claims

1. A composition comprising a soluble, non-aggregating trimeric peptide, the peptide comprising a soluble, trimeric form of a coiled coil and a sufficient portion of the N-helix coiled-coil of HIV gp41 to comprise the amino acid residues which form the hydrophobic pocket thereof, which peptide presents the hydrophobic pocket such that it is empty and available for binding by ligand.

2. The composition of claim 1 which is free of hydrophobic pocket ligand.

3. The composition of claim 1 or claim 2 wherein the peptide is a D-peptide.

4. The composition of claim 1 or claim 2 wherein the peptide is an L-peptide.

5. The composition of any one of the preceding claims wherein the coiled coil is selected from:
(a) the coiled coil of GCN4-pI_{Q}I;
(b) the coiled coil of GCN4-pII;
(c) the coiled coil of Moloney Murine Leukemia Virus; and
(d) the coiled coil of ABC heterotrimer.

6. The composition of any one of claims 1-4 wherein the amino acid sequence of the coiled coil is:
RMKQIEDKIEEIESKQKKIENEIARIKK (SEQ ID NO: 25).

7. The composition of any one of the preceding claims wherein the sufficient portion of the N-helix coiled-coil of HIV gp41 comprises the sequence:
LLQLTVWGIKQLQARIL (SEQ ID NO: 20).

8. The composition of claim 1 wherein the peptide is IQN17, wherein the amino acid sequence of IQN17 is:
ac-RMKQIEDKIEEIESKQKKIENEIARIKKLLQLTVWGIKQLQARIL-am (SEQ ID NO: 2), wherein ac- represents an N-terminal acetyl group and -am represents a C-terminal amide.

9. The composition of claim 1 wherein the peptide comprises: the amino acid sequence RMKQIEDKIEEIESKQKKIENEIARIKK (SEQ ID NO: 25) and a sequence which comprises 17 amino acid residues, wherein the 17 amino acid residues comprise the sequence: LLXLTVWGXKXLQXRXX (SEQ ID NO: 42), wherein L, T, V, W, G, K, Q and R are amino acid residues represented by the single letter amino acid code and X is any amino acid residue.

10. The composition of claim 9 wherein the sequence which comprises 17 amino acid residues is selected from the group consisting of:
LLQLTVWGIKQLQARIL (SEQ ID NO: 20);
LLRLTVWGTKNLQARVT (SEQ ID NO: 26);
LLRLTVWGTKNLQTRVT (SEQ ID NO: 27); and
LLXLTVWGXKXLQXRXX (SEQ ID NO: 42).

11. The composition of any one of the preceding claims in a pharmaceutically acceptable carrier.

12. A D-peptide which comprises at least four amino acid residues and comprises the consensus sequence WXWL, wherein W represents D-tryptophan, L represents D-leucine and X represents any moiety, which binds the pocket of the soluble non-aggregating trimeric peptide as defined in claim 1.

13. The D-peptide of claim 12 wherein X is a D-amino acid residue or a modified D-amino acid residue.

14. The D-peptide of claims 12 or 13, wherein the D-peptide comprises 4 to 21 amino acid residues.

15. A D-peptide of claim 12 which comprises at least five amino acid residues, wherein the at least five amino acid residues are EWXWL, wherein E represents D-glutamic acid, W represents D-tryptophan, L represents D-leucine and X represents an amino acid residue, a modified amino acid residue or a moiety other than an amino acid residue, which peptide binds the pocket of the soluble non-aggregating trimeric peptide as defined in claim 1.

16. The D-peptide of claim 12 or claim 15 which comprises an amino acid sequence selected from:
(a) CDLKAKEWFWLC (SEQ ID NO: 3);
(b) CEARHREWAWLC (SEQ ID NO: 4);
(c) CELLGWEWAWLC (SEQ ID NO: 5);
(d) CLLRAPEWGWLC (SEQ ID NO: 6);
(e) CSRSQPEWEWLC (SEQ ID NO: 7);
(f) CGLGQEEWFWLC (SEQ ID NO: 8);
(g) CMRGEWEWSWLC (SEQ ID NO: 9);
(h) CPPLNKEWAWLC (SEQ ID NO: 10);
(i) CVLKAKEWFWLC (SEQ ID NO: 11);
(j) KKGACGLGQEEWFWLC (SEQ ID NO: 15);
(k) KKGACELLGWEWAWLC (SEQ ID NO: 16);
(l) KKICKGACELLGWEWAWLC (SEQ ID NO: 17);
(m) KKGACMRGEWEWSWLC (SEQ ID NO: 18);
(n) KKGACPPLNKEWAWLC (SEQ ID NO: 19);
(o) a D-peptide comprising WXWL (SEQ ID NO: 23);
(p) a D-peptide comprising EWXWL (SEQ ID NO: 24);
(q) a D-peptide comprising CXXXXXEWXWLC (SEQ ID NO: 12)
(r) ac-GACEARHREWAWLCAA-am (SEQ ID NO: 34);
(s) ac-KKGACEARHREWAWLCAA-am (SEQ ID NO: 38);
(t) ac-KKKICGACEARHREWAWLCAA-am (SEQ ID NO: 43);
(u) ac-GACGLGQEEWFWLCAA-am (SEQ ID NO: 44);
(v) ac-KKGACGLGQEEWFWLCAA-am (SEQ ID NO: 15);
(w) ac-KKKICGACGLGQEEWFWLCAA-am (SEQ ID NO: 45)
(x) ac-GACDLKAKEWFWLCAA-am (SEQ ID NO: 35);
(y) ac-KKGACDLKAKEWFWLCAA-am (SEQ ID NO: 39);
(z) ac-KKKICGACDLKAKEWFWLCAA-am (SEQ ID NO: 46);
(a') ac-GACELLGWEWAWLCC-am (SEQ ID NO: 47);
(b') ac-KKGACELLGWEWAWLCAA-am (SEQ ID NO: 16);
(c') ac-KKKICGACELLGWEWAWLCAA-am (SEQ ID NO: 17);
(d') ac-GACSRSQPEWEWLCAA-am (SEQ ID NO: 36);
(e') ac-KKGACSRSQPEWEWLCAA-am (SEQ ID NO: 40);
(f') ac-KKKKGACSRSQPEWEWLCAA-am (SEQ ID NO: 48);
(g') ac-GACLLRAPEWGWLCAA-am (SEQ ID NO: 37);
(h') ac-KKGACLLRAPEWGWLCAA-am (SEQ ID NO: 41);
(i') ac-KKKKGACLLRAPEWGWLCAA-am (SEQ ID NO: 49);
(j') ac-GACMRGEWEWSWLCAA-am (SEQ ID NO: 50);
(k') ac-KKGACMRGEWEWSWLCAA-am (SEQ ID NO: 18);
(l' ac-KKKKGACMRGEWEWSWLCAA-am (SEQ ID NO: 51);
(m') ac-GACPPLNKEWAWLCAA-am (SEQ ID NO: 52);
(n') ac-KKGACPPLNKEWAWLCAA-am (SEQ ID NO: 19);
(o') ac-KKKICGACPPLNKEWAWLCAA-am (SEQ ID NO: 53);
(p') ac-GACX3CKYXEWXWLCAA-am (SEQ ID NO: 54);
(q') ac-KKGACXXXXXEWXWLCAA-am (SEQ ID NO: 55);
(r') ac-KKKKGACXXXXXEWXWLCAA-am (SEQ ID NO: 56);
(s') ac-XXCXXXXXEWXWLCXX-am (SEQ ID NO: 57);
(t') ac-KKXXCXXXXXEWXWLCXX-am (SEQ ID NO: 58);
(u') ac-KKKKXXCXXXXXEWXWLCXX-am (SEQ ID NO: 59);
(v') ac-XXCXXXXYEWXWLCXXX-am (SEQ ID NO: 60);
(w') ac-KKXXCXDCXXXEWXWLCXXX-am (SEQ ID NO: 61); and
(x') ac-KKKKXXCXXXXXEWXWLCXXX-am (SEQ ID NO: 62);
wherein ac- at the N-terminus and -am at the C- terminus are optional.

17. The composition of any one of claims 1 to 11 or peptide of any one of claims 12 to 16 for use in eliciting an immune response in an individual.

18. The composition or peptide of claim 17 wherein the immune response is elicited by introducing the peptide into the individual by a route of administration selected from the group consisting of: intramuscularly, intraperitoneally, orally, nasally and transdermally.

19. The peptide of any one of claims 12 to 16 and a carrier or base for use in a method of interfering with entry of HIV into a mucosal cell by administration or application to a mucosal surface.

20. The peptide of claim 19 wherein the carrier or base is selected from: a foam, a gel, other substance sufficiently viscous to retain the peptide, water and a buffer.

21. The peptide of claim 19 or claim 20 wherein the carrier or base is a vaginal suppository or rectal suppository.

22. The peptide of any one of claims 19 to 21 wherein the peptide is released from the carrier or base immediately or soon after it is administered or applied to the vagina, mouth or rectum.

23. The peptide of any one of claims 19 to 21 wherein the peptide is released from the carrier or base gradually or after a specified period after it is administered or applied to the vagina, mouth or rectum.

24. The peptide of claim 19 wherein the peptide is on the surface of or incorporated within a contraceptive device in a manner which permits release of the peptide under conditions of use.

25. The peptide of any one of claims 19 to 24 wherein the peptide prevents or reduces the gp41 conformational change, thereby interfering with entry of HIV into cells of the mucosal surface.

26. A method of identifying a drug which binds the N-helix coiled-coil cavity of HIV-1 gp41 envelope protein, comprising:
(a) combining: (1) the soluble peptide as defined in of any one of claims 1 to 10 that presents the N-helix coiled-coil cavity of HIV gp 41 in such a manner that it is available for binding by a drug and (2) a candidate drug, which is to be assessed for its ability to bind the N-helix coiled-coil cavity; and
(b) determining whether the candidate drug binds the HIV gp41 cavity of the soluble peptide, wherein if binding occurs, the candidate drug is a drug which binds the N-helix coiled-coil cavity of HIV gp41.

27. The method of claim 26 wherein the candidate inhibitor is detectably labeled and binding of the candidate drug to the HIV gp41 cavity is determined by detecting the presence of the detectable label on the HIV gp41 cavity.

28. The method of claim 26 or 27 wherein in (a), a peptide which binds the N-helix coiled-coil cavity of HIV gp41 is combined with the candidate inhibitor drug and the soluble peptide and in (b), whether the candidate drug binds the HIV gp41 cavity is determined in the presence of the peptide which binds the N-helix coiled-coil cavity of HIV gyp41.

29. The method of claim 28 comprising:
(a) combining a D-peptide which binds the N-helix coiled-coil cavity, the soluble peptide and a candidate inhibitor, under conditions appropriate for binding of the D-peptide to the N-helix coiled coil cavity, thereby producing a test sample;
(b) determining the extent to which binding of the D peptide to the N-helix coiled coil cavity in the test sample; and
(c) comparing the extent of binding determined in to the N-helix coiled-coil cavity in a control sample, wherein the control sample is the same as the test sample except that the control sample does not include the candidate inhibitor and is maintained under the same conditions appropriate for binding of the D-peptide to the N-helix coiled-coil cavity as is the test sample,
wherein if the extent of binding in the test sample is less than the extent of binding in the control sample, the candidate inhibitor is a compound or molecule which binds the N- helix coiled-coil cavity of HIV-1 gp41 envelope protein.

30. The method of claim 29 wherein the D-peptide is labeled with a fluorescent reporter and the soluble peptide is labeled with a quencher which, when in sufficiently close proximity to the fluorescent reporter, quenches the signal from the reporter and detection of a signal from the fluorescent reporter indicates that the candidate inhibitor is a compound or molecule which binds the N-helix coiled-coil cavity of HIV-1 gp41 envelope protein.

31. The method of claim 28 wherein the peptide which binds the N-helix coiled-coil cavity of HIV gp41 is selected from:
(a) CDLKAKEWFWLC (SEQ ID NO: 3);
(b) CEARHREWAWLC (SEQ ID NO: 4);
(c) CELLGWEWAWLC (SEQ ID NO: 5);
(d) CLLRAPEWGWLC (SEQ ID NO: 6);
(e) CSRSQPEWEWLC (SEQ ID NO: 7);
(f) CGLGQEEWFWLC (SEQ ID NO: 8);
(g) CMRGEWEWSWLC (SEQ ID NO: 9);
(h) CPPLNKEWAWLC (SEQ ID NO: 10);
(i) CVLKAKEWFWLC (SEQ ID NO: 11);
(j) KKGACGLGQEEWFWLC (SEQ ID NO: 15);
(k) KKGACELLGWEWAWLC (SEQ ID NO: 16);
(l) KKKICGACELLGWEWAWLC (SEQ ID NO: 17);
(m) KKGACMRGEWEWSWLC (SEQ ID NO: 18);
(n) KKGACPPLNKEWAWLC (SEQ ID NO: 19);
(o) a D-peptide comprising WXWL (SEQ ID NO: 23);
(p) a D-peptide comprising EWXWL (SEQ ID NO: 24);
(q) a D-peptide comprising CXXXXXEWXWLC (SEQ ID NO: 12)
(r) ac-GACEARHREWAWLCAA-am (SEQ ID NO: 34);
(r) ac-KKGACEARHREWAWLCAA-am (SEQ ID NO: 38);
(t) ac-KKKKGACEARHREWAWLCAA-am (SEQ ID NO: 43);
(u) ac-GACGLGQEEWFWLCAA-am (SEQ ID NO: 44);
(v) ac-KKGACGLGQEEWFWLCAA-am (SEQ ID NO: 15);
(w) ac-KKKKGACGLGQEEWFWLCAA-am (SEQ ID NO: 45)
(x) ac-GACDLKAKEWFWLCAA-am (SEQ ID NO: 35);
(y) ac-KKGACDLKAKEWFWLCAA-am (SEQ ID NO: 39);
(z) ac-KKKKGACDLKAKEWFWLCAA-am (SEQ ID NO: 46);
(a') ac-GACELLGWEWAWLCC-am (SEQ ID NO: 47);
(b') ac-KKGACELLGWEWAWLCAA-am (SEQ ID NO: 16);
(c') ac-KKKKGACELLGWEWAWLCAA-am (SEQ ID NO: 17);
(d') ac-GACSRSQPEWEWLCAA-am (SEQ ID NO: 36);
(e') ac-KKGACSRSQPEWEWLCAA-am (SEQ ID NO: 40);
(f) ac-KKKKGACSRSQPEWEWLCAA-am (SEQ ID NO: 48);
(g') ac-GACLLRAPEWGWLCAA-am (SEQ ID NO: 37);
(h') ac-KKGACLLRAPEWGWLCAA-am (SEQ ID NO: 41);
(i') ac-KKICKGACLLRAPEWGWLCAA-am (SEQ ID NO: 49);
(j') ac-GACMRGEWEWSWLCAA-am (SEQ ID NO: 50);
(k') ac-KKGACMRGEWEWSWLCAA-am (SEQ ID NO: 18);
(l') ac-KKKKGACMRGEWEWSWLCAA-am (SEQ ID NO: 51);
(m') ac-GACPPLNKEWAWLCAA-am (SEQ ID NO: 52);
(n') ac-KKGACPPLNKEWAWLCAA-am (SEQ ID NO: 19);
(o') ac-KKKKGACPPLNKEWAWLCAA-am (SEQ ID NO: 53);
(p') ac.GACXXXXXEWXWLCAA-am (SEQ ID NO: 54);
(q') ac-KKGACXXXXXEWXWLCAA-am (SEQ ID NO: 55);
(r') ac-KKKKGACXXXWEWXMILCAA-arn (SEQ ID NO: 56);
(s') ac-XXCXXXXXEWXWLCXX-am (SEQ ID NO: 57);
(t') ac-KKXXCXXXXXEWXWLCXX-am (SEQ ID NO: 58);
(u') ac-KKKKXXCXDCKXYEWXWLCXX-am (SEQ ID NO: 59);
(v') ac-XXCXXXXXEWXWLCXXX-am (SEQ ID NO: 60);
(w') ac-KKXXCXXXXXEWXWLCXXX-am (SEQ ID NO: 61); and
(x') ac-KKKKXXCXXXXXEWXWLCXXX-am (SEQ ID NO: 62);
wherein ac- at the N-terminus and -am at the C- terminus are optional.

32. A method of identifying a peptide that binds to the N-helix coiled-coil cavity of HIV gp41, comprising:
(a) combining the soluble D-peptide as defined in claim 3 with a phage display library of L-amino acid peptides, under conditions appropriate for binding of members of the library to the soluble peptide; and
(b) determining if binding occurs between the soluble peptide and a member or members of the phage display library, wherein if binding occurs, a peptide that binds to the N-helix coiled-coil cavity of HIV gp41 in the D-handedness is identified.

33. The method of claim 32 further comprising determining the amino acid sequence of the member or members of the phage display library which bind to the soluble peptide and producing peptides, in D form, comprising the amino acid sequences determined, wherein the peptides in D form bind the N-helix coiled-coil cavity in the natural L-handedness.

34. A method of identifying a molecule that binds to the N-helix coiled-coil cavity of HIV gyp41, comprising:
(a) combining the soluble D-peptide as defined in claim 3 with a biologically encoded library of ligands, under conditions appropriate for binding of members of the library to the peptide; and
(b) determining if binding occurs between the soluble peptide and a member or members of the biologically encoded library, wherein if binding occurs, a ligand that binds to the N-helix coiled-coil cavity of HIV gp41 is identified.

35. The method of claim 34 further comprising determining the sequence of the member or members of the biologically encoded library which bind to the soluble peptide, and producing ligands, in the mirror-image handedness of the biologically encoded ligands, comprising the sequences determined.

36. The method of claim 34 or claim 35 wherein the biologically encoded library is selected from the group consisting of a phage display library, a DNA library, an RNA library and a biologically encoded peptide library.

37. A process for producing the composition of any one of claims 1 to 10, comprising producing a fusion protein comprising: (a) a soluble, trimeric form of a coiled-coil and (b) a sufficient portion of the N-peptide region of HIV gp41 to comprise the amino acid residues which form the pocket of the N-helix coiled-coil of HIV gp41.

38. The process of claim 37 wherein the protein of (a) is GCN4-pI_{Q}I, GCN4-pII, Moloney Murine Leukemia Virus or ABC heterotrimer and the sufficient portion of (b) is selected from the group consisting of: a portion comprising the amino acid sequence LLQLTVWGIKQLQARIL (SEQ ID NO: 20); a portion comprising the amino acid sequence LLRLTVWGTKNLQARVT (SEQ ID NO: 26); a portion comprising the amino acid sequence LLRLTVWGTKNLQTRVT (SEQ ID NO: 27); and a portion comprising the amino acid sequence LLXLTVWGXKXLQXRXX (SEQ ID NO: 42).

39. The process of claim 37 or 38 wherein the fusion protein is IQN17, wherein the amino acid sequence of IQN17 is:
ac-RMKQIEDKIEEIESKQKKIENEIARIKKLLQLTVWGIKQLQARIL-am (SEQ ID NO: 2), wherein ac- represents an N-terminal acetyl group and -am represents a C-terminal amide.

40. A process for producing a drug which fits the N-helix coiled-coil pocket of HIV gyp41, comprising:
(a) obtaining a crystal of the soluble peptide as defined in any one of claims 1 to 10;
(b) obtaining the atomic coordinates of the soluble peptide by X-ray diffraction studies using the crystal obtained in (a);
(c) using the atomic coordinates obtained in (b) to define the N-helix coiled-coil pocket of HIV gp41;
(c) identifying a molecule or compound which fits the N-helix coiled-coil pocket of HIV gp41;
(d) obtaining the molecule or compound identified in (d); and
(e) contacting the molecule or compound obtained in (e) with the N-helix coiled-coil pocket of HIV gp41 to assess the ability of the molecule or compound to fit the pocket of HIV gyp41,
wherein if the molecule or compound fits the N-helix coiled-coil pocket of HIV gyp41, the molecule or compound is a drug which fits the pocket, whereby a drug which fits the N- helix coiled-coil pocket of HIV gp41 is produced.

41. The process of claim 40 wherein (f), the molecule or compound is contacted with the N-helix coiled-coil pocket of HIV gp41 by contacting the molecule or compound with IQN17, the N-helix of HIV gp41 or a polypeptide which comprises the HIV pocket.

42. The process of claim 40 or claim 41 wherein the soluble peptide is IQN17.

43. The process of any one of claims 40-42 wherein the crystal obtained in (a) is a crystal of IQN17 of space group C222.

44. A process for producing a drug which binds the N-helix coiled-coil pocket of HIV gyp41, comprising:
(a) obtaining the atomic coordinates of IQN17;
(b) using the atomic coordinates obtained in (a) to define the N-helix coiled-coil pocket of HIV gyp41;
(c) identifying a molecule or compound which fits the N-helix coiled-coil pocket of HIV gp41;
(d) obtaining the molecule or compound identified in (c); and
(e) contacting the molecule or compound obtained in (d) with the N-helix coiled-coil pocket of HIV gp41 to assess the ability of the molecule or compound to fit the pocket of HIV gp41,
wherein if the molecule or compound fits the N-helix coiled-coil pocket of HIV gp41, the molecule or compound is a drug which fits the pocket, whereby a drug which fits the N- helix coiled-coil pocket of HIV gp41 is produced.

45. The process of claim 44 wherein the atomic coordinates are the atomic coordinates in the PDB file represented in Figures 11A-11V.

46. A vaccine comprising the peptide as defined in any one of claims 1 to 10.

47. The vaccine of claim 46 for eliciting an immune response that protects partially or totally against HIV infection and/or disease.

## Patentansprüche

1. Gemisch, dieses aufweisend ein lösbares, nicht-aggegierbares trimeres Peptid, wobei das Peptid eine lösbare, trimere Form einer Doppelwindung und einen ausreichenden Anteil an der N-Helix Doppelwindung von HIV gp41 aufweist, um diejenigen Aminosäurereste zu umfassen, welche die hydrophobe Tasche davon formen, wobei das Peptid die hydrophobe Tasche derart bereitstellt, dass diese leer und verfügbar für die Bindung eines Liganden ist.

2. Gemisch nach Anspruch 1, welches frei von einem hydrophobe-Tasche-Liganden ist.

3. Gemisch nach Anspruch 1 oder Anspruch 2, wobei das Peptid ein D-Peptid ist.

4. Gemisch nach Anspruch 1 oder Anspruch 2, wobei das Peptid ein L-Peptid ist.

5. Gemisch nach einem der vorhergehenden Ansprüche, wobei die Doppelwindung selektiert ist aus:
(a) der Doppelwindung von GCN4-pI□I;
(b) der Doppelwindung von GCN4-pII;
(c) der Doppelwindung des Murinen Leukemievirus; und
(d) der Doppelwindung eines ABC-Heterotrimers.

6. Gemisch nach einem der Ansprüche 1 - 4, wobei die Aminosäuresequenz der Doppelwindung ist:
RMKQIEDKIEEIESKQKKIENEIARIKK (SEQ ID Nr. : 25).

7. Gemisch nach einem der vorhergehenden Ansprüchen, wobei der ausreichende Anteil an der N-Helix Doppelwindung des HIV gp41 die Sequenz aufweist:
LLQLTVWGIKQLQARIL (SEQ ID Nr. : 20).

8. Gemisch nach Anspruch 1, wobei das Peptid IQN17 ist, wobei die Aminosäuresequenz des IQN17 ist:
ac-RMKQIEDKIEEIESKQKKIENEIARIKKLLQLTVWGIKQLQARIL-am (SEQ ID Nr. : 2), wobei ac- eine N-terminale Acetyl-Gruppe repräsentiert und -am ein C-terminales Amid repräsentiert.

9. Gemisch nach Anspruch 1, wobei das Peptid aufweist: die Aminosäuresequenz RMKQIEDKIEEIESKQKKIENEIARIKK (SEQ ID Nr. : 25) und eine Sequenz, welche 17 Aminosäurereste umfasst, wobei die 17 Aminosäurereste die Sequenz LLXLTVWGXKXLQXRXX (SEQ ID Nr.: 42) umfassen, wobei L, T, V, W, G, K, Q und R Aminosäurereste sind, die durch den Einbuchstabencode repräsentiert werden, und X ein beliebiger Aminosäurerest ist.

10. Gemisch nach Anspruch 9, wobei die Sequenz, welche 17 Aminosäurereste umfasst, aus der Gruppe selektiert ist, bestehend aus:
LLQLTVWGIKQLQARIL (SEQ ID Nr. : 20);
LLRLTVWGTKNLQARVT (SEQ ID Nr. : 26);
LLRLTVWGTKNLQTRVT (SEQ ID Nr. : 27); und
LLXLTVWGXKXLQXRXX (SEQ ID Nr. : 42).

11. Gemisch nach einem der vorhergehenden Ansprüche in einem pharmazeutisch annehmbaren Träger.

12. D-Peptid, welches mindestens vier Aminosäurereste aufweist und welches die Konsenssequenz WXWL umfasst, wobei W D-Tryptophan repräsentiert, L D-Leucin repräsentiert und X einen beliebigen Teil repräsentiert, welcher die Tasche des lösbaren, nicht-aggegierbaren trimeren Peptids nach Anspruch 1 bindet.

13. D-Peptid nach Anspruch 12, wobei X ein D-Aminosäurerest oder ein modifizierter D-Aminosäurerest ist.

14. D-Peptid nach Anspruch 12 oder 13, wobei das D-Peptid 4 bis 21 Aminosäurereste aufweist.

15. D-Peptid nach Anspruch 12, welches mindestens fünf Aminosäurereste aufweist, wobei die mindestens fünf Aminosäurereste EWXWL sind, wobei E D-Glutaminsäure repräsentiert, W D-Tryptophan repräsentiert, L D-Leucin repräsentiert und X einen Aminosäurenrest, einen modifizierten Aminosäurerest oder einen anders als ein Aminosäurerest gearteter Teil repräsentiert, wobei das Peptid die Tasche des lösbaren, nicht-aggegierbaren trimeren Peptids nach Anspruch 1 bindet.

16. D-Peptid nach Anspruch 12 oder Anspruch 15, welches eine Aminosäuresequenz aufweist, selektiert aus:
(a) CDLKAKEWFWLC (SEQ ID Nr. : 3);
(b) CEARHREWAWLC (SEQ ID Nr. : 4);
(c) CELLGWEWAWLC (SEQ ID Nr. : 5);
(d) CLLRAPEWGWLC (SEQ ID Nr. : 6) ;
(e) CSRSQPEWEWLC (SEQ ID Nr. : 7);
(f) CGLGQEEWFWLC (SEQ ID Nr. : 8);
(g) CMRGEWEWSWLC (SEQ ID Nr. : 9);
(h) CPPLNKEWAWLC (SEQ ID Nr. : 10) ;
(i) CVLKAKEWFWLC (SEQ ID Nr. : 11);
(j) KKGACGLGQEEWFWLC (SEQ ID Nr. : 15);
(k) KKGACELLGWEWAWLC (SEQ ID Nr. : 16) ;
(l) KKKKGACELLGWEWAWLC (SEQ ID Nr. : 17);
(m) KKGACMRGEWEWSWLC (SEQ ID Nr. : 18);
(n) KKGACPPLNKEWAWLC (SEQ ID Nr. : 19);
(o) ein D-Peptid aufweisend WXWL (SEQ ID Nr. : 23);
(p) ein D-Peptid aufweisend EWXWL (SEQ ID Nr. : 24);
(q) ein D-Peptid aufweisend CXXXXXEWXWL (SEQ ID Nr. : 12)
(r) ac-GACEARHREWAWLCAA-am (SEQ ID Nr. : 34);
(s) ac-KKGACEARHREWAWLCAA-am (SEQ ID Nr. : 38);
(t) ac-KKKKGACEARHREWAWLCAA-am (SEQ ID Nr. : 43);
(u) ac-GACGLGQEEWFWLCAA-am (SEQ ID Nr. : 44)
(v) ac-KKGACGLGQEEWFWLCAA-am (SEQ ID Nr. :15);
(w) ac-KKKKGACGLGQEEWFWLCAA-am (SEQ ID NO : 45)
(x) ac-GACDLKAKEWFWLCAA-am (SEQ ID Nr. : 35);
(y) ac-KKGACDLKAKEWFWLCAA-am (SEQ ID Nr. : 39);
(z) ac-KKKKGACDLKAKEWFWLCAA-am (SEQ ID Nr. : 46) ;
(a') ac-GACELLGWEWAWLCC-am (SEQ ID Nr. : 47);
(b') ac-KKGACELLGWEWAWLCAA-am (SEQ ID Nr. : 16) ;
(c') ac-KKKKGACELLGWEWAWLCAA-am (SEQ ID Nr. : 17);
(d') ac-GACSRSQPEWEWLCAA-am (SEQ ID Nr. : 36) ;
(e') ac-KKGACSRSQPEWEWLCAA-am (SEQ ID Nr. : 40) ;
(f) ac-KKKKGACSRSQPEWEWLCAA-am (SEQ ID Nr. : 48);
(g') ac-GACLLRAPEWGWLCAA-am (SEQ ID Nr. : 37);
(h') ac-KKGACLLRAPEWGWLCAA-am (SEQ ID Nr. : 41);
(i') ac-KKKKGACLLRAPEWGWLCAA-am (SEQ ID Nr. : 49);
(j') ac-GACMRGEWEWSWLCAA-am (SEQ ID Nr. : 50) ;
(k') ac-KKGACMRGEWEWSWLCAA-am (SEQ ID Nr. : 18);
(l') ac-KKKKGACMRGEWEWSWLCAA-am (SEQ ID Nr. : 51);
(m') ac-GACPPLNKEWAWLCAA-am (SEQ ID Nr. : 52) ;
(n') ac-KKGACPPLNKEWAWLCAA-am (SEQ ID Nr. : 19);
(o') ac-KKKKGACPPLNKEWAWLCAA-am (SEQ ID Nr. : 53);
(p') ac-GACXXXXXEWXWLCAA-am (SEQ ID Nr. : 54);
(q') ac-KKGACXXXXXEWXWLCAA-am (SEQ ID Nr. : 55);
(r') ac-KKKKGACXXXXXEWXWLCAA-am (SEQ ID Nr. : 56) ;
(s') ac-XXCXXXXXEWXWLCXX-am (SEQ ID Nr. : 57);
(t') ac-KKXXCXXEYXEWXWLCXX-am (SEQ ID Nr. : 58);
(u') ac-KKKKXXCXXXXXEWXWLCXX-am (SEQ ID Nr. : 59);
(v') ac-XXCXXXXXEWXWLCXXX-am (SEQ ID Nr. : 60) ;
(w') ac-KKXXCXXXXXEWXWLCXXX-am (SEQ ID Nr. : 61); und
(x') ac-KKKKXXCXXXXXEWXWLCXXX-am (SEQ ID Nr. : 62);
wobei ac- an dem N-Terminus und -am an dem C-Terminus optional sind.

17. Gemisch nach einem der Ansprüche 1 bis 11 oder Peptid nach einem der Ansprüche 12 bis 16 zur Verwendung zum Auslösen einer Immunreaktion in einem Individuum.

18. Gemisch oder Peptid nach Anspruch 17, wobei die Immunreaktion ausgelöst wird durch das Einführen des Peptids in das Individuum mittels eines Verabreichungsweges selektiert aus der Gruppe bestehend aus: intramuskulär, intraperitoneal, oral, nasal und transdermal.

19. Peptid nach einem Ansprüche 12 bis 16 und ein Träger oder eine Basis zur Verwendung in einem Verfahren zur Störung des Eindringens von HIV in eine mukosale Zelle durch Verabreichung an oder Anwendung auf eine mukosale Oberfläche.

20. Peptid nach Anspruch 19, wobei der Träger oder die Basis selektiert ist aus: einem Schaum, einem Gel, einer anderen ausreichend viskosen Substanz um das Peptid aufzunehmen, Wasser und einem Puffer.

21. Peptid nach Anspruch 19 oder Anspruch 20, wobei der Träger oder die Basis ein vaginales Zäpfchen oder ein rektales Zäpfchen ist.

22. Peptid nach einem der Ansprüche 19 bis 21, wobei das Peptid vom Träger oder der Basis sofort oder sogleich abgelöst wird, nachdem es der Vagina, dem Mund oder dem Rektum verabreicht oder darauf angewendet ist.

23. Peptid nach einem der Ansprüche 19 bis 21, wobei das Peptid vom Träger oder der Basis mit der Zeit oder nach einer vorgegebenen Periode abgelöst wird, nachdem es der Vagina, dem Mund oder dem Rektum verabreicht oder darauf angewendet ist.

24. Peptid nach Anspruch 19, wobei das Peptid an der Oberfläche oder eingebettet in eine empfängnisverhütende Einheit ist in einer Art, die das Auslösen des Peptids unter den gegebenen Einsatzbedingungen erlaubt.

25. Peptid nach einem der Ansprüche 19 bis 24, wobei das Peptid den konformativen Wandel des gp41 verhindert oder reduziert, wodurch der Eintritt des HIV in die Zellen der mukosalen Oberfläche gestört ist.

26. Verfahren zur Identifikation eines Arzneimittels, welches die N-Helix Doppelwindungskavität des HIV-1 gp41 Hüllproteins bindet, dieses aufweisend:
(a) Kombinieren: (1) des lösbaren Peptids nach einem der Ansprüche 1 bis 10, welches die N-Helix Doppelwindungskavität von HIV gp41 in einer Art präsentiert, dass dieses zur Bindung durch ein Arzneimittel bereitsteht, und (2) eines Kandidatenarzneimittels, dessen Eignung eine N-Helix Doppelwindindung zu binden geschätzt werden kann; und
(b) Ermitteln, ob das Kandidatenarzneimittel die HIV gp41 Kavität des lösbaren Peptids bindet, wobei, falls eine Bindung auftritt, das Kandidatenarzneimittel ein Arzneimittel ist, das die N-Helix Doppelwindungskavität des HIV gp41 bindet.

27. Verfahren nach Anspruch 26, wobei der Kandidatenhemmstoff erkennbar markiert ist und die Bindung des Kandidatenarzneimittels an die HIV gp41 Kavität ermittelt wird durch das Erkennen der Präsenz der erkennbaren Markierung auf der HIV gp41 Kavität.

28. Verfahren nach Anspruch 26 oder 27, wobei in (a) ein Peptid, welches die N-Helix Doppelbindungskavität on HIV gp41 bindet, kombiniert ist mit dem Kandidatenhemmstoffarzneimittel und dem lösbaren Peptid, und wobei in (b) ermittelt wird, ob das Kandidatenarzneimittel die HIV gp41 Kavität bindet in Anwesenheit des Peptids, welches die N-Helix Doppelwindungskavität von HIV gp41 bindet.

29. Verfahren nach Anspruch 28, dieses aufweisend:
(a) Kombinieren eines D-Peptids, welches die N-Helix Doppelwindungskavität bindet, des lösbaren Peptids und eines Kandidatenhemmstoffs, unter angemessenen Bedingungen zur Bindung des D-Peptids an die N-Helix Doppelwindungskavität, um dabei eine Testprobe zu produzieren;
(b) Ermitteln des Ausmaßes der Bindung des D-Peptids an die N-Helix Doppelwindungskavität in der Testprobe; und
(c) Vergleichen des Ausmaßes der Bindung der N-Helix Doppelwindungskavität mit einer Kontrollprobe, wobei die Kontrollprobe die gleiche wie die Testprobe ist, außer dass die Kontrollprobe nicht den Kandidatenhemmstoff einschließt, und wobei sie unter den gleichen angemessenen Bedingungen gehalten wurde zur Bindung des D-Peptids an die N-Helix Doppelwindungskavität wie die Testprobe,
wobei, falls das Ausmaß der Bindung in der Testprobe geringer ist als das Ausmaß der Bindung in der Kontrollprobe, der Kandidatenhemmstoff eine Mischung oder ein Molekül ist, welche die N-Helix Doppelwindungskavität des HIV-1 gp41 Hüllproteins binden.

30. Verfahren nach Anspruch 29, wobei das D-Peptid mit einem fluoreszierenden Reporter markiert ist und das lösbare Peptid mit einem Löscher markiert ist, welcher, sofern er in ausreichend naher Nachbarschaft mit dem fluoreszierenden Reporter, das Signal von dem Reporter auslöscht, und wobei die Detektion eines Signals von dem fluoreszierenden Reporter indiziert, dass der Kandidatenhemmstoff eine Mischung oder ein Molekül ist, welche die N-Helix Doppelwindungskavität des HIV-1 gp41 Hüllproteins binden.

31. Verfahren nach Anspruch 28, wobei das Peptid, welches die N-Helix Doppelwindungskavität von HIV gp41 bindet, selektiert ist aus:
(a) CDLKAKEWFWLC (SEQ ID Nr. : 3);
(b) CEARHREWAWLC (SEQ ID Nr. : 4);
(c) CELLGWEWAWLC (SEQ ID Nr. : 5);
(d) CLLRAPEWGWLC (SEQ ID Nr. : 6) ;
(e) CSRSQPEWEWLC (SEQ ID Nr. : 7);
(f) CGLGQEEWFWLC (SEQ ID Nr. : 8);
(g) CMRGEWEWSWLC (SEQ ID Nr. : 9);
(h) CPPLNKEWAWLC (SEQ ID Nr. : 10) ;
(i) CVLKAKEWFWLC (SEQ ID Nr. : 11);
(j) KKGACGLGQEEWFWLC (SEQ ID Nr. : 15);
(k) KKGACELLGWEWAWLC (SEQ ID Nr. : 16);
(l) KKKKGACELLGWEWAWLC (SEQ ID Nr. : 17);
(m) KKGACMRGEWEWSWLC (SEQ ID Nr. : 18);
(n) KKGACPPLNKEWAWLC (SEQ ID Nr. : 19) ;
(o) ein D-Peptid aufweisend WXWL (SEQ ID Nr. : 23);
(p) ein D-Peptid aufweisend EWXWL (SEQ ID Nr. : 24);
(q) ein D-Peptid aufweisend CXXXXXEWXWL (SEQ ID Nr. : 12)
(r) ac-GACEARHREWAWLCAA-am (SEQ ID Nr. : 34);
(s) ac-KKGACEARHREWAWLCAA-am (SEQ ID Nr. : 38) ;
(t) ac-KKKKGACEARHREWAWLCAA-am (SEQ ID Nr.: 43);
(u) ac-GACGLGQEEWFWLCAA-am (SEQ ID Nr. : 44);
(v) ac-KKGACGLGQEEWFWLCAA-am (SEQ ID Nr. : 15);
(w) ac-KKKKGACGLGQEEWFWLCAA-am (SEQ ID Nr. : 45)
(x) ac-GACDLKAKEWFWLCAA-am (SEQ ID Nr. : 35);
(y) ac-KKGACDLKAKEWFWLCAA-am (SEQ ID Nr. : 39);
(z) ac-KKKKGACDLKAKEWFWLCAA-am (SEQ ID Nr. : 46) ;
(a') ac-GACELLGWEWAWLCC-am (SEQ ID Nr. : 47);
(b') ac-KKGACELLGWEWAWLCAA-am (SEQ ID Nr. : 16) ;
(c') ac-KKKKGACELLGWEWAWLCAA-am (SEQ ID Nr. : 17);
(d') ac-GACSRSQPEWEWLCAA-am (SEQ ID Nr. : 36) ;
(e') ac-KKGACSRSQPEWEWLCAA-am (SEQ ID Nr. : 40) ;
(f) ac-KKKKGACSRSQPEWEWLCAA-am (SEQ ID Nr.: 48);
(g') ac-GACLLRAPEWGWLCAA-am (SEQ ID Nr. : 37);
(h') ac-KKGACLLRAPEWGWLCAA-am (SEQ ID Nr. : 41);
(i') ac-KKKKGACLLRAPEWGWLCAA-am (SEQ ID Nr. : 49);
(j') ac-GACMRGEWEWSWLCAA-am (SEQ ID Nr. : 50) ;
(k') ac-KKGACMRGEWEWSWLCAA-am (SEQ ID Nr. : 18);
(l') ac-KKKKGACMRGEWEWSWLCAA-am (SEQ ID Nr. : 51);
(m') ac-GACPPLNKEWAWLCAA-am (SEQ ID Nr. : 52) ;
(n') ac-KKGACPPLNKEWAWLCAA-am (SEQ ID Nr. : 19);
(o') ac-KKKKGACPPLNKEWAWLCAA-am (SEQ ID Nr. : 53);
(p') ac-GACXXXXXEWXWLCAA-am (SEQ ID Nr. : 54);
(q') ac-KKGACXXXXXEWXWLCAA-am (SEQ ID Nr. : 55);
(r') ac-KKKKGACXXXXXEWXWLCAA-am (SEQ ID Nr. : 56) ;
(s') ac-XYCXXXXXEWXWLCXX-am (SEQ ID Nr. : 57);
(t') ac-KKXXCXXXXXEWXWLCXX-am (SEQ ID Nr. : 58);
(u') ac-KKKKXXCXXXXXYEWXWLCXX-am (SEQ ID Nr. : 59);
(v') ac-XXCXXXXXEWXWLCXXX-am (SEQ ID Nr. : 60);
(w') ac-KKXXCXXXXXEWXWLCXXX-am (SEQ ID Nr. : 61); und
(x') ac-KKKKXXCXXXXXEWXWLCXXX-am (SEQ ID Nr. : 62) ;
wobei ac- an dem N-Terminus und -am an dem C-Terminus optional sind.

32. Verfahren ein Peptid zu identifizieren, welches an die N-Helix Doppelbindungskavität von HIV gp41 bindet, dieses aufweisend:
(a) Kombinieren des lösbaren D-Peptides nach Anspruch 3 mit einer Phagen-Display Bibliothek von L-Aminosäurepeptiden, unter angemessenen Bedingungen zur Bindung der Mitglieder der Bibliothek an das lösbare Peptid; und
(b) Ermitteln ob eine Bindung zwischen dem lösbaren Peptid und einem Mitglied oder Mitgliedern der Phagen-Display Bibliothek auftritt, wobei, falls Bindung auftritt, ein Peptid identifiziert ist, welches an die N-Helix Doppelwindungskavität von HIV gp41 in der D-Händigkeit bindet.

33. Verfahren nach Anspruch 32, des Weiteren aufweisend die Ermittlung der Aminosäuresequenz des Mitglieds oder der Mitglieder der Phagen-Display Bibliothek, welche an das lösbare Peptid binden, und Produzieren eines Peptids in D Form, dieses aufweisend die ermittelte Aminosäuresequenz, wobei die Peptide in D Form die N-Helix Doppelwindungskavität in der natürlichen L-Händigkeit binden.

34. Verfahren ein Molekül zu identifizieren, welches an die N-Helix Doppelbindungskavität von HIV gp41 bindet, dieses aufweisend:
(a) Kombinieren des lösbaren D-Peptides nach Anspruch 3 mit einer biologisch enkodierten Bibliothek an Liganden, unter angemessenen Bedingungen zur Bindung der Mitglieder der Bibliothek an das Peptid; und
(b) Ermitteln ob eine Bindung zwischen dem lösbaren Peptid und einem Mitglied oder Mitgliedern der biologisch enkodierten Bibliothek auftritt, wobei, falls Bindung auftritt, ein Ligand identifiziert ist, welcher an die N-Helix Doppelwindungskavität von HIV gp41 bindet.

35. Verfahren nach Anspruch 34, des Weiteren aufweisend die Ermittlung der Sequenz des Mitglieds oder der Mitglieder der biologisch enkodierten Bibliothek, welche an das lösbare Peptid binden, und das Produzieren eines Liganden in spiegelbildlicher Händigkeit zu den biologisch enkodierten Liganden, welche die ermittelte Sequenzen aufweisen.

36. Verfahren nach Anspruch 34 oder Anspruch 35, wobei die biologisch enkodierte Bibliothek selektiert ist aus der Gruppe bestehend aus einer Phagen-Display Bibliothek, einer DNS Bibliothek, einer RNS Bibliothek und einer biologisch enkodierten Peptid Bibliothek.

37. Prozess zur Herstellung des Gemischs nach einem der Ansprüche 1 bis 10, aufweisend die Produktion eines Fusionproteins, dieses aufweisend:
(a) eine lösbare, trimere Form einer Doppelwindung und (b) einen ausreichenden Anteil an der N-Peptid-Region von HIV gp41, um einen Aminosäurerest zu umfassen, welcher die Tasche der N-Helix Doppelwindung von HIV gp41 formt.

38. Prozess nach Anspruch 37, wobei das Protein gemäß (a) GCN4-pI□I, GCN4-pII, Murinen Leukemievirus oder ABC Heterotrimer ist, und der ausreichende Anteil gemäß (b) ausgewählt ist aus der Gruppe bestehend aus: einem Anteil umfassend die Aminosäuresequenz LLQLTVWGIKQLQARIL (SEQ ID Nr. : 20); einem Anteil umfassend die Aminosäuresequenz LLRLTVWGTKNLQARVT (SEQ ID Nr. : 26); einem Anteil umfassend die Aminosäuresequenz LLRLTVWGTKNLQTRVT (SEQ ID Nr. : 27); und einem Anteil umfassend die Aminosäuresequenz LLXLTVWGXKXLQXRXX (SEQ ID Nr. : 42).

39. Prozess nach Anspruch 37 oder 38, wobei das Fusionsprotein IQN17 ist, wobei die Aminosäuresequenz von IQN17 ist:
ac-RMKQIEDKIEEIESKQKKIENEIARIKKLLQLTVWGIKQLQARIL-am (SEQ ID Nr. : 2), wobei ac- eine N-terminale Acetyl-Gruppe repräsentiert und -am ein C-terminales Amid repräsentiert.

40. Prozess zur Herstellung eines Arzneimittels, das in die N-Helix Doppelwindungstasche von HIV gp41 passt, dieser aufweisend:
(a) Erwirken eines Kristalls des lösbaren Peptids nach einem der Ansprüche 1 bis 10;
(b) Ermitteln der Atomkoordinaten des lösbaren Peptids mittels Röntgendiffraktionsuntersuchungen unter Verwendung des gemäß (a) erwirkten Kristalls;
(c) Verwenden der in (b) ermittelten Atomkoordinaten um die N-Helix Doppelwindungstasche von HIV gp41 zu definieren;
(d) Identifizieren eines Moleküls oder einer Mischung, welche in die N-Helix Doppelwindungstasche von HIV gp41 passen;
(e) Erwirken des Moleküls oder der Mischung wie sie in (d) identifiziert wurden; und
(f) Inkontaktbringen des Moleküls oder der Mischung, wie sie in (e) erwirkt wurden, mit der N-Helix Doppelwindungstasche von HIV gp41, um die Eignung des Moleküls oder der Mischung in die N-Helix Doppelwindungstasche von HIV gp41 zu passen zu schätzen,
wobei, falls das Molekül oder die Mischung in die N-Helix Doppelwindungstasche von HIV pg41 passt, das Molekül oder die Mischung ein Arzneimittel ist, welches in die Tasche passt, wodurch ein Arzneimittel hergestellt ist, welches in die N-Helix Doppelwindungstasche von HIV gp41 passt.

41. Prozess nach Anspruch 40 wobei in (f) das Molekül oder die Mischung mit der N-Helix Doppelwindungstasche von HIV gp41 in Kontakt gebracht ist durch das Inkontaktbringen des Moleküls oder des Gemischs mit IQN17, der N-Helix von HIV gp41 oder einem Polypeptid, welches die HIV Tasche aufweist.

42. Prozess nach Anspruch 40 oder Anspruch 41, wobei das lösbare Peptid IQN17 ist.

43. Prozess nach einem der Ansprüche 40-42, wobei der in (a) erwirkte Kristall ein Kristall aus IQN17 aus der Raumgruppe C222 ist.

44. Prozess zur Herstellung eines Arzneimittels, welches die N-Helix Doppelwindungstasche von HIV gp41 bindet, dieser aufweisend:
(a) Ermitteln der Atomkoordinaten von IQN17;
(b) Verwenden der in (a) ermittelten Atomkoordinaten um die N-Helix Doppelwindungstasche von HIV gp41 zu definieren;
(c) Identifizieren eines Moleküls oder einer Mischung, welche in die N-Helix Doppelwindungstasche von HIV gp41 passt;
(d) Erwirken des Moleküls oder der Mischung wie sie in (c) identifiziert wurden; und
(e) Inkontaktbringen des Moleküls oder der Mischung, wie sie in (d) erwirkt wurden, mit der N-Helix Doppelwindungstasche von HIV gp41, um die Eignung des Moleküls oder der Mischung in die N-Helix Doppelwindungstasche von HIV gp41 zu passen zu schätzen,
wobei, falls das Molekül oder die Mischung in die N-Helix Doppelwindungstasche von HIV pg41 passt, das Molekül oder die Mischung ein Arzneimittel ist, welches in die Tasche passt, wodurch ein Arzneimittel hergestellt ist, welches in die N-Helix Doppelwindungstasche von HIV gp41 passt.

45. Prozess nach Anspruch 44, wobei die Atomkoordinaten die Atomkoordinaten in der PDB-Datei sind, dargestellt in Figuren 11A - 11V.

46. Impfstoff aufweisend das Peptid nach einem der Ansprüche 1 bis 10.

47. Impfstoff nach Anspruch 46 zum Hervorrufen einer Immunreaktion, welche teilweise oder ganz vor einer HIV Infektion und/oder Erkrankung schützt.

## Revendications

1. Composition comprenant un peptide trimère soluble ne s'agrégeant pas, le peptide comprenant une forme trimère soluble d'une superhélice et une partie suffisante de l'hélice N-terminale de la superhélice de la gp41 exprimée par le VIH pour comprendre les résidus d'acides aminés qui forment la poche hydrophobe de celle-ci, ledit peptide présentant la poche hydrophobe de sorte qu'elle soit vide et disponible pour se lier à un ligand.

2. Composition selon la revendication 1, qui est exempte du système ligand - poche hydrophobe.

3. Composition selon la revendication 1 ou la revendication 2, dans laquelle le peptide est un peptide D.

4. Composition selon la revendication 1 ou la revendication 2, dans laquelle le peptide est un peptide L.

5. Composition selon l'une quelconque des revendications précédentes, dans laquelle la superhélice est choisie parmi :
(a) la superhélice de la GCN4-pI_{Q}I ;
(b) la superhélice de la GCN4-pII ;
(c) la superhélice du virus de la leucémie murine de Moloney ; et
(d) la superhélice de l'hétérotrimère ABC.

6. Composition selon l'une quelconque des revendications 1 à 4, dans laquelle la séquence d'acides aminés de la superhélice est :
RMKQIEDKIEEIESKQKKIENEIARIKK (SEQ ID No. : 25).

7. Composition selon l'une quelconque des revendications précédentes, dans laquelle la partie suffisante de l'hélice N-terminale de la superhélice de la gp41 exprimée par le VIH comprend la séquence :
LLQLTVWGIKQLQARIL (SEQ ID No. : 20).

8. Composition selon la revendication 1, dans laquelle le peptide est l'IQN17, dans laquelle la séquence d'acides aminés de l'IQN17 est :
ac-RMKQIEDKIEEIESKQKKIENEIARIKKLLQLTVWGIKQLQARIL-am (SEQ ID No. : 2), où ac- représente un groupe acétyle N-terminal et -am représente un amide C-terminal.

9. Composition selon la revendication 1, dans laquelle le peptide comprend : la séquence d'acides aminés RMKQIEDKIEEIESKQKKIENEIARIKK (SEQ ID No. : 25) et une séquence qui comprend 17 résidus d'acides aminés, dans laquelle les 17 résidus d'acides aminés comprennent la séquence : LLXLTVWGXKXLQXRXX (SEQ ID No. : 42), où L, T, V, W, G, K, Q et R sont des résidus d'acides aminés représentés par le code pour les acides aminés à une seule lettre et X représente un résidu acide aminé quelconque.

10. Composition selon la revendication 9, dans laquelle la séquence qui comprend les 17 résidus d'acides aminés est choisie dans le groupe constitué de :
LLQLTVWGIKQLQARIL (SEQ ID No. : 20) ;
LLRLTVWGTKNLQARVT (SEQ ID No. : 26) ;
LLRLTVWGTKNLQTRVT (SEQ ID No. : 27) ; et
LLXLTVWGXKXLQXRXX (SEQ ID No. : 42).

11. Composition selon l'une quelconque des revendications précédentes, dans un transporteur pharmaceutiquement acceptable.

12. Peptide D qui comprend au moins quatre résidus d'acides aminés ainsi que la séquence fondamentale WXWL, où W représente un tryptophane D, L représente une leucine D et X représente un groupement quelconque, qui se lie à la poche du peptide trimère soluble ne s'agrégeant pas tel que défini dans la revendication 1.

13. Peptide D selon la revendication 12, dans lequel X représente un résidu acide aminé D ou un résidu acide aminé D modifié.

14. Peptide D selon la revendication 12 ou 13, dans lequel le peptide D comprend 4 à 21 résidus d'acides aminés.

15. Peptide D selon la revendication 12, qui comprend au moins cinq résidus d'acides aminés, dans lequel les au moins cinq résidus d'acides aminés représentent la séquence EWXWL, où E représente l'acide glutamique D, W représente un tryptophane D, L représente une leucine D et X représente un résidu acide aminé, un résidu acide aminé modifié ou un groupement autre qu'un résidu acide aminé, ledit peptide se liant à la poche du peptide trimère soluble ne s'agrégeant pas tel que défini dans la revendication 1.

16. Peptide D selon la revendication 12 ou la revendication 15, qui comprend une séquence d'acides aminés choisie parmi :
(a) CDLKAKEWFWLC (SEQ ID No. : 3) ;
(b) CEARHREWAWLC (SEQ ID No. : 4) ;
(c) CELLGWEWAWLC (SEQ ID No. : 5) ;
(d) CLLRAPEWGWLC (SEQ ID No. : 6) ;
(e) CSRSQPEWEWLC (SEQ H) No. : 7) ;
(f) CGLGQEEWFWLC (SEQ ID No. : 8) ;
(g) CMRGEWEWSWLC (SEQ ID No. : 9) ;
(h) CPPLNKEWAWLC (SEQ ID No. : 10) ;
(i) CVLKAKEWFWLC (SEQ ID No. : 11) ;
(j) KKGACGLGQEEWFWLC (SEQ ID No. : 15) ;
(k) KKGACELLGWEWAWLC (SEQ ID No. : 16) ;
(1) KKKKGACELLGWEWAWLC (SEQ ID No. : 17) ;
(m) KKGACMRGEWEWSWLC (SEQ ID No. : 18) ;
(n) KKGACPPLNKEWAWLC (SEQ ID No. : 19) ;
(o) un peptide D comprenant WXWL (SEQ ID No. : 23) ;
(p) un peptide D comprenant EWXWL (SEQ ID No. : 24) ;
(q) un peptide D comprenant CXXXXXEWXWLC (SEQ ID No. : 12) ;
(r) ac-GACEARHREWAWLCAA-am (SEQ ID No. : 34) ;
(s) ac-KKGACEARHREWAWLCAA-am (SEQ ID No. : 38) ;
(t) ac-KKKKGACEARHREWAWLCAA-am (SEQ ID No. : 43) ;
(u) ac-GACGLGQEEWFWLCAA-am (SEQ ID No. : 44) ;
(v) ac-KKGACGLGQEEWFWLCAA-am (SEQ ID No. : 15) ;
(w) ac-KKKKGACGLGQEEWFWLCAA-am (SEQ ID No. : 45) ;
(x) ac-GACDLKAKEWFWLCAA-am (SEQ ID No. : 35) ;
(y) ac-KKGACDLKAKEWFWLCAA-am (SEQ ID No. : 39) ;
(z) ac-KKKKGACDLKAKEWFWLCAA-am (SEQ ID No. : 46) ;
(a') ac-GACELLGWEWAWLCC-am (SEQ ID No. : 47) ;
(b') ac-KKGACELLGWEWAWLCAA-am (SEQ ID No. : 16) ;
(c') ac-KKKKGACELLGWEWAWLCAA-am (SEQ ID No. : 17) ;
(d') ac-GACSRSQPEWEWLCAA-am (SEQ ID No. : 36) ;
(e') ac-KKGACSRSQPEWEWLCAA-am (SEQ ID No. : 40) ;
(f') ac-KKKKGACSRSQPEWEWLCAA-am (SEQ ID No. : 48) ;
(g') ac-GACLLRAPEWGWLCAA-am (SEQ ID No. : 37) ;
(h') ac-KKGACLLRAPEWGWLCAA-am (SEQ ID No. : 41) ;
(i') ac-KKKKGACLLRAPEWGWLCAA-am (SEQ ID No. : 49) ;
(j') ac-GACMRGEWEWSWLCAA-am (SEQ ID No. : 50) ;
(k') ac-KKGACMRGEWEWSWLCAA-am (SEQ ID No. : 18) ;
(l') ac-KKKKGACMRGEWEWSWLCAA-am (SEQ ID No. : 51) ;
(m') ac-GACPPLNKEWAWLCAA-am (SEQ ID No. : 52) ;
(n') ac-KKGACPPLNKEWAWLCAA-am (SEQ ID No. : 19) ;
(o') ac-KKKKGACPPLNKEWAWLCAA-am (SEQ ID No. : 53) ;
(p') ac-GACXXXXXEWXWLCAA-am (SEQ ID No. : 54) ;
(q') ac-KKGACXXXXXEWXWLCAA-am (SEQ ID No. : 55) ;
(r') ac-KKKKGACXXXXXEWXWLCAA-am (SEQ ID No. : 56) ;
(s') ac-XXCXXXXXEWXWLCXX-am (SEQ ID No. : 57) ;
(t') ac-KKXXCXXXXXEWXWLCXX-am (SEQ ID No. : 58) ;
(u') ac-KKKKXXCXXXXXEWXWLCXX-am (SEQ ID No. : 59) ;
(v') ac-XXCXXXXXEWXWLCXXX-am (SEQ ID No. : 60) ;
(w') ac-KKXXCXXXXXEWXWLCXXX-am (SEQ ID No. : 61) ; et
(x') ac-KKKKXXCXXXXXEWXWLCXXX-am (SEQ ID No. : 62) ;
où les groupes ac- à l'extrémité N-terminale et - am à l'extrémité C-terminale sont facultatifs.

17. Composition selon l'une quelconque des revendications 1 à 11, ou peptide selon l'une quelconque des revendications 12 à 16, destiné à être utilisé pour provoquer une réponse immunitaire chez un individu.

18. Composition ou peptide selon la revendication 17, dans lesquels la réponse immunitaire est provoquée en introduisant le peptide à l'intérieur de l'individu par le biais d'une voie d'administration choisie dans le groupe constitué : des voies intramusculaire, intrapéritonéale, orale, nasale et transdermique.

19. Peptide selon l'une quelconque des revendications 12 à 16, et transporteur ou base destinés à être utilisés dans un procédé interférant avec l'entrée du VIH à l'intérieur d'une cellule mucosale par le biais de l'administration ou de l'application de ceux-ci sur une surface mucosale.

20. Peptide selon la revendication 19, dans lequel le transporteur ou la base est choisi parmi : une mousse, un gel, une autre substance suffisamment visqueuse pour retenir le peptide, de l'eau et une solution tampon.

21. Peptide selon la revendication 19 ou la revendication 20, dans lequel le transporteur ou la base est un ovule à administrer par voie vaginale ou un suppositoire à administrer par voie rectale.

22. Peptide selon l'une quelconque des revendications 19 à 21, dans lequel le peptide est libéré du transporteur ou de la base immédiatement ou rapidement après son administration ou son application dans le vagin, la bouche ou le rectum.

23. Peptide selon l'une quelconque des revendications 19 à 21, dans lequel le peptide est libéré du transporteur ou de la base progressivement ou après une période déterminée qui suit son administration ou son application dans le vagin, la bouche ou le rectum.

24. Peptide selon la revendication 19, dans lequel le peptide se trouve à la surface ou est incorporé à l'intérieur d'un dispositif contraceptif de manière à ce que le dispositif puisse libérer le peptide dans les conditions d'utilisation.

25. Peptide selon l'une quelconque des revendications 19 à 24, dans lequel le peptide bloque ou réduit les changements de conformation de la gp41, interférant ainsi avec l'entrée du VIH à l'intérieur des cellules de la surface mucosale.

26. Procédé d'identification d'un médicament se liant à la cavité hélicoïdale N-terminale de la superhélice de la protéine de l'enveloppe de la gp41 exprimée par le VIH-1, consistant à :
(a) combiner : (1) le peptide soluble tel que défini dans l'une quelconque des revendications 1 à 10 qui présente la cavité hélicoïdale N-terminale de la superhélice de la gp41 exprimée par le VIH de manière à ce qu'elle soit disponible pour se lier à un médicament et (2) un médicament candidat, qui doit être évalué pour sa capacité à se lier à la cavité hélicoïdale N-terminale de la superhélice ; et
(b) déterminer si le médicament candidat se lie à la cavité de la gp41 exprimée par le VIH du peptide soluble, le médicament candidat représentant, si la liaison a bien lieu, un médicament qui se lie à la cavité hélicoïdale N-terminale de la superhélice de la gp41 exprimée par le VIH.

27. Procédé selon la revendication 26, dans lequel l'inhibiteur candidat est marqué de sorte à être détecté, et la liaison du médicament candidat à la cavité de la gp41 exprimée par le VIH est déterminée en détectant la présence du marqueur détectable dans la cavité de la gp41 exprimée par le VIH.

28. Procédé selon la revendication 26 ou 27, dans lequel, en (a), un peptide qui se lie à la cavité hélicoïdale N-terminale de la superhélice de la gp41 exprimée par le VIH est combiné au médicament inhibiteur candidat et au peptide soluble, et en (b), il est déterminé si le médicament candidat se lie à la cavité de la gp41 exprimée par le VIH en présence du peptide se liant à la cavité hélicoïdale N-terminale de la superhélice de la gp41 exprimée par le VIH.

29. Procédé selon la revendication 28, consistant à :
(a) combiner un peptide D se liant à la cavité hélicoïdale N-terminale de la superhélice, le peptide soluble et un inhibiteur candidat, dans des conditions appropriées pour la liaison du peptide D à la cavité hélicoïdale N-terminale de la superhélice, produisant ainsi un échantillon test ;
(b) déterminer le degré de liaison du peptide D à la cavité hélicoïdale N-terminale de la superhélice dans l'échantillon test ; et
(c) comparer le degré déterminé de liaison à la cavité hélicoïdale N-terminale de la superhélice dans un échantillon de contrôle avec celui du peptide D, l'échantillon de contrôle étant le même que l'échantillon test si ce n'est que l'échantillon de contrôle ne comprend pas l'inhibiteur candidat et est maintenu dans les mêmes conditions appropriées pour la liaison du peptide D à la cavité hélicoïdale N-terminale de la superhélice comme dans l'échantillon test,
dans lequel, si le degré de liaison dans l'échantillon test est plus faible que le degré de liaison dans l'échantillon de contrôle, le candidat inhibiteur est un composé ou une molécule qui se lie à la cavité hélicoïdale N-terminale de la superhélice de la protéine de l'enveloppe de la gp41 exprimée par le VIH-1.

30. Procédé selon la revendication 29, dans lequel le peptide D est marqué avec un rapporteur fluorescent et le peptide soluble est marqué avec un groupement désactiveur qui, lorsqu'il est suffisamment à proximité étroite du rapporteur fluorescent, désactive le signal du rapporteur et la détection d'un signal provenant du rapporteur fluorescent indique que l'inhibiteur candidat est un composé ou une molécule qui se lie à la cavité hélicoïdale N-terminale de la superhélice de la protéine de l'enveloppe de la gp41 exprimée par le VIH-1.

31. Procédé selon la revendication 28, dans lequel le peptide qui se lie à la cavité hélicoïdale N-terminale de la superhélice de la gp41 exprimée par le VIH est choisi parmi :
(a) CDLKAKEWFWLC (SEQ ID No. : 3) ;
(b) CEARHREWAWLC (SEQ ID No. : 4) ;
(c) CELLGWEWAWLC (SEQ ID No. : 5) ;
(d) CLLRAPEWGWLC (SEQ ID No. : 6) ;
(e) CSRSQPEWEWLC (SEQ ID No. : 7) ;
(f) CGLGQEEWFWLC (SEQ ID No. : 8) ;
(g) CMRGEWEWSWLC (SEQ ID No. : 9) ;
(h) CPPLNKEWAWLC (SEQ ID No. : 10) ;
(i) CVLKAKEWFWLC (SEQ ID No. : 11) ;
(j) KKGACGLGQEEWFWLC (SEQ ID No. : 15) ;
(k) KKGACELLGWEWAWLC (SEQ ID No. : 16) ;
(1) KKKKGACELLGWEWAWLC (SEQ ID No. : 17) ;
(m) KKGACMRGEWEWSWLC (SEQ ID No. : 18) ;
(n) KKGACPPLNKEWAWLC (SEQ ID No. : 19) ;
(o) un peptide D comprenant WXWL (SEQ ID No. : 23) ;
(p) un peptide D comprenant EWXWL (SEQ ID No. : 24) ;
(q) un peptide D comprenant CXXXXXEWXWLC (SEQ ID No. : 12) ;
(r) ac-GACEARHREWAWLCAA-am (SEQ ID No. : 34) ;
(r) ac-KKGACEARHREWAWLCAA-am (SEQ ID No. : 38) ;
(t) ac-KKKKGACEARHREWAWLCAA-am (SEQ ID No.. 43) ;
(u) ac-GACGLGQEEWFWLCAA-am (SEQ ID No. : 44) ;
(v) ac-KKGACGLGQEEWFWLCAA-am (SEQ ID No. : 15) ;
(w) ac-KKKKGACGLGQEEWFWLCAA-am (SEQ ID No. : 45) ;
(x) ac-GACDLKAKEWFWLCAA-am (SEQ ID No. : 35) ;
(y) ac-KKGACDLKAKEWFWLCAA-am (SEQ ID No. : 39) ;
(z) ac-KKKKGACDLKAKEWFWLCAA-am (SEQ ID No. : 46) ;
(a') ac-GACELLGWEWAWLCC-am (SEQ ID No. : 47) ;
(b') ac-KKGACELLGWEWAWLCAA-am (SEQ ID No. : 16) ;
(c') ac-KKKKGACELLGWEWAWLCAA-am (SEQ ID No. : 17) ;
(d') ac-GACSRSQPEWEWLCAA-am (SEQ ID No. : 36) ;
(e') ac-KKGACSRSQPEWEWLCAA-am (SEQ ID No. : 40) ;
(f') ac-KKKKGACSRSQPEWEWLCAA-am (SEQ ID No. : 48) ;
(g') ac-GACLLRAPEWGWLCAA-am (SEQ ID No. : 37) ;
(h') ac-KKGACLLRAPEWGWLCAA-am (SEQ ID No. : 41) ;
(i') ac-KKKKGACLLRAPEWGWLCAA-am (SEQ ID No. : 49) ;
(j') ac-GACMRGEWEWSWLCAA-am (SEQ ID No. : 50) ;
(k') ac-KKGACMRGEWEWSWLCAA-am (SEQ ID No. : 18) ;
(l') ac-KKKKGACMRGEWEWSWLCAA-am (SEQ ID No. : 51) ;
(m') ac-GACPPLNKEWAWLCAA-am (SEQ ID No. : 52) ;
(n') ac-KKGACPPLNKEWAWLCAA-am (SEQ ID No. : 19) ;
(o') ac-KKKKGACPPLNKEWAWLCAA-am (SEQ ID No. : 53) ;
(p') ac-GACXXXXXEWXWLCAA-am (SEQ ID No. : 54) ;
(q') ac-KKGACXXXXXEWXWLCAA-am (SEQ ID No. : 55) ;
(r') ac-KKKKGACXXXXXEWXWLCAA-am (SEQ ID No. : 56) ;
(s') ac-XXCXXXXXEWXWLCXX-am (SEQ ID No. : 57) ;
(t') ac-KKXXCXXXXXEWXWLCXX-am (SEQ ID No. : 58) ;
(u') ac-KKKKXXCXXXXXEWXWLCXX-am (SEQ ID No. : 59) ;
(v') ac-XXCXXXXXEWXWLCXXX-am (SEQ ID No. : 60) ;
(w') ac-KKXXCXXXXXEWXWLCXXX-am (SEQ ID No. : 61) ; et
(x') ac-KKKKXXCXXXXXEWXWLCXXX-am (SEQ ID No. : 62) ;
où les groupes ac- à l'extrémité N-terminale et - am à l'extrémité C-terminale sont facultatifs.

32. Procédé d'identification d'un peptide se liant à la cavité hélicoïdale N-terminale de la superhélice de la gp41 exprimée par le VIH, consistant à :
(a) combiner le peptide D soluble tel que défini dans la revendication 3 avec une bibliothèque de peptides comprenant des acides aminés L exposés à la surface des phages, dans des conditions appropriées pour la liaison des composés de la bibliothèque au peptide soluble ; et
(b) déterminer si une liaison a lieu entre le peptide soluble et un composé ou des composés de la bibliothèque exposés à la surface des phages, un peptide de chiralité D se liant à la cavité hélicoïdale N-terminale de la superhélice de la gp41 exprimée par le VIH étant identifié si la liaison a bien lieu.

33. Procédé selon la revendication 32, consistant en outre à déterminer la séquence d'acides aminés du composé ou des composés de la bibliothèque exposés à la surface des phages qui se lient au peptide soluble et à produire les peptides, sous la forme D, comprenant les séquences d'acides aminés déterminées, dans lequel les peptides sous la forme D se lient à la cavité hélicoïdale N-terminale de la superhélice sous la forme chirale L naturelle.

34. Procédé d'identification d'une molécule se liant à la cavité hélicoïdale N-terminale de la superhélice de la gp41 exprimée par le VIH, consistant à :
(a) combiner le peptide D soluble tel que défini dans la revendication 3 avec une bibliothèque de ligands codés de manière biologique, dans des conditions appropriées pour la liaison des composés de la bibliothèque au peptide ; et
(b) déterminer si une liaison a lieu entre le peptide soluble et un composé ou des composés de la bibliothèque codés de manière biologique, un ligand se liant à la cavité hélicoïdale N-terminale de la superhélice de la gp41 exprimée par le VIH étant identifié si la liaison a bien lieu.

35. Procédé selon la revendication 34, consistant en outre à déterminer la séquence du composé ou des composés de la bibliothèque codés de manière biologique qui se lient au peptide soluble et à produire les ligands, de sorte qu'ils soient chiraux par rapport aux ligands codés de manière biologique, comprenant les séquences déterminées.

36. Procédé selon la revendication 34 ou la revendication 35, dans lequel la bibliothèque de composés codés de manière biologique est choisie dans le groupe constitué d'une bibliothèque de composés exposés à la surface des phages, une bibliothèque d'ADN, une bibliothèque d'ARN et une bibliothèque de peptides codés de manière biologique.

37. Procédé de production de la composition selon l'une quelconque des revendications 1 à 10, consistant à produire une protéine de fusion comprenant : (a) une forme trimère soluble d'une superhélice et (b) une partie suffisante de la région peptidique N-terminale de la gp41 exprimée par le VIH pour comprendre les résidus d'acides aminés qui forment la poche de l'hélice N-terminale de la superhélice de la gp41 exprimée par le VIH.

38. Procédé selon la revendication 37, dans lequel la protéine de (a) est la GCN4-pI_{Q}I, la GCN4-pII, le virus de la leucémie murine de Moloney ou un hétérotrimère ABC et la partie suffisante de (b) est choisie dans le groupe constitué : d'une partie comprenant la séquence d'acides aminés LLQLTVWGIKQLQARIL (SEQ ID No. : 20) ; une partie comprenant la séquence d'acides aminés LLRLTVWGTKNLQARVT (SEQ ID No. : 26) ; une partie comprenant la séquence d'acides aminés LLRLTVWGTKNLQTRVT (SEQ ID No. : 27) ; et une partie comprenant la séquence d'acides aminés LLXLTVWGXKXLQXRXX (SEQ ID No. : 42).

39. Procédé selon la revendication 37 ou 38, dans lequel la protéine de fusion est l'IQN17, dans lequel la séquence d'acides aminés de l'IQN17 est :
ac-RMKQIEDKIEEIESKQKKIENEIARIKKLLQLTVWGIKQLQARIL-am (SEQ ID No. : 2), où ac- représente un groupe acétyle N-terminal et -am représente un amide C-terminal.

40. Procédé de production d'un médicament qui s'adapte à la poche de l'hélice N-terminale de la superhélice de la gp41 exprimée par le VIH, consistant à :
(a) obtenir un cristal du peptide soluble tel que défini dans l'une quelconque des revendications 1 à 10 ;
(b) obtenir les coordonnées atomiques du peptide soluble par le biais d'études de diffraction aux rayons X en utilisant le cristal obtenu en (a) ;
(c) utiliser les coordonnées atomiques obtenues en (b) pour définir la poche de l'hélice N-terminale de la superhélice de la gp41 exprimée par le VIH ;
(c) identifier une molécule ou un composé qui s'adapte à la poche de l'hélice N-terminale de la superhélice de la gp41 exprimée par le VIH ;
(d) obtenir la molécule ou le composé identifié en (d) ; et
(e) mettre en contact la molécule ou le composé obtenu en (e) avec la poche de l'hélice N-terminale de la superhélice de la gp41 exprimée par le VIH afin d'évaluer la capacité de la molécule ou du composé à s'adapter à la poche de la gp41 exprimée par le VIH,
dans lequel la molécule ou le composé représente un médicament qui s'adapte à la poche si la molécule ou le composé s'adapte à la poche de l'hélice N-terminale de la superhélice de la gp41 exprimée par le VIH, un médicament qui s'adapte à la poche de l'hélice N-terminale de la superhélice de la gp41 exprimée par le VIH étant alors produit.

41. Procédé selon la revendication 40, dans lequel (f), la molécule ou le composé est mis en contact avec la poche de l'hélice N-terminale de la superhélice de la gp41 exprimée par le VIH en mettant en contact la molécule ou le composé avec l'IQN17, l'hélice N-terminale de la gp41 exprimée par le VIH ou un polypeptide qui comprend la poche du VIH.

42. Procédé selon la revendication 40 ou la revendication 41, dans lequel le peptide soluble est l'IQN17.

43. Procédé selon l'une quelconque des revendications 40 à 42, dans lequel le cristal obtenu en (a) est un cristal d'IQN17 de groupe spatial C222.

44. Procédé de production d'un médicament qui se lie à la poche de l'hélice N-terminale de la superhélice de la gp41 exprimée par le VIH, consistant à :
(a) obtenir les coordonnées atomiques de l'IQN17 ;
(b) utiliser les coordonnées atomiques obtenues en (a) pour définir la poche de l'hélice N-terminale de la superhélice de la gp41 exprimée par le VIH ;
(c) identifier une molécule ou un composé qui s'adapte à la poche de l'hélice N-terminale de la superhélice de la gp41 exprimée par le VIH ;
(d) obtenir la molécule ou le composé identifié en (c) ; et
(e) mettre en contact la molécule ou le composé obtenu en (d) avec la poche de l'hélice N-terminale de la superhélice de la gp41 exprimée par le VIH afin d'évaluer la capacité de la molécule ou du composé à s'adapter à la poche de la gp41 exprimée par le VIH,
dans lequel la molécule ou le composé représente un médicament qui s'adapte à la poche si la molécule ou le composé s'adapte à la poche de l'hélice N-terminale de la superhélice de la gp41 exprimée par le VIH, un médicament qui s'adapte à la poche de l'hélice N-terminale de la superhélice de la gp41 exprimée par le VIH étant alors produit.

45. Procédé selon la revendication 44, dans lequel les coordonnées atomiques sont les coordonnées atomiques dans le fichier PDB représenté sur les figures 11A à 11V.

46. Vaccin comprenant le peptide tel que défini dans l'une quelconque des revendications 1 à 10.

47. Vaccin selon la revendication 46, destiné à provoquer une réponse immunitaire protégeant totalement ou en partie contre une infection et/ou une maladie induite par le VIH.
